(19) Europäisches Patentamt
European Patent Office
Office européen des brevets

(11) EP 4 342 492 A1

(12) **EUROPEAN PATENT APPLICATION**
published in accordance with Art. 153(4) EPC

(43) Date of publication:
**27.03.2024 Bulletin 2024/13**

(21) Application number: **22804057.2**

(22) Date of filing: **20.05.2022**

(51) International Patent Classification (IPC):
**A61K 39/395** (2006.01)    **A61K 31/7084** (2006.01)
**A61P 35/00** (2006.01)

(52) Cooperative Patent Classification (CPC):
**A61K 31/7084; A61K 39/395; A61P 35/00**

(86) International application number:
**PCT/CN2022/094047**

(87) International publication number:
**WO 2022/242737 (24.11.2022 Gazette 2022/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.05.2021 CN 202110555249**

(71) Applicant: **Tianjin Lipogen Technology Co., Ltd**
**Tianjin 300392 (CN)**

(72) Inventors:
• **WANG, Chenguang**
**Tianjin 300392 (CN)**
• **ZHANG, Xiaojiao**
**Tianjin 300392 (CN)**

• **DANG, Xiaomeng**
**Tianjin 300392 (CN)**
• **ZHANG, Jin**
**Tianjin 300392 (CN)**
• **FU, Yuting**
**Tianjin 300392 (CN)**
• **WANG, Yihan**
**Tianjin 300392 (CN)**
• **WANG, Chao**
**Tianjin 300392 (CN)**

(74) Representative: **HGF**
**HGF BV**
**Benoordenhoutseweg 46**
**2596 BC The Hague (NL)**

Remarks:
The complete document including Reference Table(s) and the Sequence Listing(s) can be downloaded from the EPO website

(54) **PHARMACEUTICAL COMBINATION AND USE THEREOF**

(57) A pharmaceutical combination and a use thereof. The pharmaceutical combination comprises a PD-1 inhibitor and/or a PD-L1 immune checkpoint inhibitor; and a STING pathway agonist. The pharmaceutical combination has a better antitumor efficacy than an immune checkpoint inhibitor alone or a STING pathway agonist alone, realizes treatment by both innate immune pathway activation of organisms and immune escape prevention, and thus has efficient and low-toxicity clinical application prospects.

EP 4 342 492 A1

## Description

### Technical Field

The disclosure relates to the field of biomedicine, specifically to development and application of a pharmaceutical combination.

### Background

Over the past decade, targeting PD-1 and PD-L1 has opened up a new era for clinical treatment of cancers. However, for many types of tumors, an immune checkpoint inhibitor monotherapy has poor efficacy. Even for immune responsive tumors, sustained clinical benefits have not been achieved in most patients. The vast majority of patients develop primary or acquired therapeutic drug resistance. In most cases, immunotherapy drug resistance can be attributed to the presence of immunosuppressive TME and insufficient activation of T cell anti-tumor effects due to a decrease in the number of immune cells in vivo. Therefore, compared to monotherapy, it is particularly important to actively seek new therapeutic strategies capable of reducing immune suppression in tumor environments or enhancing the response of cytotoxic cells to tumors.

The PD-L1/PD-1 signaling pathway is a very important co-inhibitory signaling pathway in the immune response. Researches have shown that when PD-L1 binds to PD-1, it will complement protein-tyrosine-phosphatases SHP-1 and SHP-2 with SH2 domains. These two phosphatases can reduce the extent of phosphorylation of the immunoreceptor tyrosine-based activation motif (ITAM) of CD3 $\zeta$ strand, weaken the activation of ZAP-70, and inhibit TCR downstream signaling, thereby playing a co-inhibitory role in T cell activation, and the autoimmune injury due to excessive activation of effector T cells can be prevented through such negative regulatory effect.

The stimulator of interferon genes (STING) is an immunostimulatory small molecule target mainly distributed in immune-related tissue cells, such as high expression in thymus, spleen, and peripheral blood leukocytes. When tumor cells undergo necrosis, cGAMP can bind and activate STING in the endoplasmic reticulum. Activation of STING leads to nuclear translocation of transcription factors, induces the expressions of interferons (INFs) and cytokines, promotes aggregation and activation of T cells, and ultimately kills tumor cells. The STING pathway can also be activated by synthesized cyclic dinucleotides (CDNs), triggering innate immune responses.

The combination of PD-L1/PD-1 inhibitors and STING pathway agonists, on the one hand, relieves inhibitory signals, enhances activation of T cells, and promotes adaptive immune system responses; on the other hand, it induces the expressions of INFs and cytokines, promotes aggregation of T cells, activates the innate immune system responses, and enhances the responses of cytotoxic cells to tumors, etc. through a dual approach, which thus has very high clinical prospects and application values.

Moreover, researches have shown that some viral infections are also closely related to the PD-L1/PD-1 signaling pathway. For example, in chronic HIV infections, PD-1 was found to be highly expressed on the surface of CD8+T cells that specifically recognize HIV. The virus activates the PD-L1/PD-1 signaling pathway, through which the activity of CD8+T cells that specifically recognize HIV is inhibited, and the secretion ability of cytokines and the proliferation ability of T cells themselves are greatly weakened, leading to adaptive immune dysfunctions. It can be seen that this therapy may also have considerable application values in the treatment of such diseases.

### Summary

The disclosure provides a pharmaceutical combination and a use thereof in antitumor drugs. The pharmaceutical combination mainly consists of two parts:
(1) an immune checkpoint inhibitor (e.g., PD-1/PD-L1); (2) a STING pathway agonist (e.g., cyclic dinucleotide cGAMP or derivatives thereof). The pharmaceutical combination of the disclosure can be used to prepare efficient and low-toxicity anti-tumor drugs.

In one aspect, the disclosure provides a pharmaceutical combination including a programmed cell death protein 1 (PD-1) inhibitor and/or a programmed death ligand 1 (PD-L1) inhibitor, and a STING pathway agonist.

In some embodiments, where the STING pathway agonist includes a cyclic dinucleotide.

In some embodiments, where the cyclic dinucleotide is selected from the group consisting of c-di-AMP, c-di-GMP, c-di-GMP-F, 3',3'-cGAMP, 3',3'-cGAMP-F, 2',3'-cGAMP, Rp/Sp(CL656), ADU-S100, ADU-S100 disodium, and derivatives or combinations thereof.

In some embodiments, where the STING pathway agonist includes 2',3'-cGAMP, or derivatives thereof.

In some embodiments, where the STING pathway agonist includes flavonoids.

In some embodiments, where the flavonoid includes CMA, DMXAA, methoxyflavone, 6,4'-dimethoxyflavone, 4'-methoxyflavone, 3',6'-dihydroxyflavone, 7,2'-dihydroxyflavone, daidzein, Formononetin, retinene 7-methyl ether, xanthone, and/or any combination thereof.

In some embodiments, where the STING pathway agonist includes DNA.

In some embodiments, where the STING pathway agonist includes type I interferons (IFNs).

In some embodiments, where the type I interferon includes IFN-α or IFN-β.

In some embodiments, where the PD-1 inhibitor has one or more of the following characteristics:

a. inhibition or reduction of PD-1 expression, such as transcription or translation of PD-1;

b. inhibition or reduction of PD-1 activity, such as inhibition or reduction of PD-1 binding to its homologous ligands, such as PD-L1 or PD-L2; and

c. binding PD-1 or one or more of its ligands, such as PD-L1 or PD-L2.

In some embodiments, where the PD-1 inhibitor includes an anti PD-1 antibody or antigen-binding fragments thereof.

In some embodiments, where the anti PD-1 antibody is selected from the group consisting of Pembrolizumab, Nivolumab, Pidilizumab, SHR-1210, MEDI0680, BGB-A317, TSR-042, REGN2810, PF-06801591, RB0004, Tislelizumab, Camrelizumab, Toripalimab, Sintilimab, bio-analogues, bio-enhancers, bio-equivalents, or combinations thereof.

In some embodiments, where the anti PD-1 antibody includes at least one CDR in the antibody heavy chain variable region (VH), and the VH includes an amino acid sequence shown in SEQ ID NO: 8.

In some embodiments, where the anti PD-1 antibody includes a VH including HCDR3, and the HCDR3 includes an amino acid sequence shown in SEQ ID NO: 3.

In some embodiments, where the VH further includes HCDR2, wherein the HCDR2 includes an amino acid sequence shown in SEQ ID NO: 2.

In some embodiments, where the VH further includes HCDR1, wherein the HCDR1 includes an amino acid sequence shown in SEQ ID NO: 1.

In some embodiments, where the VH includes HCDR1, HCDR2, and HCDR3, wherein the HCDR3 includes an amino acid sequence shown in SEQ ID NO: 3, the HCDR2 includes an amino acid sequence shown in SEQ ID NO: 2, and the HCDR1 includes an amino acid sequence shown in SEQ ID NO: 1.

In some embodiments, where the VH includes a framework region HFR1, the C-terminal of HFR1 is directly or indirectly connected to the N-terminal of HCDR1, and the HFR1 includes an amino acid sequence shown in SEQ ID NO: 4 or an amino acid sequence having at least about 70% sequence identity to the amino acid sequence shown in SEQ ID NO: 4.

In some embodiments, where the VH includes a framework region HFR2, the N-terminal of HFR2 is directly or indirectly connected to the C-terminal of HCDR1, and the C-terminal of HFR2 is directly or indirectly connected to the N-terminal of HCDR2; and the HFR2 includes an amino acid sequence shown in SEQ ID NO: 5 or an amino acid sequence having at least about 70% sequence identity to the amino acid sequence shown in SEQ ID NO: 5.

In some embodiments, where the VH includes a framework region HFR3, the N-terminal of HFR3 is directly or indirectly connected to the C-terminal of HCDR2, and the C-terminal of HFR3 is directly or indirectly connected to the N-terminal of HCDR3; and the HFR3 includes an amino acid sequence shown in SEQ ID NO: 6 or an amino acid sequence having at least about 70% sequence identity to the amino acid sequence shown in SEQ ID NO: 6.

In some embodiments, where the VH includes a framework region HFR4, the N-terminal of HFR4 is directly or indirectly connected to the C-terminal of HCDR3, and the HFR4 includes an amino acid sequence shown in SEQ ID NO: 7 or an amino acid sequence having at least about 70% sequence identity to the amino acid sequence shown in SEQ ID NO: 7.

In some embodiments, where the VH includes framework regions HFR1, HFR2, HFR3, and HFR4, the C-terminal of HFR1 is directly or indirectly connected to the N-terminal of HCDR1, the N-terminal of HFR2 is directly or indirectly connected to the C-terminal of HCDR1, and the C-terminal of HFR2 is directly or indirectly connected to the N-terminal of HCDR2, the N-terminal of HFR3 is directly or indirectly connected to the C-terminal of HCDR2, and the C-terminal of HFR3 is directly or indirectly connected to the N-terminal of HCDR3, the N-terminal of HFR4 is directly or indirectly connected to the C-terminal of HCDR3; among them, the HFR1 includes an amino acid sequence shown in SEQ ID NO: 4 or an amino acid sequence having at least about 70% sequence identity to the amino acid sequence shown in SEQ ID NO: 4, the HFR2 includes an amino acid sequence shown in SEQ ID NO: 5 or an amino acid sequence having at least about 70% sequence identity to the amino acid sequence shown in SEQ ID NO: 5, the HFR3 includes an amino acid sequence shown in SEQ ID NO: 6 or an amino acid sequence having at least about 70% sequence identity to the amino acid sequence shown in SEQ ID NO: 6, and the HFR4 includes an amino acid sequence shown in SEQ ID NO: 7 or an amino acid sequence having at least about 70% sequence identity to the amino acid sequence shown in SEQ ID NO: 7.

In some embodiments, where the anti PD-1 antibody includes a VH, and the VH includes an amino acid sequence shown in SEQ ID NO: 8.

In some embodiments, where the anti PD-1 antibody includes an antibody heavy chain (HC), and the HC includes an amino acid sequence shown in SEQ ID NO: 9.

In some embodiments, where the anti PD-1 antibody includes at least one CDR in the antibody light chain variable region (VL), and the VL includes an amino acid sequence shown in SEQ ID NO: 17.

In some embodiments, where the anti PD-1 antibody includes at least one CDR in VH, wherein the VH includes an amino acid sequence shown in SEQ ID NO: 8, and the anti PD-1 antibody includes at least one CDR in VL, and the VL includes an amino acid sequence shown in SEQ ID NO: 17.

In some embodiments, where the anti PD-1 antibody includes a VL including LCDR1, and the LCDR1 includes an amino acid sequence shown in SEQ ID NO: 10.

In some embodiments, where the VL further includes LCDR2, wherein the LCDR2 includes an amino acid sequence shown in SEQ ID NO: 11.

In some embodiments, where the VL further includes LCDR3, wherein the LCDR3 includes an amino acid sequence shown in SEQ ID NO: 12.

In some embodiments, where the VL includes LCDR1, LCDR2 and LCDR3, wherein the LCDR1 includes an amino acid sequence shown in SEQ ID NO: 10, the LCDR2 includes an amino acid sequence shown in SEQ ID NO: 11, and the LCDR3 includes an amino acid sequence shown in SEQ ID NO: 12.

In some embodiments, where the anti PD-1 antibody includes VH and antibody VL, the VH includes HCDR1, HCDR2, and HCDR3, wherein the HCDR3 includes an amino acid sequence shown in SEQ ID NO: 3, the HCDR2 includes an amino acid sequence shown in SEQ ID NO: 2, and the HCDR1 includes an amino acid sequence shown in SEQ ID NO: 1; and the VL includes LCDR1, LCDR2, and LCDR3, wherein the LCDR1 includes an amino acid sequence shown in SEQ ID NO: 10, the LCDR2 includes an amino acid sequence shown in SEQ ID NO: 11, and the LCDR3 includes an amino acid sequence shown in SEQ ID NO: 12.

In some embodiments, where the VL includes a framework region LFR1, the C-terminal of LFR1 is directly or indirectly connected to the N-terminal of LCDR1, and the LFR1 includes an amino acid sequence shown in SEQ ID NO: 13 or an amino acid sequence having at least about 70% sequence identity to the amino acid sequence shown in SEQ ID NO: 13.

In some embodiments, where the VL includes a framework region LFR2, the N-terminal of LFR2 is directly or indirectly connected to the C-terminal of LCDR1, and the C-terminal of LFR2 is directly or indirectly connected to the N-terminal of LCDR2; and the LFR2 includes an amino acid sequence shown in SEQ ID NO: 14 or an amino acid sequence having at least about 70% sequence identity to the amino acid sequence shown in SEQ ID NO: 14.

In some embodiments, where the VL includes a framework region LFR3, the N-terminal of LFR3 is directly or indirectly connected to the C-terminal of LCDR2, and the C-terminal of LFR3 is directly or indirectly connected to the N-terminal of LCDR3; and the LFR3 includes an amino acid sequence shown in SEQ ID NO: 15 or an amino acid sequence having at least about 70% sequence identity to the amino acid sequence shown in SEQ ID NO: 15.

In some embodiments, where the VL includes a framework region LFR4, the N-terminal of LFR4 is directly or indirectly connected to the C-terminal of LCDR3, and the LFR4 includes an amino acid sequence shown in SEQ ID NO: 16 or an amino acid sequence having at least about 70% sequence identity to the amino acid sequence shown in SEQ ID NO: 16.

In some embodiments, where the VL includes framework regions LFR1, LFR2, LFR3 and LFR4, the C-terminal of LFR1 is directly or indirectly connected to the N-terminal of LCDR1, the N-terminal of LFR2 is directly or indirectly connected to the C-terminal of LCDR1, and the C-terminal of LFR2 is directly or indirectly connected to the N-terminal of LCDR2, the N-terminal of LFR3 is directly or indirectly connected to the C-terminal of LCDR2, and the C-terminal of LFR3 is directly or indirectly connected to the N-terminal of LCDR3, the N-terminal of LFR4 is directly or indirectly connected to the C-terminal of LCDR3; among them, the LFR1 includes an amino acid sequence shown in SEQ ID NO: 13 or an amino acid sequence having at least about 70% sequence identity to the amino acid sequence shown in SEQ ID NO: 13, the LFR2 includes an amino acid sequence shown in SEQ ID NO: 14 or an amino acid sequence having at least about 70% sequence identity to the amino acid sequence shown in SEQ ID NO: 14, the LFR3 includes an amino acid sequence shown in SEQ ID NO: 15 or an amino acid sequence having at least about 70% sequence identity to the amino acid sequence shown in SEQ ID NO: 15, and the LFR4 includes an amino acid sequence shown in SEQ ID NO: 16 or an amino acid sequence having at least about 70% sequence identity to the amino acid sequence shown in SEQ ID NO:16.

In some embodiments, where the anti PD-1 antibody includes a VL, and the VL includes an amino acid sequence shown in SEQ ID NO: 17.

In some embodiments, where the anti PD-1 antibody includes VH and VL, the VH includes an amino acid sequence shown in SEQ ID NO: 8, and the VL includes an amino acid sequence shown in SEQ ID NO: 17.

In some embodiments, where the anti PD-1 antibody includes an antibody light chain (LC), and the LC includes an amino acid sequence shown in SEQ ID NO: 18.

In some embodiments, where the anti PD-1 antibody includes HC and LC, the HC includes an amino acid sequence shown in SEQ ID NO: 9, and the LC includes an amino acid sequence shown in SEQ ID NO: 18.

In some embodiments, where the PD-L1 inhibitor has one or more of the following characteristics:

a. inhibition or reduction of PD-L1 expression, such as transcription or translation of PD-L1;

b. inhibition or reduction of PD-L1 activity, such as inhibition or reduction of PD-L1 binding to its associated receptors such as PD-1; and

c. binding of PD-L1 or its receptors such as PD-1.

In some embodiments, where the PD-L1 inhibitor includes an anti PD-L1 antibody or antigen-binding fragments thereof.

In some embodiments, where the anti PD-L1 antibody is selected from the group consisting of Durvalumab, Atezolizumab, Avelumab, Envifolimab, MDX-1105, YW243.55.S70, MDPL3280A, AMP-224, LY3300054, RB0005, bio-analogues, bio-enhancers, bio-equivalents, or combinations thereof.

In some embodiments, where the anti PD-L1 antibody includes at least one CDR in VH, the VH includes an amino acid sequence shown in SEQ ID NO: 25.

In some embodiments, where the anti PD-L1 antibody includes a VH, and the VH includes HCDR3, and the HCDR3 includes an amino acid sequence shown in SEQ ID NO: 21.

In some embodiments, where the VH further includes HCDR2, wherein the HCDR2 includes an amino acid sequence shown in SEQ ID NO: 20.

In some embodiments, where the VH further includes HCDR1, wherein the HCDR1 includes an amino acid sequence shown in SEQ ID NO: 19.

In some embodiments, where the VH includes HCDR1, HCDR2, and HCDR3, wherein the HCDR3 includes an amino acid sequence shown in SEQ ID NO: 21, the HCDR2 includes an amino acid sequence shown in SEQ ID NO: 20, and the HCDR1 includes an amino acid sequence shown in SEQ ID NO: 19.

In some embodiments, where the VH includes a framework region HFR1, the C-terminal of HFR1 is directly or indirectly connected to the N-terminal of HCDR1, and the HFR1 includes an amino acid sequence shown in SEQ ID NO: 22 or an amino acid sequence having at least about 70% sequence identity to the amino acid sequence shown in SEQ ID NO: 22.

In some embodiments, where the VH includes a framework region HFR2, the N-terminal of HFR2 is directly or indirectly connected to the C-terminal of HCDR1, and the C-terminal of HFR2 is directly or indirectly connected to the N-terminal of HCDR2; and the HFR2 includes an amino acid sequence shown in SEQ ID NO: 23 or an amino acid sequence having at least about 70% sequence identity to the amino acid sequence shown in SEQ ID NO: 23.

In some embodiments, where the VH includes a framework region HFR3, the N-terminal of HFR3 is directly or indirectly connected to the C-terminal of HCDR2, and the C-terminal of HFR3 is directly or indirectly connected to the N-terminal of HCDR3; and the HFR3 includes an amino acid sequence shown in SEQ ID NO: 24 or an amino acid sequence having at least about 70% sequence identity to the amino acid sequence shown in SEQ ID NO: 24.

In some embodiments, where the VH includes a framework region HFR4, the N-terminal of HFR4 is directly or indirectly connected to the C-terminal of HCDR3, and the HFR4 includes an amino acid sequence shown in SEQ ID NO: 7 or an amino acid sequence having at least about 70% sequence identity to the amino acid sequence shown in SEQ ID NO: 7.

In some embodiments, where the VH includes framework regions HFR1, HFR2, HFR3, and HFR4, the C-terminal of HFR1 is directly or indirectly connected to the N-terminal of HCDR1, the N-terminal of HFR2 is directly or indirectly connected to the C-terminal of HCDR1, and the C-terminal of HFR2 is directly or indirectly connected to the N-terminal of HCDR2, the N-terminal of HFR3 is directly or indirectly connected to the C-terminal of HCDR2, and the C-terminal of HFR3 is directly or indirectly connected to the N-terminal of HCDR3, the N-terminal of HFR4 is directly or indirectly connected to the C-terminal of HCDR3; among them, the HFR1 includes an amino acid sequence shown in SEQ ID NO: 22 or an amino acid sequence having at least about 70% sequence identity to the amino acid sequence shown in SEQ ID NO: 22, the HFR2 includes an amino acid sequence shown in SEQ ID NO: 23 or an amino acid sequence having at least about 70% sequence identity to the amino acid sequence shown in SEQ ID NO: 23, the HFR3 includes an amino acid sequence shown in SEQ ID NO: 24 or an amino acid sequence having at least about 70% sequence identity to the amino acid sequence shown in SEQ ID NO: 24, and the HFR4 includes an amino acid sequence shown in SEQ ID NO: 7 or an amino acid sequence having at least about 70% sequence identity to the amino acid sequence shown in SEQ ID NO: 7.

In some embodiments, where the anti PD-L1 antibody includes VH, and the VH includes an amino acid sequence shown in SEQ ID NO: 25.

In some embodiments, where the anti PD-L1 antibody includes HC, and the HC includes an amino acid sequence shown in SEQ ID NO: 26.

In some embodiments, where the anti PD-L1 antibody includes at least one CDR in VL, and the VL includes an amino acid sequence shown in SEQ ID NO: 37.

In some embodiments, where the anti PD-L1 antibody includes at least one CDR in VH, the VH includes an amino acid sequence shown in SEQ ID NO: 25, and the anti PD-L1 antibody includes at least one CDR in VL, and the VL includes an amino acid sequence shown in SEQ ID NO: 37.

In some embodiments, where the anti PD-L1 antibody includes at least one CDR in VH, the VH includes an amino acid sequence shown in SEQ ID NO: 25, and the anti PD-L1 antibody includes at least one CDR in VL, and the VL includes an amino acid sequence shown in SEQ ID NO:38, SEQ ID NO:39 or SEQ ID NO:40.

In some embodiments, where the anti PD-L1 antibody includes a VL including LCDR1, and the LCDR1 includes an amino acid sequence shown in SEQ ID NO: 27.

In some embodiments, where the anti PD-L1 antibody includes a VL including LCDR1, and the LCDR1 includes an amino acid sequence shown in SEQ ID NO:28, SEQ ID NO:29 or SEQ ID NO:30.

In some embodiments, where the VL further includes LCDR2, wherein the LCDR2 includes an amino acid sequence shown in SEQ ID NO: 31.

In some embodiments, where the VL further includes LCDR3, wherein the LCDR3 includes an amino acid sequence shown in SEQ ID NO: 32.

In some embodiments, where the VL includes LCDR1, LCDR2 and LCDR3, wherein the LCDR1 includes an amino acid sequence shown in SEQ ID NO: 27, the LCDR2 includes an amino acid sequence shown in SEQ ID NO: 31, and the LCDR3 includes an amino acid sequence shown in SEQ ID NO: 32.

In some embodiments, where the VL includes LCDR1, LCDR2 and LCDR3, wherein the LCDR1 includes an amino acid sequence shown in SEQ ID NO: 28, the LCDR2 includes an amino acid sequence shown in SEQ ID NO: 31, and the LCDR3 includes an amino acid sequence shown in SEQ ID NO: 32;

the LCDR1 includes an amino acid sequence shown in SEQ ID NO: 29, the LCDR2 includes an amino acid sequence shown in SEQ ID NO: 31, and the LCDR3 includes an amino acid sequence shown in SEQ ID NO: 32; or

the LCDR1 includes an amino acid sequence shown in SEQ ID NO: 30, the LCDR2 includes an amino acid sequence shown in SEQ ID NO: 31, and the LCDR3 includes an amino acid sequence shown in SEQ ID NO: 32.

In some embodiments, where the anti PD-L1 antibody includes VH and antibody VL, the VH includes HCDR1, HCDR2, and HCDR3, wherein the HCDR3 includes an amino acid sequence shown in SEQ ID NO: 21, the HCDR2 includes an amino acid sequence shown in SEQ ID NO: 20, and the HCDR1 includes an amino acid sequence shown in SEQ ID NO: 19; and the VL includes LCDR1, LCDR2, and LCDR3, wherein the LCDR1 includes an amino acid sequence shown in SEQ ID NO: 27, the LCDR2 includes an amino acid sequence shown in SEQ ID NO: 31, and the LCDR3 includes an amino acid sequence shown in SEQ ID NO: 32.

In some embodiments, where the anti PD-L1 antibody includes VH and antibody VL, the VH includes HCDR1, HCDR2, and HCDR3, wherein the HCDR3 includes an amino acid sequence shown in SEQ ID NO: 21, the HCDR2 includes an amino acid sequence shown in SEQ ID NO: 20, and the HCDR1 includes an amino acid sequence shown in SEQ ID NO: 19; and the VL includes LCDR1, LCDR2, and LCDR3, wherein the LCDR1 includes an amino acid sequence shown in SEQ ID NO:28, SEQ ID NO:29 or SEQ ID NO:30, the LCDR2 includes an amino acid sequence shown in SEQ ID NO: 31, and the LCDR3 includes an amino acid sequence shown in SEQ ID NO: 32.

In some embodiments, where the VL includes a framework region LFR1, the C-terminal of LFR1 is directly or indirectly connected to the N-terminal of LCDR1, and the LFR1 includes an amino acid sequence shown in SEQ ID NO: 33 or an amino acid sequence having at least about 70% sequence identity to the amino acid sequence shown in SEQ ID NO: 33.

In some embodiments, where the VL includes a framework region LFR2, the N-terminal of LFR2 is directly or indirectly connected to the C-terminal of LCDR1, and the C-terminal of LFR2 is directly or indirectly connected to the N-terminal of LCDR2; and the LFR2 includes an amino acid sequence shown in SEQ ID NO: 34 or an amino acid sequence having at least about 70% sequence identity to the amino acid sequence shown in SEQ ID NO: 34.

In some embodiments, where the VL includes a framework region LFR3, the N-terminal of LFR3 is directly or indirectly connected to the C-terminal of LCDR2, and the C-terminal of LFR3 is directly or indirectly connected to the N-terminal of LCDR3; and the LFR3 includes an amino acid sequence shown in SEQ ID NO: 35 or an amino acid sequence having at least about 70% sequence identity to the amino acid sequence shown in SEQ ID NO: 35.

In some embodiments, where the VL includes a framework region LFR4, the N-terminal of LFR4 is directly or indirectly connected to the C-terminal of LCDR3, and the LFR4 includes an amino acid sequence shown in SEQ ID NO:

36 or an amino acid sequence having at least about 70% sequence identity to the amino acid sequence shown in SEQ ID NO: 36.

In some embodiments, where the VL includes framework regions LFR1, LFR2, LFR3 and LFR4, the C-terminal of LFR1 is directly or indirectly connected to the N-terminal of LCDR1, the N-terminal of LFR2 is directly or indirectly connected to the C-terminal of LCDR1, and the C-terminal of LFR2 is directly or indirectly connected to the N-terminal of LCDR2, the N-terminal of LFR3 is directly or indirectly connected to the C-terminal of LCDR2, and the C-terminal of LFR3 is directly or indirectly connected to the N-terminal of LCDR3, the N-terminal of LFR4 is directly or indirectly connected to the C-terminal of LCDR3; among them, the LFR1 includes an amino acid sequence shown in SEQ ID NO: 33 or an amino acid sequence having at least about 70% sequence identity to the amino acid sequence shown in SEQ ID NO: 33, the LFR2 includes an amino acid sequence shown in SEQ ID NO: 34 or an amino acid sequence having at least about 70% sequence identity to the amino acid sequence shown in SEQ ID NO: 34, the LFR3 includes an amino acid sequence shown in SEQ ID NO: 35 or an amino acid sequence having at least about 70% sequence identity to the amino acid sequence shown in SEQ ID NO: 35, and the LFR4 includes an amino acid sequence shown in SEQ ID NO: 36 or an amino acid sequence having at least about 70% sequence identity to the amino acid sequence shown in SEQ ID NO:36.

In some embodiments, where the anti PD-L1 antibody includes a VL, and the VL includes an amino acid sequence shown in SEQ ID NO: 37.

In some embodiments, where the anti PD-L1 antibody includes a VL, and the VL includes an amino acid sequence shown in SEQ ID NO:38, SEQ ID NO:39 or SEQ ID NO:40.

In some embodiments, where the anti PD-L1 antibody includes VH and VL, the VH includes an amino acid sequence shown in SEQ ID NO: 25, and the VL includes an amino acid sequence shown in SEQ ID NO: 37.

In some embodiments, where the anti PD-L1 antibody includes VH and VL, the VH includes an amino acid sequence shown in SEQ ID NO: 25, and the VL includes an amino acid sequence shown in SEQ ID NO: 38, SEQ ID NO:39 or SEQ ID NO:40.

In some embodiments, where the anti PD-L1 antibody includes LC, and the LC includes an amino acid sequence shown in SEQ ID NO: 41.

In some embodiments, where the anti PD-L1 antibody includes LC, and the LC includes an amino acid sequence shown in SEQ ID NO:42, SEQ ID NO:43 or SEQ ID NO:44.

In some embodiments, where the anti PD-L1 antibody includes HC and LC, the HC includes an amino acid sequence shown in SEQ ID NO: 26, and the LC includes an amino acid sequence shown in SEQ ID NO: 41.

In some embodiments, where the anti PD-L1 antibody includes HC and LC, the HC includes an amino acid sequence shown in SEQ ID NO: 26, and the LC includes an amino acid sequence shown in SEQ ID NO:42, SEQ ID NO:43 or SEQ ID NO:44.

In some embodiments, i) the PD-1 inhibitor and/or PD-L1 inhibitor in the pharmaceutical combination are not mixed with ii) the STING pathway agonist to each other in the pharmaceutical combination.

In some embodiments, i) the PD-1 inhibitor and/or PD-L1 inhibitor , and ii) the STING pathway agonist are present in the pharmaceutical combination in a single dosage form.

In some embodiments, where the pharmaceutical combination is formulated into a pharmaceutical composition.

In some embodiments, where the pharmaceutical composition includes a PD-1 inhibitor or a PD-L1 inhibitor, and a STING pathway agonist.

In some embodiments, where the STING pathway agonist is present in an amount of about 0.0001 mg/kg to about 200 mg/kg.

In some embodiments, where the PD-1 inhibitor or PD-L1 inhibitor is present in an amount of 0.0001 mg/kg to about 200 mg/kg.

In some embodiments, where the pharmaceutical composition further includes one or more pharmaceutically acceptable carriers.

In another aspect, the disclosure also provides a use of the aforementioned pharmaceutical combination in the preparation of drugs for the treatment of neoplastic diseases.

In some embodiments, where the neoplastic diseases include tumors and/or wart diseases.

In another aspect, the disclosure also provides the aforementioned pharmaceutical combination for use in the treatment of neoplastic diseases.

In another aspect, the disclosure also provides a drug including the aforementioned pharmaceutical combination for treating neoplastic diseases.

In another aspect, the disclosure also provides a method for treating neoplastic diseases, which includes administering an effective amount of the aforementioned pharmaceutical combination to a subject in need thereof.

In some embodiments, where the subject suffers from neoplasm.

In some embodiments, where the neoplasm includes tumors and/or warts.

In some embodiments, where the administration includes local, intraneoplasitc (e.g., in tumors or warts), or systemic administrations.

In some embodiments, where the administration includes intravenous injection, intravenous instillation, intramuscular injection, subcutaneous injection, and/or intraneoplasitc injection.

In some embodiments, i) the PD-1 inhibitor or PD-L1 inhibitor, and ii) the STING pathway agonist in the pharmaceutical combination are administered by use of the same or different administration routes.

In some embodiments, it includes injection of the STING pathway agonist into the neoplasm.

In some embodiments, it also includes injection or systemic infusion of the PD-1 inhibitor or PD-L1 inhibitor into the neoplasm.

In some embodiments, it includes injection of the STING pathway agonist and systemic infusion of the PD-1 inhibitor or PD-L1 inhibitor into the neoplasm.

In some embodiments, it includes injection of i) the PD-1 inhibitor or PD-L1 inhibitor STING pathway agonist, and ii) the STING pathway agonist in the pharmaceutical combination into the neoplasm.

In some embodiments, where i) the PD-1 inhibitor or PD-L1 inhibitor, and ii) the STING pathway agonist in the pharmaceutical combination are administered simultaneously or at different times.

In some embodiments, where the PD-1 inhibitor or PD-L1 inhibitor is administered before and/or after the administration of the STING pathway agonist.

In some embodiments, where the PD-1 inhibitor or PD-L1 inhibitor is administered after the administration of the STING pathway agonist.

In some embodiments, the method includes: i) injection of the STING pathway agonist into the neoplasm; ii) injection or systemic infusion of the PD-1 inhibitor or PD-L1 inhibitor into the neoplasm after administering the STING pathway agonist.

In some embodiments, where i) the PD-1 inhibitor or PD-L1 inhibitor, and ii) the STING pathway agonist in the pharmaceutical combination are administered simultaneously.

In some embodiments, where i) the PD-1 inhibitor or PD-L1 inhibitor, and ii) the STING pathway agonist in the pharmaceutical combination are simultaneously administered by way of intraneoplasitc injection, and the PD-1 inhibitor or PD-L1 inhibitor and the STING pathway agonist are present in a same dosage form.

In another aspect, the disclosure provides a pharmaceutical kit including the aforementioned pharmaceutical combination.

Those skilled in the art can have an insight into other aspects and advantages of the disclosure from the detailed description below. The detailed description below only shows and describes exemplary embodiments of the disclosure. As those skilled in the art will recognize, the content of the disclosure enables those skilled in the art to make modifications to the disclosed specific embodiments without departing from the spirit and scope of the invention involved in the disclosure. Correspondingly, the accompanying drawings and the descriptions in the specification of the disclosure are only illustrative, rather than being restrictive.

**Brief Description of the Drawings**

The specific features of the invention involved in the disclosure are shown in the attached claims. The characteristics and advantages of the invention involved in the disclosure can be better understood by referring to the exemplary embodiments and accompanying drawings described in detail hereinafter. A brief explanation of the attached drawings is as follows:

Figure 1 shows the in vivo efficacy study of the pharmaceutical combination of PD-L1 inhibitor RB0005 and 2',3'-cGAMP according to the disclosure, as shown by changes in body weight of each group of mice during administration.

Figure 2 shows the in vivo efficacy study of the pharmaceutical combination of PD-L1 inhibitor RB0005 and 2',3'-cGAMP according to the disclosure, as shown by changes in survival rate of each group of mice during administration.

Figure 3A shows the in vivo efficacy study of the pharmaceutical combination of PD-L1 inhibitor RB0005 and 2',3'-cGAMP according to the disclosure, as shown by changes in tumor proliferation of each group of mice during administration.

Figure 3B shows the in vivo efficacy study of the pharmaceutical combination of PD-L1 inhibitor RB0005 and 2',3'-cGAMP according to the disclosure, as shown by changes in tumor proliferation of each group of mice on day 17 after treatment.

Figure 4 shows the in vivo efficacy study of the pharmaceutical combination of PD-L1 inhibitor RB0005 and 2',3'-cGAMP according to the disclosure, as shown by tumor weight inhibitory rate of mice obtained through dissection after the administration and observation have completed.

Figure 5 shows the in vivo efficacy study of the pharmaceutical combination of PD-1 inhibitor RB0004 and 2',3'-cGAMP according to the disclosure, as shown by changes in tumor proliferation of each group of mice during administration.

Figure 6 shows the in vivo efficacy study of the pharmaceutical combination of PD-1 inhibitor RB0004 and 2',3'-cGAMP according to the disclosure, as shown by tumor weight inhibitory rate of mice obtained through dissection after the administration and observation have completed.

Figure 7 shows the in vivo efficacy study of the pharmaceutical combination of PD-L1 inhibitor RB0005 and different doses of 2',3'-cGAMP according to the disclosure, as shown by tumor weight inhibitory rate of mice obtained through dissection after the administration and observation have completed.

Figure 8 shows the in vivo efficacy study of the pharmaceutical combination of PD-1 inhibitor RB0004 and different doses of 2',3'-cGAMP according to the disclosure, as shown by changes in tumor proliferation of each group of mice during administration.

Figure 9 shows the in vivo efficacy study of the pharmaceutical combination of PD-1 inhibitor RB0004 and different doses of 2',3'-cGAMP according to the disclosure, as shown by changes in survival rate of each group of mice during administration.

Figure 10 shows the trend of tumor proliferation in each group of mice during administration in Example 5 of the disclosure.

Figure 11 shows the survival curve of each group of mice during administration in Example 5 of the disclosure.

Figure 12 shows the mean tumor mass of each group of mice during administration in Example 5 of the disclosure.

Figure 13 shows the trend of tumor proliferation in each group of mice during administration in Example 6 of the disclosure.

Figure 14 shows the survival curve of each group of mice during administration in Example 6 of the disclosure.

Figure 15 shows the mean tumor mass of each group of mice during administration in Example 6 of the disclosure.

Figure 16 shows the trend of tumor proliferation in each group of mice during administration in Example 7.2.1 of the disclosure.

Figure 17 shows the survival curve of each group of mice during administration in Example 7.2.1 of the disclosure.

Figure 18 shows the mean tumor mass of each group of mice during administration in Example 7.2.1 of the disclosure.

Figure 19 shows the trend of tumor proliferation in each group of mice during administration in Example 7.2.2 of the disclosure.

Figure 20 shows the survival curve of each group of mice during administration in Example 7.2.2 of the disclosure.

Figure 21 shows the mean tumor mass of each group of mice during administration in Example 7.2.2 of the disclosure.

Figure 22 shows the trend of tumor proliferation in each group of mice during administration in Example 7.2.3 of the disclosure.

Figure 23 shows the survival curve of each group of mice during administration in Example 7.2.3 of the disclosure.

Figure 24 shows the trend of changes in tumor volume of single mouse in the composite formulation group during administration in Example 7.2.3 of the disclosure.

Figure 25 shows the mean tumor mass of each group of mice during administration in Example 7.2.3 of the disclosure.

Figure 26 shows the relative tumor volume inhibitory rate of each group of mice during administration in Example 8 of the disclosure.

Figure 27 shows the trend of tumor proliferation in each group of mice during administration in Example 9 of the disclosure.

Figure 28 shows the survival curve of each group of mice during administration in Example 9 of the disclosure.

Figure 29 shows the mean tumor mass of each group of mice during administration in Example 9 of the disclosure.

Figure 30 shows the trend of tumor proliferation in each group of mice during administration in Example 10 of the disclosure.

Figure 31 shows the mean tumor volume of each group of mice at the endpoint of the experiment in Example 10 of the disclosure.

Figure 32 shows the survival curve of each group of mice during administration in Example 10 of the disclosure.

Figure 33 shows the trend of changes in tumor volume of single mouse in G5 group during administration in Example 10 of the disclosure.

Figure 34 shows the trend of tumor proliferation in each group of mice during administration in Example 11 of the disclosure.

Figure 35 shows the survival curve of each group of mice during administration in Example 11 of the disclosure.

Figure 36 shows the mean tumor mass of each group of mice during administration in Example 11 of the disclosure.

Figure 37 shows the trend of tumor proliferation in each group of mice during administration in Example 12 of the disclosure.

Figure 38 shows the survival curve of each group of mice during administration in Example 12 of the disclosure.

Figure 39 shows the mean tumor volume of each group of mice on day 7 in Example 12 of the disclosure.

Figure 40 shows the mean tumor mass of each group of mice during administration in Example 12 of the disclosure.

Figure 41 shows the trend of tumor proliferation in each group of mice during administration in Example 13 of the disclosure.

Figure 42 shows the survival curve of each group of mice during administration in Example 13 of the disclosure.

Figure 43 shows the mean tumor mass of each group of mice during administration in Example 13 of the disclosure.

## Detailed Description of the Embodiments

The embodiments of the present invention will be illustrated below by way of specific examples, and other advantages and effects of the present disclosure can be easily understood by those familiar with this technology from the content disclosed in this specification.

Definitions of Terms

In the disclosure, the term "PD-1" generally refers to programmed cell death protein 1, i.e. a type I membrane protein of 288 amino acids first described in 1992 (Ishida et al., EMBO J., 11(1992), 3887-3895). PD-1 is a member of the expanded CD28/CTLA-4T cell regulator family and has two ligands, i.e. PD-L1 (B7-H1 and CD274) and PD-L2 (B7-DC and CD273). The structure of this protein includes an extracellular IgV domain, followed by a transmembrane region and an intracellular tail. The intracellular tail contains two phosphorylation sites located in the immunoreceptor tyrosine-based inhibitory motif and the immunoreceptor tyrosine-based switch motif, indicating a negative regulation of TCR signaling by PD-1. This is consistent with the binding of SHP-1 and SHP-2 phosphatases to the cytoplasmic tail of PD-1 after ligand binding. Although PD-1 is not expressed on naive T cells, it is upregulated after the T cell receptor (TCR) mediated activation and observed on both activated and depleted T cells (Agata et al., Int. Immunology 8 (1996), 765-772). These depleted T cells exhibit a dysfunctional phenotype and are unable to respond appropriately. Although PD-1 has a relatively broad expression pattern, its most important role is likely to act as a co-inhibitory receptor on T cells (Chinai et al., Trends in Pharmacological Sciences 36(2015), 587-595). The current treatment approaches thus focus on blocking the interaction between PD-1 and its ligand to enhance T cell response. In this disclosure, the PD-1 may include human PD-1 (hPD-1), or variants, isotypes, and species homologs thereof, as well as analogues having at least one common epitope with hPD-1. The amino acid sequence of exemplary hPD-1 can be found under GenBank accession No.: U64863.

In this disclosure, the term "PD-L1" generally refers to programmed cell death 1 ligand 1, which can also be referred to as B7 homolog 1, B7-H1, differentiation cluster 274, (3) 274, or CD274, which, when combined with PD-1, downregulates T cell activation and cytokine secretion. "PD-L1" includes any natural PD-L1 from any vertebrate sources, including mammals such as primates (e.g., humans and Macaca fascicularis) and rodents (e.g., mice and rats). The term contemplates "full length", unprocessed PD-L1, and any form of PD-L1 produced by cell processing. PD-L1 can exist as a transmembrane protein or as a soluble protein. "PD-L1" includes the full PD-L1 and fragments thereof, as well as functional variants, isoforms, species homologs, derivatives, analogues of PD-L1, and analogues having at least one common epitope with PD-L1. The basic structure of PD-L1 includes four domains, i.e. extracellular Ig like V-type domain and Ig like C2-type domain, transmembrane domain, and cytoplasmic domain. An exemplary human PD-L1 amino acid sequence can be found under the NCBI accession No.: NP_001254653 or UniProt accession No.: Q9NZQ7.

In this disclosure, the term "inhibitor" generally refers to a compound/substance or composition that can completely or partially prevent or reduce the physiological functions of one or more specific biomolecules (e.g., proteins (such as PD-1 or PD-L1), polypeptides, lipopolysaccharides, glycoproteins, ribonucleoprotein complexes, etc.). The reduce of physiological functions of one or more specific proteins can include a decrease in the activity of the protein itself (e.g., its ability to bind to other molecules, etc.) or a decrease in the existence quantity of the protein itself. Suitable inhibitor molecules can include antagonist antibodies or antibody fragments, small molecular fragments or derivatives, peptides, antisense oligonucleotides, small organic molecules, etc. In some embodiments, the inhibitor can block the activation of cellular signaling pathways. In some embodiments, the PD-1/PD-L1 inhibitor is an anti PD-1/PD-L1 antibody or antigen-binding fragments thereof.

In this disclosure, the terms "Pembrolizumab", "Nivolumab", "Pidilizumab", "SHR-1210", "MEDI0680", "BGB-A317", "TSR-042", "REGN2810", "PF-06801591", "Durvalumab", "Atezolizumab", "Avelumab", "Envafolimab", "MDX-1105", "YW243.55.S70", "MDPL3280A", "AMP-224", "LY3300054", "RB0004", "RB0005", "Tislelizumab", "Camrelizumab", "Toripalimab", "Sintilimab" are used according to their general and ordinary meanings understood in the art.

In this disclosure, the term "STING (stimulator of interferon genes; also known as THEM173, MITA, ERIS, and MPYS)" generally refers to the endoplasmic reticulum (ER) transmembrane proteins expressed in the thymus, spleen, placenta, and THP1 human monocytes. Human STING is encoded by the gene TMEM173. The STING pathway can be activated by exogenous cyclic dinucleotides (CDNs) produced by bacterial infections or structurally different endogenous CDNs (e.g., cyclic GMP-AMP (cGAMP) produced by cyclic GMP-AMP Synthase (cGAS) in response to sensing the cytoplasmic double stranded DNAs (dsDNAs)) (Ablaser et al., 2013; Diner et al., 2013). The cytoplasmic domain of STING forms dimers, and CDNs are bound at the interface of dimers (Burdette, D.L. et al., 2011). After the binding of ligands, the cytoplasmic tail of STING acts as an adapter for TBK-1 and IRF-3, causing their phosphorylation. Phosphorylated IRF-3 enters the nucleus to induce transcription of genes encoding type I IFNs and cytokines used to promote intercellular host immune defenses (Keating et al., 2011). The activation of STING can lead to the production of type I interferons (e.g., IFN-$\alpha$ and IFN-$\beta$) through the IRF3 (Interferon Regulatory Factor 3) pathway and the production of proinflammatory cytokines (IL-1$\alpha$, IL-1$\beta$, IL-2, IL-6, and TNF-$\alpha$, etc.) through the oncogenic transcription factor NF-$\kappa$B (activated B nuclear factor $\kappa$-hght chain enhancer) pathway. The STING pathway can regulate the innate immune recognition of immunogenic tumors and promote the anti-tumor effect of interferon. IFN-$\gamma$ exerts the anti-tumor efficiency through TRAIL (tumor necrosis factor-related apoptosis-inducing ligand) in vivo, promoting apoptosis of tumor cells.

In this disclosure, the term "STING pathway agonist" generally refers to a molecule that binds to STING (stimulator of interferon genes, or TMEM173), activate STING, and triggers the activation of IRF3-TBK1 pathway, leading to increased transcription of type I interferon and other genes.

In this disclosure, the term "cyclic dinucleotide (CDN)" generally refers to a class of cyclic molecules having two phosphodiester bonds or two thiophosphate diester bonds, or one phosphodiester diester bond and one thiophosphate diester bond, between two nucleosides. Exemplary cyclic dinucleotides include but are not limited to 3'-5'c-di-AMP(c-di-AMP), 3'-5'c-di-GMP(c-di-GMP), c-di-GMP-F, 3',3'cGAMP (also known as 3'-5',3'-5'cGAMP, a product of Vibrio cholerae DncV protein), 3',3'-cGAMP-F, 2',3'-cGAMP (also known as 2'-5',3'-5'cGAMP, a product of human cGAS protein), Rp/Sp(CL656), ADU-S100, ADU-S100 disodium and a combination thereof.

In this disclosure, the term "nucleoside" generally refers to a glycosylamine containing a nitrogen containing base and a pentose, wherein the nitrogen containing base binds to pentose through a β-glucosidic bond. The term "nucleotide" generally refers to any nucleoside linked to a phosphate group at positions 5', 3' or 2' in the sugar moiety.

In this disclosure, the term "antibody" generally refers to one being used in the broadest sense and specifically covering monoclonal antibodies, polyclonal antibodies, dimers, multimers, multispecific antibodies (e.g., bispecific antibodies), and antibody fragments, as long as they exhibit the desired biological activities (Miller et al. (2003) Jour. of Immunology 170:4854-4861). Antibodies can be mouse, human, humanized, chimeric antibodies, or those derived from other species.

A full-length antibody typically refers to an antibody composed of two "full-length antibody heavy chains" and two "full-length antibody light chains". The "full length antibody heavy chain" is generally such a polypeptide that is composed of the antibody heavy chain variable domain (VH), antibody constant heavy chain domain 1 (CH1), antibody hinge region (HR), antibody heavy chain constant domain 2 (CH2), and antibody heavy chain constant domain 3 (CH3) in the direction of N-terminal to C-terminal, abbreviated as VH-CH1-HR-CH2-CH3; and in the case of IgE subtype antibodies, optionally further including the antibody heavy chain constant domain 4 (CH4). In some embodiments, the "full length antibody heavy chain" is a polypeptide composed of VH, CH1, HR, CH2, and CH3 in the direction of N-terminal to C-terminal. The "full length antibody light chain" is generally a polypeptide composed of an antibody light chain variable domain (VL) and an antibody light chain constant domain (CL) in the direction of N-terminal to C-terminal, abbreviated as VL-CL. The antibody light chain constant domain (CL) can be κ(kappa) or λ(lambda). Two full-length antibody chains are linked together by an interpolypeptide disulfide bond between the CL domain and the CH1 domain, and an interpolypeptide disulfide bond between the hinge regions of the full-length antibody heavy chain. Typical examples of full-length antibodies are natural antibodies such as IgG (e.g., IgG1 and IgG2), IgM, IgA, IgD, and IgE).

In this disclosure, the term "antigen binding fragment" generally refers to a portion of the antibody molecule that contains amino acids responsible for the specific binding between the antibody and the antigen. The part of an antigen that is specifically recognized and bound by an antibody is called as an "epitope" as mentioned above. The antigen binding domain can typically include the antibody light chain variable region (VL) and the antibody heavy chain variable region (VH); however, it does not necessarily include both. Fd fragments for example have two VH regions, and generally retain some antigen-binding functions of the whole antigen-binding domain. Examples of antigen binding fragments of an antibody include (1) Fab fragments, monomeric fragments having VL, VH, constant light chain (CL), and CH1 domains; (2) F(ab')$_2$ fragments, diatomic fragments having two Fab fragments linked by a disulfide bridge of the hinge region; (3) Fd fragments having two VH and CH1 domains; (4) Fv fragments having single-armed VL and VH domains of antibodies, (5) dAb fragments (Ward et al., "Binding Activities of a Repertoire of Single Immunoglobulin Variable Domains Secreted From Escherichia coli,"Nature 341:544-546(1989), incorporated by reference into the present disclosure in its entirety), having VH domains; (6) isolated complementary determining region (CDR); (7) single chain Fv (scFv), for example, derived from the scFV library. Although the two domains VL and VH of the Fv fragment are encoded by independent genes, they can be conjugated using a recombination method by a synthetic linker, the synthetic linker enables it to be made into a single protein chain (called as single chain Fv (scFv)) in which the VL and VH regions pair to form a monomeric molecule (see, for example, Huston et al., "Protein Engineering of Antibody Binding Sites: Recovery of Specific Activity in an Anti-Digoxin Single-Chain Fv Analogue Produced in Escherichia coli,"Proc.Natl.Acad.Sci.USA 85:5879-5883(1988)); and (8) VHH, "VHH" involves variable antigen-binding domains of heavy chain antibodies from the Camelidae (camels, dromedaries, llamas, and alpacas, etc.) (see Nguyen V.K. et al., 2000, The EMBO Journal, 19, 921-930; Muyldermans S., 2001, J Biotechnol., 74, 277-302; and Review Vanlandschoot P. et al., 2011, Antiviral Research 92, 389-407). VHH can also be referred to as nanobody (Nb) and/or single domain antibody. These antibody fragments can be obtained using conventional techniques known to those skilled in the art, and their functionalities can be evaluated in the same way as intact antibodies.

In this disclosure, the terms "variable region" or "variable domain" generally refer to regions where there may be significant differences in sequence of certain segments of the variable domains between antibodies. The "variable region" in a light chain can include the light chain variable region VL; and the "variable region" in a heavy chain can include the heavy chain variable region VH. Variable domains mediate antigen binding and determine the specificity of specific antibodies against their specific antigens. However, variability does not mean the uniform distribution throughout the entire scope of variable domains. It is generally concentrated in three segments called as hypervariable regions (CDR or HVR) in the light chain and heavy chain variable domains. The more highly conserved part of the variable domains is called as the framework region (FR). Each of the variable domains of natural heavy and light chains contains four FR regions, most of which adopt β-sheet configuration and are connected by three CDRs, forming a circular connection, and in some cases forming a part of a β-sheet structure. The CDRs in each chain are closely held together through the FR region, and promote the formation of antigen-binding sites of antibodies together with the CDRs from another chain (see Kabat et al, Sequences of Immunological Interest, Fifth Edition, National Institute of Health,Bethesda,Md.(1991)). The terms "VH" and "VH domain" can be interchangeably used to refer to the heavy chain variable region of an antibody or antigen-binding molecule thereof.

In this disclosure, the term "CDR" generally refers to the complementary determining region within the antibody variable sequence. There are three CDRs in each variable region of the heavy and light chains, and for each variable region, they are called as CDR1, CDR2, and CDR3, respectively. The precise boundaries of these CDRs have been defined differently based on different systems. The system described by Kabat (Kabat et al., Sequences of Proteins of Immunological Interest (National Institutes of Health, Bethesda, Md. (1987) and (1991)) not only provides a definite residue numbering system applicable to any variable region of the antibody, but also provides precise residue boundaries that define these three CDRs. These CDRs can be referred to as Kabat CDRs. Chothia and co-workers (Chothia&Lesk, J.Mol.Biol.196:901-917(1987) and Chothia et al., Nature 342:877-883(1989)) found that some sub-moieties within Kabat CDR exhibit almost identical peptide skeleton conformations, despite significant differences at the amino acid sequence level. These sub-moieties are designated as L1, L2, and L3, or H1, H2, and H3, where "L" and "H" refer to the light and heavy chain regions, respectively. These regions can be called as Chothia CDRs, which have boundaries that overlap with Kabat CDRs. Padlan (FASEB J.9:133-139(1995)) and MacCallum (JMol Biol 262(5):732-45(1996)) have already described other boundaries that define CDRs overlapping with Kabat CDRs. Other CDR boundaries may not strictly conform to one of the above systems, but still overlap with Kabat CDR. Although they can be shortened or extended based on predictions or experimental findings below, specific residues or groups of residues, or even the whole CDRs, do not significantly affect the antigen binding. Unless otherwise explicitly stated in the specification, as used in this disclosure, the terms "CDR", "HCDR1", "HCDR2", "HCDR3", "LCDR1", "LCDR2", and "LCDR3" include CDRs as defined in any of the aforementioned methods (Kabat, Chothia, or IMGT).

In this disclosure, the term "percentage (%) sequence identity" generally refers to the number of matches ("hits") of consistent amino acids when two or more aligned amino acid sequences are compared with the number of amino acid residues making up the total length of the amino acid sequences. In other words, when alignment is used for two or more sequences, the percentage of identical amino acid residues (e.g., 70%, 75%, 80%, 85%, 90%, 95%, 96%, 97%, 98%, or 99% sequence identity) can be determined when comparing and aligning these sequences against the maximum correspondence (as measured using sequence comparison algorithms known in the art), or when manually aligning and visually inspecting. Therefore, sequences that are compared to determine sequence identity can be distinguished by one or more amino acid substitutions, additions, or deletions. The suitable programs for alignment of protein sequences are known to those skilled in the art. The percentage sequence identity of protein sequences can be determined, for example, using programs such as CLUSTALW, Cluster Omega, FASTA, or BLAST, such as the use of NCBI BLAST algorithm (AltschulSF et al. (1997), Nuclear Acids Res. 25:3389-3402).

In this disclosure, the terms "combination", "therapeutic combination", "combination therapy", or "pharmaceutical combination" generally refer to a combination including at least two active ingredients/therapeutic agents, where a STING pathway agonist and a PD-1/PD-L1 inhibitor can be administered independently at the same time or separately within time intervals that allow the combinations to exhibit cooperation (e.g. synergistic) effects. In some embodiments, each active ingredient/therapeutic agent can be respectively prepared into an independent preparation (solid, liquid, and gel, etc.). In some embodiments, each active ingredient/therapeutic agent can be contained in different containers, and can also be prepared into the desired preparations concurrently or separately with an appropriate carrier as needed. In some embodiments, each active ingredient/therapeutic agent can be of different sources (e.g., prepared, produced by different manufacturers, or sold by different merchants). In some embodiments, each active ingredient/therapeutic agent can form pharmaceutical compositions in a mixed form.

In this disclosure, the term "pharmaceutical composition" generally refers to a preparation being in a form that allows the biological activity of active ingredients to be effective and containing no other components that have unacceptable toxicity to the subject for who the composition is going to be administered. Such composition can be sterile and can contain pharmaceutically acceptable carriers, such as physiological saline. A suitable pharmaceutical composition may include one or more buffer solutions (e.g., acetate, phosphate, or citrate buffer solutions), surfactants (e.g., polysorbate), stabilizers (e.g., human albumin), preservatives (e.g., benzyl alcohol), absorption enhancers for enhancing bioavailability, and/or other conventional solubilizers or dispersants. The pharmaceutical compositions of the disclosure include but are not limited to liquid, frozen, and lyophilized compositions.

In this disclosure, the term "pharmaceutically acceptable carrier" generally refers to one or more non-toxic materials that do not interfere with the biological activity of the active ingredients. Such preparation can conventionally contain a salt, a buffer, a preservative, a compatible carrier, and optionally other therapeutic agents. Such pharmaceutically acceptable preparation can also contain a compatible solid or liquid filler, a diluent, or an encapsulating substance suitable for human administration. Other contemplated carriers, excipients, and/or additives that can be used in the formulations described herein may include, for example, a flavouring agent, an antimicrobial agent, a sweetener, an antioxidant, an antistatic agent, lipid, a protein excipient (e.g., serum albumin, gelatin, casein), a salt-forming counterion (e.g., sodium), and the like. These and other known pharmaceutical carriers, excipients, and/or additives suitable for use in the formulations described herein are known in the art.

In this disclosure, the term "neoplastic cells" generally refers to cells that undergo new and abnormal proliferation, especially those in which proliferation is uncontrollable and progresses, leading to neoplastic diseases. Neoplastic cells can be malignant, i.e., invasive and metastatic, or benign.

In this disclosure, the term "neoplasm" generally refers to an abnormal lump of tissues, where the growth of the lump exceeds that of normal tissues and is not coordinated with the growth of normal tissues. The "neoplasm" can be defined to be "benign" or "malignant", depending on the following characteristics: degree of cell differentiation, including morphology and function, rate of growth, local invasion and metastasis. "Benign neoplasm" is generally well-differentiated, has slower growth characteristics than malignant neoplasm, and maintains localization to the origin site. In addition, benign tumors do not have the ability to infiltrate, invade, or metastasize to the distal sites. "Malignant neoplasm" is generally poorly differentiated (anaplastic) and has characteristic rapid growth, accompanied by progressive infiltration, invasion, and destruction of surrounding tissues. In addition, malignant neoplasm has the ability to metastasize to the distal sites.

In this disclosure, the term "tumor" or "cancer" generally refers to any medical disorders characterized by the growth, proliferation, or metastasis of neoplastic or malignant cells, and the tumor can be a solid or non-solid tumor.

In this disclosure, the term "wart" generally refers to a type of superficial benign neoplasm of the skin caused by human papilloma virus (HPV), mainly characterized by cell proliferative responses. HPV belongs to the Papovaviridae A genus in the DNA virus family. HPV is divided into more than 80 subtypes and is associated with different types of warts. The term "wart" generally refers to all types of warts, including but not limiting to verruca plantaris, verruca vulgaris, and genital warts, unless otherwise specified.

In this disclosure, the term "administer" and similar terms are generally not limited to bodily administration, and suitable methods include in vitro, in vitro followed by in vivo, or in vivo methods. For example, any administration method known to those skilled in the art for bringing cells, organs, or tissues into contact with the composition can be used. For example, the compound can be introduced into the body of a subject in need of treatment through any introduction or delivery routes. In some embodiments, the composition of the disclosure can be administered orally, locally, intranasally, intramuscularly, subcutaneously, intradermally, intrathecally, intraperitoneally, percutaneously, or intratumorally.

In this disclosure, the term "effective amount" or "effective dose" generally refers to an amount sufficient to achieve or at least partially achieve the desired effect. The "therapeutically effective amount" or "therapeutically effective dose" of a drug or therapeutic agent is generally any amount of a drug that promotes regression of a disease when used alone or in combination with another therapeutic agent (as evidenced by a decrease in the severity of disease symptoms, an increase in the frequency and duration of asymptomatic periods of a disease, or the prevention of damage or disability caused by suffering from a disease).

In this disclosure, the term "treatment" generally refers to the slowing down or improvement of the progression, severity, and/or duration of proliferative disorders, or the improvement of one or more symptoms of proliferative disorders (e.g., one or more identifiable symptoms) due to the administration of one or more therapies (e.g., one or more therapeutic agents such as the pharmaceutical composition of this disclosure). In this disclosure, the term "treatment" may also refer to the improvement of at least one measurable physical parameter of proliferative disorders, such as tumor growth, not necessarily to be distinguishable by the patient. The term "treatment" in this disclosure can also refer to the inhibition of the progression of proliferative disorders through, for example, stabilizing the identifiable symptoms by physical means (for example, stabilizing physical parameters), or by physiological means, or both. In some cases, the term "treatment" can refer to reduction or stabilization of tumor size or cancer cell counts.

In this disclosure, the term "synergy" generally refers to the efficacy of the combination of two or more active drugs being greater than the sum of efficacies obtained by using the individual drugs alone. Therefore, when the combination of two or more pharmaceutical ingredients leads to a "synergistic inhibition" of an activity or process, such as tumor growth, it means that the inhibitory effect on the activity or process is greater than the sum of the inhibitory effects of the active drugs alone. In this disclosure, the synergistic effect calculation of pharmaceutical combinations can be evaluated using the coefficient of drug interaction (CDI/CI). CDI(CI) is calculated according to the following formula: CDI(CI)=AB/A*B. AB is the ratio of the combination group of two drugs to the control group, as calculated based on the anatomical tumor weight. A or B is the ratio of single drug group to control group. If CDI is less than 1, it indicates that the two drugs have a synergistic effect; If CDI equals to 1, the two drugs have an additive effect; If CDI is greater than 1, the two drugs have an antagonistic effect. (Pikman,Yet al, Synergistic Drug Combinations with a CDK4/6 Inhibitor in T-cell Acute Lymphoblastic Leukemia, (2017) Clin Cancer Res 23,1012-1024.PMID:28151717 PMCID:PMC5432118 DOI:10.1158/1078-0432.CCR-15-2869.)

In this disclosure, the term "subject" generally refers to human or non-human animals, including but not limiting to cats, dogs, horses, pigs, cows, sheep, rabbits, mice, rats, or monkeys, etc.

In this disclosure, the term "about" generally refers to a variation within a range of (0.5% to 10%) above or below the specified value, such as a variation within a range of about 0.5%, about 1%, about 1.5%, about 2%, about 2.5%, about 3%, about 3.5%, about 4%, about 4.5%, about 5%, about 5.5%, about 6%, about 6.5%, about 7%, about 7.5%, about 8%, about 8.5%, about 9%, about 9.5%, or about 10% above or below the specified value.

In this disclosure, the term "comprising" and its variant forms including "containing", "including" and other forms, generally refer to the inclusion of other components, elements, numerical values, and steps, etc.

**DETAILED DESCRIPTION OF INVNTION**

This disclosure provides an agonist that induces the expression of INF and cytokines as well as activates innate immune system responses in combination with an immune checkpoint inhibitor for use as a pharmaceutical combination, including a STING agonist (e.g., cyclic dinucleotide 2'3'-cGAMP or derivatives thereof) and an immune checkpoint inhibitor (PD-1/PD-L1), etc.

In one aspect, the disclosure provides a pharmaceutical combination, which can include include a programmed cell death protein 1 (PD-1) inhibitor and/or a programmed death ligand 1 (PD-L1) inhibitor, and a STING pathway agonist.

In some embodiments, where the STING pathway agonist includes a cyclic dinucleotide.

In some embodiments, where the cyclic dinucleotide is selected from the group consisting of c-di-AMP, c-di-GMP, c-di-GMP-F, 3',3'-cGAMP, 3',3'-cGAMP-F, 2',3'-cGAMP, Rp/Sp(CL656), ADU-S100, ADU-S100 disodium, and derivatives or combinations thereof.

In some embodiments, where the STING pathway agonist includes 2',3'-cGAMP, or derivatives thereof.

In some embodiments, where the STING pathway agonist includes flavonoids.

In some embodiments, where the flavonoid includes CMA, DMXAA, methoxyflavone, 6,4'-dimethoxyflavone, 4'-methoxyflavone, 3',6'-dihydroxyflavone, 7,2'-dihydroxyflavone, daidzein, Formononetin, retinene 7-methyl ether, xanthone, and/or any combination thereof.

In some embodiments, where the STING pathway agonist includes DNA.

In some embodiments, where the STING pathway agonist includes type I interferons (IFNs).

In some embodiments, where the type I interferon includes IFN-$\alpha$ or IFN-$\beta$.

In some embodiments, where the PD-1 inhibitor has one or more of the following characteristics:

a. inhibition or reduction of PD-1 expression, such as transcription or translation of PD-1;

b. inhibition or reduction of PD-1 activity, such as inhibition or reduction of PD-1 binding to its homologous ligands, such as PD-L1 or PD-L2; and

c. binding PD-1 or one or more of its ligands, such as PD-L1 or PD-L2.

In some embodiments, where the PD-1 inhibitor includes an anti PD-1 antibody or antigen-binding fragments thereof.

For example, the pharmaceutical combination can include: 1) an anti PD-1 antibody or antigen-binding fragments thereof; and 2) a cyclic dinucleotide.

In some embodiments, where the anti PD-1 antibody is selected from the group consisting of Pembrolizumab, Nivolumab, Pidilizumab, SHR-1210 (Incyte/Jiangsu Hengrui Pharmaceuticals Co., Ltd.), MEDI0680 (also known as AMP-514; Amplimmune Inc./Medimmune), BGB-A317 (BeiGene Ltd.), TSR-042 (also known as ANB011; Anaptys-Bio/Tesaro, nc.), REGN2810 (Regeneron Pharmaceuticals, Inc./Sanofi-Aventis), PF-06801591 (Pfizer), RB0004, Tislelizumab, Camrelizumab, Toripalimab, Sintilimab, bio-analogues, bio-enhancers, bio-equivalents, or combinations thereof.

For example, the pharmaceutical combination can include: 1) an anti PD-1 antibody, which can be selected from the group consisting of Pembrolizumab, Nivolumab, Pidilizumab, SHR-1210 (Incyte/Jiangsu Hengrui Pharmaceuticals Co., Ltd.), MEDI0680 (also known as AMP-514; Amplimmune Inc./Medimmune), BGB-A317 (BeiGene Ltd.), TSR-042 (also known as ANB011; AnaptysBio/Tesaro, nc.), REGN2810 (Regeneron Pharmaceuticals, Inc./Sanofi-Aventis), PF-06801591 (Pfizer), RB0004, bio-analogues, bio-enhancers, bio-equivalents, or combinations thereof; and 2) a cyclic dinucleotide, which can be selected from the group consisting of c-di-AMP, c-di-GMP, c-di-GMP-F, 3',3'-cGAMP, 3',3'-cGAMP-F, 2',3'-cGAMP, Rp/Sp(CL656), ADU-S100, ADU-S100 disodium or combinations thereof.

In some embodiments, the anti PD-1 antibody is RB0004. RB0004 and other humanized anti PD-1 monoclonal antibodies are disclosed in CN201610345750.1 and WO2017201766A1. For example, the pharmaceutical combination includes: 1) the anti PD-1 antibody, which is RB0004 or bio-enhancers, and bio-equivalents, as well as combinations thereof; and 2) a STING pathway agonist including 2',3'-cGAMP or derivatives thereof.

In another embodiments, the antibody PD-1 is Pembrolizumab (trade name Keytruda, previously Lambrolzumab, also known as Merck 3745, MK-3475, or SCH-900475), a humanized IgG4 monoclonal antibody that binds to PD-1. Pembrolizumab is disclosed in, for example Hamid et al. (2013) New England Journal of Medicine 369(2):134-44, WO2009/114335 and US 8,354,509. For example, the pharmaceutical combination includes: 1) the anti PD-1 antibody, which is Pembrolizumab or bio-enhancers, and bio-equivalents, or combinations thereof; and 2) a STING pathway agonist including 2',3'-cGAMP or derivatives thereof.

In another embodiments, the anti PD-1 antibody is Nivolumab (CAS Registry No.: 946414-94-4, alternative names include MDX-1106, MDX-1106-04, ONO-4538, or BMS-936558). Nivolumab is a complete human IgG4 monoclonal antibody that specifically blocks PD-1. Nivolumab (Clone 5C4) and other human monoclonal antibodies specifically binding to PD-1 have been disclosed in US8,008,449 and W02006/121168. For example, the pharmaceutical combination includes: 1) the anti PD-1 antibody, which is Nivolumab or bio-enhancers, and bio-equivalents, as well as combinations thereof; and 2) a STING pathway agonist including 2',3'-cGAMP or derivatives thereof.

In another embodiments, the anti PD-1 antibody is Pidilizumab. Pidilizumab (CT-011; Cure Tech) is a humanized IgGI monoclonal antibody that binds to PD-1. Pidilizumab and other humanized anti PD-1 monoclonal antibodies are disclosed in W02009/101611. Other anti Rpd-1 antibodies have been disclosed in US 8,609,089, US2010028330, and/or US20120114649. Other anti PD-1 antibodies include AMP514 (Amplimmune). For example, the pharmaceutical combination includes: 1) the anti PD-1 antibody, which is Pidilizumab or bio-enhancers, and bio-equivalents, as well as combinations thereof; and 2) a STING pathway agonist including 2',3'-cGAMP or derivatives thereof.

In some embodiments, where the anti PD-1 antibody includes at least one CDR in the antibody heavy chain variable region (VH), and the VH includes an amino acid sequence shown in SEQ ID NO: 8.

In some embodiments, where the anti PD-1 antibody includes VH including HCDR3, the HCDR3 includes an amino acid sequence shown in SEQ ID NO: 3 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 3.

In some embodiments, where the VH further includes HCDR2, wherein the HCDR2 includes an amino acid sequence shown in SEQ ID NO: 2 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 2.

In some embodiments, where the VH further includes HCDR1, wherein the HCDR1 includes an amino acid sequence shown in SEQ ID NO: 1 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 1.

In some embodiments, where the VH includes HCDR1, HCDR2, and HCDR3, wherein the HCDR3 includes an amino acid sequence shown in SEQ ID NO: 3 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 3, the HCDR2 includes an amino acid sequence shown in SEQ ID NO: 2 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 2, and the HCDR1 includes an amino acid sequence shown in SEQ ID NO: 1 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 1.

In some embodiments, where the VH includes a framework region HFR1, wherein the C-terminal of HFR1 is directly or indirectly connected to the N-terminal of HCDR1, and the HFR1 includes an amino acid sequence shown in SEQ ID NO: 4 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 4.

In some embodiments, where the VH includes a framework region HFR2, wherein the N-terminal of HFR2 is directly or indirectly connected to the C-terminal of HCDR1, and the C-terminal of HFR2 is directly or indirectly connected to the N-terminal of HCDR2; and the HFR2 includes an amino acid sequence shown in SEQ ID NO: 5 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 5.

In some embodiments, where the VH includes a framework region HFR3, wherein the N-terminal of HFR3 is directly or indirectly connected to the C-terminal of HCDR2, and the C-terminal of HFR3 is directly or indirectly connected to the N-terminal of HCDR3; and the HFR3 includes an amino acid sequence shown in SEQ ID NO: 6 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 6.

In some embodiments, where the VH includes a framework region HFR4, wherein the N-terminal of HFR4 is directly or indirectly connected to the C-terminal of HCDR3, and the HFR4 includes an amino acid sequence shown in SEQ ID NO: 7 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 7.

In some embodiments, where the VH includes framework regions HFR1, HFR2, HFR3, and HFR4, the C-terminal of HFR1 is directly or indirectly connected to the N-terminal of HCDR1, the N-terminal of HFR2 is directly or indirectly connected to the C-terminal of HCDR1, and the C-terminal of HFR2 is directly or indirectly connected to the N-terminal of HCDR2, the N-terminal of HFR3 is directly or indirectly connected to the C-terminal of HCDR2, and the C-terminal of HFR3 is directly or indirectly connected to the N-terminal of HCDR3, the N-terminal of HFR4 is directly or indirectly connected to the C-terminal of HCDR3; among them, the HFR1 includes an amino acid sequence shown in SEQ ID NO: 4 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 4, the HFR2 includes an amino acid sequence shown in SEQ ID NO: 5 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 5, the HFR3 includes an amino acid sequence shown in SEQ ID NO: 6 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 6, and the HFR4 includes an amino acid sequence shown in SEQ ID NO: 7 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 7.

In some embodiments, where the anti PD-1 antibody includes VH, the VH includes an amino acid sequence shown in SEQ ID NO: 8 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 8.

In some embodiments, where the anti PD-1 antibody includes an antibody heavy chain (HC), the HC includes an amino acid sequence shown in SEQ ID NO: 9 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 9.

In some embodiments, where the anti PD-1 antibody includes at least one CDR in the antibody light chain variable region (VL), and the VL includes an amino acid sequence shown in SEQ ID NO: 17.

In some embodiments, where the anti PD-1 antibody includes at least one CDR in VH, wherein the VH includes an amino acid sequence shown in SEQ ID NO: 8, and the anti PD-1 antibody includes at least one CDR in VL, and the VL includes an amino acid sequence shown in SEQ ID NO: 17.

In some embodiments, where the anti PD-1 antibody includes VL including LCDR1, the LCDR1 includes an amino acid sequence shown in SEQ ID NO: 10 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 10.

In some embodiments, where the VL further includes LCDR2, wherein the LCDR2 includes an amino acid sequence shown in SEQ ID NO: 11 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 11.

In some embodiments, where the VL further includes LCDR3, wherein the LCDR3 includes an amino acid sequence shown in SEQ ID NO: 12 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 12.

In some embodiments, where the VL includes LCDR1, LCDR2 and LCDR3, wherein the LCDR1 includes an amino acid sequence shown in SEQ ID NO: 10 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 10, the LCDR2 includes an amino acid sequence shown in SEQ ID NO: 11 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 11, and the LCDR3 includes an amino acid sequence shown in SEQ ID NO: 12 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 12.

In some embodiments, where the anti PD-1 antibody includes VH and an antibody VL, the VH includes HCDR1, HCDR2, and HCDR3, wherein the HCDR3 includes an amino acid sequence shown in SEQ ID NO: 3 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 3, the HCDR2 includes an amino acid sequence shown in SEQ ID NO: 2 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 2, and the HCDR1 includes an amino acid sequence shown in SEQ ID NO: 1 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 1; and the VL includes LCDR1, LCDR2, and LCDR3, wherein the LCDR1 includes an amino acid sequence shown in SEQ ID NO: 10 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 10, the LCDR2 includes an amino acid sequence shown in SEQ ID NO: 11 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 11, and the LCDR3 includes an amino acid sequence shown in SEQ ID NO: 12 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 12.

For example, the anti PD-1 antibody can include VH and antibody VL, the VH can include HCDR1, HCDR2, and HCDR3, wherein the HCDR3 can include an amino acid sequence shown in SEQ ID NO: 3, the HCDR2 can include an amino acid sequence shown in SEQ ID NO: 2, and the HCDR1 can include an amino acid sequence shown in SEQ ID NO: 1; and the VL can include LCDR1, LCDR2, and LCDR3, wherein the LCDR1 can include an amino acid sequence shown in SEQ ID NO: 10, the LCDR2 can include an amino acid sequence shown in SEQ ID NO: 11, and the LCDR3 can include an amino acid sequence shown in SEQ ID NO: 12.

For example, the pharmaceutical combination can include: 1) anti PD-1 antibody, the anti PD-1 antibody can include VH and antibody VL, the VH can include HCDR1, HCDR2, and HCDR3, wherein the HCDR3 can include an amino acid sequence shown in SEQ ID NO: 3, the HCDR2 can include an amino acid sequence shown in SEQ ID NO: 2, and the HCDR1 can include an amino acid sequence shown in SEQ ID NO: 1; and the VL can include LCDR1, LCDR2, and LCDR3, wherein the LCDR1 can include an amino acid sequence shown in SEQ ID NO: 10, the LCDR2 can include an amino acid sequence shown in SEQ ID NO: 11, and the LCDR3 can include an amino acid sequence shown in SEQ ID NO: 12; 2) a STING pathway agonist, which can be 2',3'-cGAMP, or derivatives thereof.

In some embodiments, where the VL includes a framework region LFR1, the C-terminal of LFR1 is directly or indirectly connected to the N-terminal of LCDR1, and the LFR1 includes an amino acid sequence shown in SEQ ID NO: 13 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 13.

In some embodiments, where the VL includes a framework region LFR2, wherein the N-terminal of LFR2 is directly or indirectly connected to the C-terminal of LCDR1, and the C-terminal of LFR2 is directly or indirectly connected to the N-terminal of LCDR2; and the LFR2 includes an amino acid sequence shown in SEQ ID NO: 14 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 14.

In some embodiments, where the VL includes a framework region LFR3, wherein the N-terminal of LFR3 is directly or indirectly connected to the C-terminal of LCDR2, and the C-terminal of LFR3 is directly or indirectly connected to the N-terminal of LCDR3; and the LFR3 includes an amino acid sequence shown in SEQ ID NO: 15 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 15.

In some embodiments, where the VL includes a framework region LFR4, wherein the N-terminal of LFR4 is directly or indirectly connected to the C-terminal of LCDR3, and the LFR4 includes an amino acid sequence shown in SEQ ID NO: 16 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 16.

In some embodiments, where the VL includes framework regions LFR1, LFR2, LFR3 and LFR4, the C-terminal of LFR1 is directly or indirectly connected to the N-terminal of LCDR1, the N-terminal of LFR2 is directly or indirectly connected to the C-terminal of LCDR1, and the C-terminal of LFR2 is directly or indirectly connected to the N-terminal of LCDR2, the N-terminal of LFR3 is directly or indirectly connected to the C-terminal of LCDR2, and the C-terminal of LFR3 is directly or indirectly connected to the N-terminal of LCDR3, the N-terminal of LFR4 is directly or indirectly connected to the C-terminal of LCDR3; among them, the LFR1 includes an amino acid sequence shown in SEQ ID NO: 13 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 13, the LFR2 includes an amino acid sequence shown in SEQ ID NO: 14 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 14, the LFR3 includes an amino acid sequence shown in SEQ ID NO: 15 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 15, and the LFR4 includes an amino acid sequence shown in SEQ ID NO: 16 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 16.

In some embodiments, where the anti PD-1 antibody includes VL, the VL includes an amino acid sequence shown in SEQ ID NO: 17 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 17.

In some embodiments, where the anti PD-1 antibody includes VH and VL, the VH includes an amino acid sequence shown in SEQ ID NO: 8 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 8, and the VL includes an amino acid sequence shown in SEQ ID NO: 17 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 17.

For example, where the anti PD-1 antibody can include VH and VL, the VH can include an amino acid sequence shown in SEQ ID NO: 8, and the VL can include an amino acid sequence shown in SEQ ID NO: 17.

For example, the pharmaceutical combination can include: 1) anti PD-1 antibody, wherein the anti PD-1 antibody can include VH and VL, the VH can include an amino acid sequence shown in SEQ ID NO: 8, and the VL can include an amino acid sequence shown in SEQ ID NO: 17; 2) a STING pathway agonist, which can be 2',3'-cGAMP or derivatives thereof.

In some embodiments, where the anti PD-1 antibody includes an antibody light chain (LC), the LC includes an amino acid sequence shown in SEQ ID NO: 18 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 18.

In some embodiments, where the anti PD-1 antibody includes HC and LC, the HC includes an amino acid sequence shown in SEQ ID NO: 9 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 9, and the LC includes an amino acid sequence shown in SEQ ID NO: 18 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 18.

For example, where the anti PD-1 antibody can include HC and LC, the HC can include an amino acid sequence shown in SEQ ID NO: 9, and the LC can include an amino acid sequence shown in SEQ ID NO: 18.

For example, the pharmaceutical combination can include: 1) anti PD-1 antibody, wherein the anti PD-1 antibody can include HC and LC, the HC can include an amino acid sequence shown in SEQ ID NO: 9, and the LC can include an amino acid sequence shown in SEQ ID NO: 18; 2) a STING pathway agonist, which can be 2',3'-cGAMP or derivatives thereof.

In some embodiments, where the PD-L1 inhibitor has one or more of the following characteristics:

a. inhibition or reduction of PD-L1 expression, such as transcription or translation of PD-L1;

b. inhibition or reduction of PD-L1 activity, such as inhibition or reduction of PD-L1 binding to its associated receptors such as PD-1; and

c. binding of PD-L1 or its receptors such as PD-1.

In some embodiments, where the PD-L1 inhibitor includes an anti PD-L1 antibody or antigen-binding fragments thereof.

For example, the pharmaceutical combination can include: 1) an anti PD-L1 antibody or antigen-binding fragments thereof; and 2) a cyclic dinucleotide.

In some embodiments, where the anti PD-L1 antibody is selected from the group consisting of Durvalumab (MEDI4736, disclosed in US2013/0034559A1), Atezolizumab (MPDL3280A, disclosed in US8,217,149), Avelumab (MSB0010718C, disclosed in US2014/0341917A1), MDX-1105, YW243.55.S70, MDPL3280A, AMP-224 (Amplimume, GlaxoSmithKline), LY3300054 (Eli Lilly and Co.), RB0005, bio-analogues, bio-enhancers, bio-equivalents, or combinations thereof.

For example, the pharmaceutical combination can include: 1) an anti PD-L1 antibody, wherein the anti PD-L1 antibody is selected from the group consisting of Durvalumab (MEDI4736, disclosed in US2013/0034559A1), Atezolizumab (MPDL3280A, disclosed in US8,217,149), Avelumab (MSB0010718C, disclosed in US2014/0341917A1), MDX-1105, YW243.55.S70, MDPL3280A, AMP-224, LY3300054, RB0005, bio-analogues, bio-enhancers, bio-equivalents, or combinations thereof; and 2) a cyclic dinucleotide, which is selected from the group consisting of c-di-AMP, c-di-GMP, c-di-GMP-F, 3',3'-cGAMP, 3',3'-cGAMP-F, 2',3'-cGAMP, Rp/Sp(CL656), ADU-S100, ADU-S100 disodium, or combinations thereof.

In some embodiments, the anti PD-L1 antibody is RB0005. RB0005 and other humanized anti PD-L1 monoclonal antibodies have been disclosed in CN201610340678.3 and WO2017197667A1. For example, the pharmaceutical combination includes: 1) an anti PD-L1 antibody, which is RB0005, or bio-enhancers, bio-equivalents, or combinations thereof; and 2) a STING pathway agonist including 2',3'-cGAMP or derivatives thereof.

In other embodiments, the PD-LI inhibitor is MDX-1105. MDX-1105, also known as BMS-936559, is an anti PD-LI antibody described in WO 2007/005874. For example, the pharmaceutical combination includes: 1) an anti PD-L1 antibody, which is MDX-1105, or bio-enhancers, bio-equivalents, or combinations thereof; and 2) a STING pathway agonist including 2',3'-cGAMP or derivatives thereof.

In other embodiments, the PD-LI inhibitor is YW243.55.S70. The YW243.55.S70 antibody is an anti PD-L1 antibody described in WO 2010/077634.

In other embodiments, the PD-LI inhibitor is MDPL3280A (Genentech/Roche). MDPL3280A is a human Fc optimized IgG1 monoclonal antibody that binds to PD-L1. Other human monoclonal antibodies of MDPL3280A and PD-L1 have been disclosed in US patent number 7,943,743 and US patent publication number 20120039906.

In some embodiments, where the anti PD-L1 antibody includes at least one CDR in VH, the VH includes an amino acid sequence shown in SEQ ID NO: 25.

In some embodiments, where the anti PD-L1 antibody includes VH including HCDR3, the HCDR3 includes an amino acid sequence shown in SEQ ID NO: 21 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 21.

In some embodiments, where the VH further includes HCDR2, wherein the HCDR2 includes an amino acid sequence shown in SEQ ID NO: 20 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 20.

In some embodiments, where the VH further includes HCDR1, wherein the HCDR1 includes an amino acid sequence shown in SEQ ID NO: 19 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 19.

In some embodiments, where the VH includes HCDR1, HCDR2 and HCDR3, wherein the HCDR3 includes an amino acid sequence shown in SEQ ID NO: 21 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 21, the HCDR2 includes an amino acid sequence shown in SEQ ID NO: 20 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 20, and the HCDR1 includes an amino acid sequence shown in SEQ ID NO: 19 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 19.

For example, where the VH can include HCDR1, HCDR2, and HCDR3, wherein the HCDR3 includes an amino acid sequence shown in SEQ ID NO: 21, the HCDR2 includes an amino acid sequence shown in SEQ ID NO: 20, and the HCDR1 includes an amino acid sequence shown in SEQ ID NO: 19.

In some embodiments, where the VH includes a framework region HFR1, the C-terminal of HFR1 is directly or indirectly connected to the N-terminal of HCDR1, and the HFR1 includes an amino acid sequence shown in SEQ ID NO: 22 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 22.

In some embodiments, where the VH includes a framework region HFR2, the N-terminal of HFR2 is directly or indirectly connected to the C-terminal of HCDR1, and the C-terminal of HFR2 is directly or indirectly connected to the N-terminal of HCDR2; and the HFR2 includes an amino acid sequence shown in SEQ ID NO: 23 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 23.

In some embodiments, where the VH includes a framework region HFR3, the N-terminal of HFR3 is directly or indirectly connected to the C-terminal of HCDR2, and the C-terminal of HFR3 is directly or indirectly connected to the N-terminal of HCDR3; and the HFR3 includes an amino acid sequence shown in SEQ ID NO: 24 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 24.

In some embodiments, where the VH includes a framework region HFR4, the N-terminal of HFR4 is directly or indirectly connected to the C-terminal of HCDR3, and the HFR4 includes an amino acid sequence shown in SEQ ID NO: 7 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 7.

In some embodiments, where the VH includes framework regions HFR1, HFR2, HFR3, and HFR4, the C-terminal of HFR1 is directly or indirectly connected to the N-terminal of HCDR1, the N-terminal of HFR2 is directly or indirectly connected to the C-terminal of HCDR1, and the C-terminal of HFR2 is directly or indirectly connected to the N-terminal of HCDR2, the N-terminal of HFR3 is directly or indirectly connected to the C-terminal of HCDR2, and the C-terminal of HFR3 is directly or indirectly connected to the N-terminal of HCDR3, the N-terminal of HFR4 is directly or indirectly connected to the C-terminal of HCDR3; among them, the HFR1 includes an amino acid sequence shown in SEQ ID NO: 22 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 22, the HFR2 includes an amino acid sequence shown in SEQ ID NO: 23 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 23, the HFR3 includes an amino acid sequence shown in SEQ ID NO: 24 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 24, and the HFR4 includes an amino acid sequence shown in SEQ ID NO: 7 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 7.

In some embodiments, where the anti PD-L1 antibody includes VH, and the VH includes an amino acid sequence shown in SEQ ID NO: 25 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 25.

In some embodiments, where the anti PD-L1 antibody includes HC, and the HC includes an amino acid sequence shown in SEQ ID NO: 26 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 26.

In some embodiments, where the anti PD-L1 antibody includes at least one CDR in VL, and the VL includes an amino acid sequence shown in SEQ ID NO: 37.

In some embodiments, where the anti PD-L1 antibody includes at least one CDR in VH, the VH includes an amino acid sequence shown in SEQ ID NO: 25, and the anti PD-L1 antibody includes at least one CDR in VL, and the VL includes an amino acid sequence shown in SEQ ID NO: 37.

In some embodiments, where the anti PD-L1 antibody includes at least one CDR in VH, the VH includes an amino acid sequence shown in SEQ ID NO: 25, and the anti PD-L1 antibody includes at least one CDR in VL, and the VL includes an amino acid sequence shown in SEQ ID NO:38, SEQ ID NO:39 or SEQ ID NO:40.

In some embodiments, where the anti PD-L1 antibody includes VL including LCDR1, the LCDR1 includes an amino acid sequence shown in SEQ ID NO: 27 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 27.

In some embodiments, where the anti PD-L1 antibody includes VL including LCDR1, the LCDR1 includes an amino acid sequence shown in SEQ ID NO:28, SEQ ID NO:29 or SEQ ID NO:30 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO:28, SEQ ID NO:29 or SEQ ID NO:30.

In some embodiments, where the VL further includes LCDR2, wherein the LCDR2 includes an amino acid sequence shown in SEQ ID NO: 31 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 31.

In some embodiments, where the VL further includes LCDR3, wherein the LCDR3 includes an amino acid sequence shown in SEQ ID NO: 32 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 32.

In some embodiments, where the VL includes LCDR1, LCDR2 and LCDR3, wherein the LCDR1 includes an amino acid sequence shown in SEQ ID NO: 27 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO:27, the LCDR2 includes an amino acid sequence shown in SEQ ID NO: 31 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 31, and the LCDR3 includes an amino acid sequence shown in SEQ ID NO: 32 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 32.

In some embodiments, where the VL includes LCDR1, LCDR2 and LCDR3, wherein the LCDR1 includes an amino acid sequence shown in SEQ ID NO: 28 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO:28, the LCDR2 includes an amino acid sequence shown in SEQ ID NO: 31 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 31, and the LCDR3 includes an amino acid sequence shown in SEQ ID NO: 32 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 32;

In some embodiments, where the VL includes LCDR1, LCDR2 and LCDR3, wherein the LCDR1 includes an amino acid sequence shown in SEQ ID NO: 29 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO:29, the LCDR2 includes an amino acid sequence shown in SEQ ID NO: 31 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 31, and the LCDR3 includes an amino acid sequence shown in SEQ ID NO: 32 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 32; or

In some embodiments, where the VL includes LCDR1, LCDR2 and LCDR3, wherein the LCDR1 includes an amino acid sequence shown in SEQ ID NO: 30 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 30, the LCDR2 includes an amino acid sequence shown in SEQ ID NO: 31 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 31, and the LCDR3 includes an amino acid sequence shown in SEQ ID NO: 32 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 32.

In some embodiments, where the anti PD-L1 antibody includes VH and an antibody VL, the VH includes HCDR1, HCDR2, and HCDR3, wherein the HCDR3 includes an amino acid sequence shown in SEQ ID NO: 21 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 21, the HCDR2 includes an amino acid sequence shown in SEQ ID NO: 20 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 20, and the HCDR1 includes an amino acid sequence shown in SEQ ID NO: 19 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 19; and the VL includes LCDR1, LCDR2, and LCDR3, wherein the LCDR1 includes an amino acid sequence shown in SEQ ID NO: 27 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 27, the LCDR2 includes an amino acid sequence shown in SEQ ID NO: 31 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 31, and the LCDR3 includes an amino acid sequence shown in SEQ ID NO: 32 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 32.

For example, where the anti PD-L1 antibody can include VH and an antibody VL, the VH can include HCDR1, HCDR2, and HCDR3, wherein the HCDR3 can include an amino acid sequence shown in SEQ ID NO: 21, the HCDR2 can include an amino acid sequence shown in SEQ ID NO: 20, and the HCDR1 can include an amino acid sequence shown in SEQ ID NO: 19; and the VL can include LCDR1, LCDR2, and LCDR3, wherein the LCDR1 can include an amino acid sequence shown in SEQ ID NO: 27, the LCDR2 can include an amino acid sequence shown in SEQ ID NO: 31, and the LCDR3 can include an amino acid sequence shown in SEQ ID NO: 32.

For example, the pharmaceutical combination can include: 1) anti PD-L1 antibody, wherein the anti PD-L1 antibody can include VH and antibody VL, the VH can include HCDR1, HCDR2, and HCDR3, wherein the HCDR3 can include an amino acid sequence shown in SEQ ID NO: 21, the HCDR2 can include an amino acid sequence shown in SEQ ID NO: 20, and the HCDR1 can include an amino acid sequence shown in SEQ ID NO: 19; and the VL can include LCDR1, LCDR2, and LCDR3, wherein the LCDR1 can include an amino acid sequence shown in SEQ ID NO: 27, the LCDR2 can include an amino acid sequence shown in SEQ ID NO: 31, and the LCDR3 can include an amino acid sequence shown in SEQ ID NO: 32; 2) a STING pathway agonist, which can be 2',3'-cGAMP, or derivatives thereof.

In some embodiments, where the anti PD-L1 antibody includes VH and an antibody VL, the VH includes HCDR1, HCDR2, and HCDR3, wherein the HCDR3 includes an amino acid sequence shown in SEQ ID NO: 21 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 21, the HCDR2 includes an amino acid sequence shown in SEQ ID NO: 20 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 20, and the HCDR1 includes an amino acid sequence shown in SEQ ID NO: 19 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 19; and the VL includes LCDR1, LCDR2, and LCDR3, wherein the LCDR1 includes an amino acid sequence shown in SEQ ID NO:28, SEQ ID NO:29 or SEQ ID NO:30 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO:28, SEQ ID NO:29 or SEQ ID NO:30, the LCDR2 includes an amino acid sequence shown in SEQ ID NO: 31 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 31, and the LCDR3 includes an amino acid sequence shown in SEQ ID NO: 32 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 32.

For example, where the anti PD-L1 antibody can include VH and an antibody VL, the VH can include HCDR1, HCDR2, and HCDR3, wherein the HCDR3 can include an amino acid sequence shown in SEQ ID NO: 21, the HCDR2 can include an amino acid sequence shown in SEQ ID NO: 20, and the HCDR1 can include an amino acid sequence shown in SEQ ID NO: 19; and the VL include LCDR1, LCDR2, and LCDR3, wherein the LCDR1 can include an amino acid sequence shown in SEQ ID NO:28, SEQ ID NO:29 or SEQ ID NO:30, the LCDR2 can include an amino acid sequence shown in SEQ ID NO: 31, and the LCDR3 can include an amino acid sequence shown in SEQ ID NO: 32.

For example, the pharmaceutical combination can include: 1) anti PD-L1 antibody, wherein the anti PD-L1 antibody can include VH and antibody VL, the VH can include HCDR1, HCDR2, and HCDR3, wherein the HCDR3 can include an amino acid sequence shown in SEQ ID NO: 21, the HCDR2 can include an amino acid sequence shown in SEQ ID NO: 20, and the HCDR1 can include an amino acid sequence shown in SEQ ID NO: 19; and the VL can include LCDR1, LCDR2, and LCDR3, wherein the LCDR1 can include an amino acid sequence shown in SEQ ID NO:28, SEQ ID NO:29 or SEQ ID NO:30, the LCDR2 can include an amino acid sequence shown in SEQ ID NO: 31, and the LCDR3 can include an amino acid sequence shown in SEQ ID NO: 32; 2) a STING pathway agonist, which can be 2',3'-cGAMP, or derivatives thereof.

In some embodiments, where the VL includes a framework region LFR1, the C-terminal of LFR1 is directly or indirectly connected to the N-terminal of LCDR1, and the LFR1 includes an amino acid sequence shown in SEQ ID NO:

33 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 33.

In some embodiments, where the VL includes a framework region LFR2, wherein the N-terminal of LFR2 is directly or indirectly connected to the C-terminal of LCDR1, and the C-terminal of LFR2 is directly or indirectly connected to the N-terminal of LCDR2; and the LFR2 includes an amino acid sequence shown in SEQ ID NO: 34 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 34.

In some embodiments, where the VL includes a framework region LFR3, wherein the N-terminal of LFR3 is directly or indirectly connected to the C-terminal of LCDR2, and the C-terminal of LFR3 is directly or indirectly connected to the N-terminal of LCDR3; and the LFR3 includes an amino acid sequence shown in SEQ ID NO: 35 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 35.

In some embodiments, where the VL includes a framework region LFR4, wherein the N-terminal of LFR4 is directly or indirectly connected to the C-terminal of LCDR3, and the LFR4 includes an amino acid sequence shown in SEQ ID NO: 36 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 36.

In some embodiments, where the VL includes framework regions LFR1, LFR2, LFR3 and LFR4, the C-terminal of LFR1 is directly or indirectly connected to the N-terminal of LCDR1, the N-terminal of LFR2 is directly or indirectly connected to the C-terminal of LCDR1, and the C-terminal of LFR2 is directly or indirectly connected to the N-terminal of LCDR2, the N-terminal of LFR3 is directly or indirectly connected to the C-terminal of LCDR2, and the C-terminal of LFR3 is directly or indirectly connected to the N-terminal of LCDR3, the N-terminal of LFR4 is directly or indirectly connected to the C-terminal of LCDR3; among them, the LFR1 includes an amino acid sequence shown in SEQ ID NO: 33 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 33, the LFR2 includes an amino acid sequence shown in SEQ ID NO: 34 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 34, the LFR3 includes an amino acid sequence shown in SEQ ID NO: 35 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 35, and the LFR4 includes an amino acid sequence shown in SEQ ID NO: 36 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 36.

In some embodiments, where the anti PD-L1 antibody includes VL, and the VL includes an amino acid sequence shown in SEQ ID NO: 37 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 37.

In some embodiments, where the anti PD-L1 antibody includes VL, and the VL includes an amino acid sequence shown in SEQ ID NO:38, SEQ ID NO:39 or SEQ ID NO:40 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO:38, SEQ ID NO:39 or SEQ ID NO:40.

In some embodiments, where the anti PD-L1 antibody includes VH and VL, the VH includes an amino acid sequence shown in SEQ ID NO: 25 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 25, and the VL includes an amino acid sequence shown in SEQ ID NO: 37 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 37.

For example, where the anti PD-L1 antibody can include VH and VL, the VH can include an amino acid sequence shown in SEQ ID NO: 25, and the VL can include an amino acid sequence shown in SEQ ID NO: 37.

For example, the pharmaceutical combination can include: 1) anti PD-L1 antibody, wherein the anti PD-L1 antibody can include VH and VL, the VH can include an amino acid sequence shown in SEQ ID NO: 25, and the VL can include an amino acid sequence shown in SEQ ID NO: 37; 2) a STING pathway agonist, which can be 2',3'-cGAMP or derivatives thereof.

In some embodiments, where the anti PD-L1 antibody includes VH and VL, the VH includes an amino acid sequence shown in SEQ ID NO: 25 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 25, and the VL includes an amino acid sequence shown in SEQ ID NO:38, SEQ ID NO:39 or SEQ ID NO:40 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO:38, SEQ ID NO:39 or SEQ ID NO:40.

For example, where the anti PD-L1 antibody can include VH and VL, the VH can include an amino acid sequence shown in SEQ ID NO: 25, and the VL can include an amino acid sequence shown in SEQ ID NO:38, SEQ ID NO:39 or SEQ ID NO:40.

For example, the pharmaceutical combination can include: 1) anti PD-L1 antibody, wherein the anti PD-L1 antibody can include VH and VL, the VH can include an amino acid sequence shown in SEQ ID NO: 25, and the VL can include an amino acid sequence shown in SEQ ID NO:38, SEQ ID NO:39 or SEQ ID NO:40; 2) a STING pathway agonist, which can be 2',3'-cGAMP or derivatives thereof.

In some embodiments, where the anti PD-L1 antibody includes LC, and the LC includes an amino acid sequence shown in SEQ ID NO: 41 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 41.

In some embodiments, where the anti PD-L1 antibody includes LC, and the LC includes an amino acid sequence shown in SEQ ID NO:42, SEQ ID NO:43 or SEQ ID NO:44 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO:42, SEQ ID NO:43 or SEQ ID NO:44.

In some embodiments, where the anti PD-L1 antibody includes HC and LC, the HC includes an amino acid sequence shown in SEQ ID NO: 26 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 26, and the LC includes an amino acid sequence shown in SEQ ID NO: 41 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 41.

For example, where the anti PD-L1 antibody can include HC and LC, the HC can include an amino acid sequence shown in SEQ ID NO: 26, and the LC can include an amino acid sequence shown in SEQ ID NO: 41.

For example, the pharmaceutical combination can include: 1) anti PD-L1 antibody, wherein the anti PD-L1 antibody can include HC and LC, the HC can include an amino acid sequence shown in SEQ ID NO: 26, and the LC can include an amino acid sequence shown in SEQ ID NO: 41; 2) a STING pathway agonist, which can be 2',3'-cGAMP or derivatives thereof.

In some embodiments, where the anti PD-L1 antibody includes HC and LC, the HC includes an amino acid sequence shown in SEQ ID NO: 26 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO: 26, and the LC includes an amino acid sequence shown in SEQ ID NO:42, SEQ ID NO:43 or SEQ ID NO:44 or an amino acid sequence having at least about 70%, about 75%, about 80%, about 85%, about 90%, about 91%, about 92%, about 93%, about 94%, about 95%, about 96%, about 97%, about 98%, or about 99% sequence identity to the amino acid sequence shown in SEQ ID NO:42, SEQ ID NO:43 or SEQ ID NO:44.

For example, where the anti PD-L1 antibody can include HC and LC, the HC can include an amino acid sequence shown in SEQ ID NO: 26, and the LC can include an amino acid sequence shown in SEQ ID NO:42, SEQ ID NO:43 or SEQ ID NO:44.

For example, the pharmaceutical combination can include: 1) anti PD-L1 antibody, wherein the anti PD-L1 antibody can include HC and LC, the HC can include an amino acid sequence shown in SEQ ID NO: 26, and the LC can include an amino acid sequence shown in SEQ ID NO:42, SEQ ID NO:43 or SEQ ID NO:44; 2) a STING pathway agonist, which can be 2',3'-cGAMP or derivatives thereof.

In some embodiments, i) the PD-1 inhibitor and/or PD-L1 inhibitor in the pharmaceutical combination are not mixed with ii) the STING pathway agonist to each other in the pharmaceutical combination, i.e., i) the PD-1 inhibitor and/or ii) the PD-L1 inhibitor and STING pathway agonist are located in separate dosage forms.

In some embodiments, i) the PD-1 inhibitor and/or PD-L1 inhibitor, and ii) the STING pathway agonist are present in the pharmaceutical combination in a single dosage form.

In some embodiments, where the pharmaceutical combination is formulated into a pharmaceutical composition (e.g., a composite formulation or compound formulation). This pharmaceutical combination can be directly injected into large tumors without affecting normal (surrounding) tissues, enabling the killing of cancer cells, preventing or delaying the growth of malignant mass (e.g., making the tumor smaller or regression), and enabling advanced cancer patients to live with the tumor in a similar manner to those living with parasites. When a pharmaceutical combination is injected into a tumor, the drug can flow along blood vessels or lymphatic vessels to the metastatic lesion, and it will kill the metastatic cells. The injection of pharmaceutical combination into the tumor results in minimal trauma to the patient and can be repeated multiple times a month, for example. Direct injection can also be applied simultaneously to primary tumors and secondary tumors to which cancer has metastasized.

In some embodiments, where the pharmaceutical composition includes a PD-1 inhibitor or a PD-L1 inhibitor, and a STING pathway agonist.

For example, the pharmaceutical composition can include a PD-1 inhibitor and a STING pathway agonist. For example, the pharmaceutical composition can include an anti PD-1 antibody or antigen-binding fragments thereof and a cyclic dinucleotide.

For example, the pharmaceutical composition can include a PD-L1 inhibitor and a STING pathway agonist. Yet for example, the pharmaceutical composition can include an anti PD-L1 antibody or antigen-binding fragments thereof and a cyclic dinucleotide.

In some embodiments, where the STING pathway agonist is present in an amount of about 0.0001 mg/kg to about 200 mg/kg. For example, the STING pathway agonist can be present in the combination described herein in an amount relative to the subject's body weight (i.e., mg/kg). In some cases, the STING pathway agonist is present in an amount equivalent to about 0.0001 mg/kg to about 200 mg/kg, 0.001 mg/kg to about 200 mg/kg, 0.01 mg/kg to about 200 mg/kg, 0.01 mg/kg to about 150 mg/kg, 0 01 mg/kg to about 100 mg/kg, 0.01 mg/kg to about 50 mg/kg, 0.01 mg/kg to about 25 mg/kg, 0.01 mg/kg to about 10 mg/kg or 0.01 mg/kg to about 5 mg/kg, 0.05 mg/kg to about 200 mg/kg, 0.05 mg/kg to about 150 mg/kg, 0.05 mg/kg to about 100 mg/kg, 0.05 mg/kg to about 50 mg/kg, 0.05 mg/kg to about 25 mg/kg, 0.05 mg/kg to about 10mg/kg or 0.05mg/kg to about 5 mg/kg, 0.5 mg/kg to about 200 mg/kg, 0.5 mg/kg to about 150 mg/kg, 0.5 mg/kg to about 100 mg/kg, 0.5 mg/kg to about 50 mg/kg, 0.5 mg/kg to about 25 mg/kg, 0.5 mg/kg to about 10 mg/kg or 0.5 mg/kg to about 5 mg/kg. In other cases, the STING pathway agonist is present in an amount equivalent to about 1 mg/kg to about 200 mg/kg, 1 mg/kg to about 150 mg/kg, 1 mg/kg to about 100 mg/kg, 1 mg/kg to about 50 mg/kg, 1 mg/kg to about 25 mg/kg, 1 mg/kg to about 10 mg/kg, or 1 mg/kg to about 5 mg/kg.

Yet for example, the STING pathway agonist can be present in the combination in an amount of about 1 mg, 5 mg, 10 mg, 15 mg, 20 mg, 25 mg, 30 mg, 40 mg, 50 mg, 60 mg, 70 mg, 75 mg, 80 mg, 90 mg, 100 mg, 150 mg, 200 mg, 250 mg, 300 mg, 400 mg, 500 mg, 600 mg, 700 mg, 800 mg, 900 mg, 1000 mg, 1100 mg, 1200 mg, 1300 mg, 1400 mg, 1500 mg, 1600 mg, 1700 mg, 1800 mg, 1900 mg or 2000 mg. The STING pathway agonist can be present in the combination in an amount of about 1 mg to about 10 mg, 10 mg to about 20 mg, 25 mg to about 50 mg, 30 mg to about 60 mg, 40 mg to about 50 mg, 50 mg to about 100 mg, 75 mg to about 150 mg, 100 mg to about 200 mg, 200 mg to about 500 mg, 500 mg to about 1000 mg, 1000 mg to about 1200 mg, 1000 mg to about 1500 mg, 1200 mg to about 1500 mg, or 1500 mg to about 2000 mg.

Yet for example, the STING pathway agonist can be present in the combination in an amount of about 0.1 mg/mL, 0.5 mg/mL, 1 mg/mL, 2 mg/mL, 3 mg/mL, 4 mg/mL, 5 mg/mL, 6 mg/mL, 7 mg/mL, 8 mg/mL, 9 mg/mL, 10 mg/mL, 15 mg/mL, 20 mg/mL, 25 mg/mL, 30 mg/mL, 40 mg/mL, 50 mg/mL, 60 mg/mL, 70 mg/mL, 80 mg/mL, 90 mg/mL, 100 mg/mL, 150 mg/mL, 200 mg/mL, 250 mg/mL, 300 mg/mL, 400 mg/mL, or 500 mg/mL. In some embodiments, the STING pathway agonist can be present in the combination in an amount of about 1 mg/mL to about 10 mg/mL, 5 mg/mL to about 10mg/mL, 5 mg/mL to about 15 mg/mL, 10 mg/mL to about 25 mg/mL, 20 mg/mLto about 30 mg/mL, 25 mg/mL to about 50 mg/mL or 50 mg/mL to about 100 mg/mL.

In some embodiments, where the PD-1 inhibitor or PD-L1 inhibitor (e.g., the anti PD-1/PD-L1 antibody) is present in an amount of about 0.0001 mg/kg to about 200 mg/kg. For example, the PD-1 inhibitor or PD-L1 inhibitor can be present in the combination described herein in an amount relative to the subject's body weight (i.e., mg/kg). In some cases, the PD-1 inhibitor or PD-L1 inhibitor is present in an amount equivalent to about 0.0001 mg/kg to about 200 mg/kg, 0.001 mg/kg to about 200 mg/kg, 0.01 mg/kg to about 200 mg/kg, 0.01 mg/kg to about 150 mg/kg, 0 01 mg/kg to about 100 mg/kg, 0.01 mg/kg to about 50 mg/kg, 0.01 mg/kg to about 25 mg/kg, 0.01 mg/kg to about 10 mg/kg or 0.01 mg/kg to about 5 mg/kg, 0.05 mg/kg to about 200 mg/kg, 0.05 mg/kg to about 150 mg/kg, 0.05 mg/kg to about 100 mg/kg, 0.05 mg/kg to about 50 mg/kg, 0.05 mg/kg to about 25 mg/kg, 0.05 mg/kg to about 10 mg/kg or 0.05mg/kg to about 5 mg/kg, 0.5 mg/kg to about 200 mg/kg, 0.5 mg/kg to about 150 mg/kg, 0.5 mg/kg to about 100 mg/kg, 0.5 mg/kg to about 50 mg/kg, 0.5 mg/kg to about 25 mg/kg, 0.5 mg/kg to about 10 mg/kg or 0.5 mg/kg to about 5 mg/kg. In other cases, the PD-1 inhibitor or PD-L1 inhibitor is present in an amount equivalent to about 1 mg/kg to about 200 mg/kg, 1 mg/kg to about 150 mg/kg, 1 mg/kg to about 100 mg/kg, 1 mg/kg to about 50 mg/kg, 1 mg/kg to about 25 mg/kg, 1 mg/kg to about 10 mg/kg, or 1 mg/kg to about 5 mg/kg.

Yet for example, the anti PD-1/PD-L1 antibody can be present in the combination in an amount of about 1 mg, 5 mg, 10 mg, 15 mg, 20 mg, 25 mg, 30 mg, 40 mg, 50 mg, 60 mg, 70 mg, 75 mg, 80 mg, 90 mg, 100 mg, 150 mg, 200 mg, 250 mg, 300 mg, 400 mg, 500 mg, 600 mg, 700 mg, 800 mg, 900 mg, 1000 mg, 1100 mg, 1200 mg, 1300 mg, 1400 mg, 1500 mg, 1600 mg, 1700 mg, 1800 mg, 1900 mg, or 2000 mg. The anti PD-1/PD-L1 antibody can be present in the combination in an amount of about 1 mg to about 10 mg, 10 mg to about 20 mg, 25 mg to about 50 mg, 30 mg to about 60 mg, 40 mg to about 50 mg, 50 mg to about 100 mg, 75 mg to about 150 mg, 100 mg to about 200 mg, 200 mg to about 500 mg, 500 mg to about 1000 mg, 1000 mg to about 1200 mg, 1000 mg to about 1500 mg, 1200 mg to about 1500 mg, or 1500 mg to about 2000 mg.

Yet for example, the anti PD-1/PD-L1 antibody can be present in the combination in an amount of about 0.1 mg/mL, 0.5 mg/mL, 1 mg/mL, 2 mg/mL, 3 mg/mL, 4 mg/mL, 5 mg/mL, 6 mg/mL, 7 mg/mL, 8 mg/mL, 9 mg/mL, 10 mg/mL, 15 mg/mL, 20 mg/mL, 25 mg/mL, 30 mg/mL, 40 mg/mL, 50 mg/mL, 60 mg/mL, 70 mg/mL, 80 mg/mL, 90 mg/mL, 100 mg/mL, 150 mg/mL, 200 mg/mL, 250 mg/mL, 300 mg/mL, 400 mg/mL, or 500 mg/mL. In some embodiments, the anti PD-1/PD-L1 antibody can be present in the combination in an amount of about 1 mg/mL to about 10 mg/mL, 5 mg/mL to about 10mg/mL, 5 mg/mL to about 15 mg/mL, 10 mg/mL to about 25 mg/mL, 20 mg/mLto about 30 mg/mL, 25 mg/mL to about 50 mg/mL or 50 mg/mL to about 100 mg/mL.

In some embodiments, the STING pathway agonist can be provided in a synergistic amount with the amount of the PD-1/PD-L1 inhibitor. The administration dosage will undoubtedly vary with known factors, such as the pharmacokinetic properties of a particular agent and mode and route of administration thereof; age, physical condition, and weight of the recipient; nature and extent of symptoms, type of concurrent treatment, frequency of treatment, and expected effect.

In some embodiments, where the pharmaceutical composition further includes one or more pharmaceutically acceptable carriers.

In another aspect, the disclosure also provides a use of the aforementioned pharmaceutical combination in the preparation of drugs for the treatment of neoplastic diseases.

In some embodiments, where the neoplastic diseases include tumors and/or wart diseases.

In some embodiments, the tumors (or cancers) include but are not limited to: hepatocellular carcinoma, hepatic metastasis cancer, advanced hepatocellular carcinoma, pancreatic carcinoma, adenocarcinoma, mastocytoma or mast cell tumor, ovarian carcinoma, non-small-cell lung carcinoma, small-cell lung carcinoma, melanoma, retinoblastoma, breast neoplasms, colorectal carcinoma, histiocytosarcoma, brain neoplasms, astrocytoma, glioblastoma, neuroma, neuroblastoma, colorectal carcinoma, cervical carcinoma, sarcoma, prostatic neoplams, bladder neoplasms, reticuloendothelium neoplasms, nephroblastoma, ovarian carcinoma, osteocarcinoma, osteosarcoma, renal carcinoma, or head and neck cancer, oral carcinoma, laryngocarcinoma, or oropharyngeal carcinoma, breast carcinoma, genitourinary tract carcinoma, lung carcinoma, gastrointestinal carcinoma, epidermoid carcinoma, or melanoma.

In another aspect, the disclosure also provides the aforementioned pharmaceutical combination for use in the treatment of neoplastic diseases.

In another aspect, the disclosure also provides a drug including the aforementioned pharmaceutical combination for treating neoplastic diseases.

In another aspect, the disclosure also provides a method for treating neoplastic diseases, which includes administering an effective amount of the aforementioned pharmaceutical combination to a subject in need thereof.

In some embodiments, where the subject suffers from neoplasm.

In some embodiments, where the neoplasm of mammals is located at a site selected from the group consisting of: brain, head, eyes, nasopharynx, mouth, tongue, neck, thyroid, gastrointestinal system, liver, pancreas, gall bladder, lungs, respiratory system, genitourinary system, kidney, bladder, breast, lymphatic system, cardiovascular system, nervous system, skin, thoracic cavity, pleura, musculoskeletal system, and abdomen, which has primary or secondary natures.

In some embodiments, where the neoplasm includes tumors and/or warts.

In some embodiments, where the administration includes local, intraneoplasitc (e.g., in tumors or warts), or systemic administrations. For example, using intratumoral or intraverrucous injection allows for both less trauma to the patient and the killing of cancer cells instead of normal cells. The direct injection of pharmaceutical combinations into malignant tumors also greatly reduces or eliminates many common side effects.

In some embodiments, where the administration includes intravenous injection, intravenous instillation, intramuscular injection, subcutaneous injection, and/or intraneoplasitc injection.

In some embodiments, the tumor includes:

(i) superficial malignant diseases of skin, eyes, tongue, mouth, thyroid, breast, cervix, uterus, anus, prostate, vagina, osteosarcoma, urethral carcinoma, penis, testicles, and epididymis, and the pharmaceutical combination is directly injected into the tumor body without dilution using a syringe; or

(ii) cancer of nasopharynx, and the pharmaceutical combination is injected into the tumor body through a nasopharyngoscopy using a syringe or a needle; or

(iii) cancers of liver, kidney, and gallbladder, and the pharmaceutical combination is injected into the tumor body through the skin with the assistance of ultrasound using a syringe, or into the tumor body through a pore formed in the patients' abdominal wall during laparoscopic surgery; or

(iv) cancers of ovarian, fallopian tube, pancreas, lymph node metastasis or direct peritoneal invasion of the abdominal cavity, and abdominal lymphoma, and the pharmaceutical combination is injected into the tumor body through a pore formed in the patients' abdominal wall during laparoscopic surgery using a syringe; or

(v) cancers or sarcoma of esophagus, stomach, duodenum, and small intestine, and the pharmaceutical combination is injected into the tumor body through colonoscopy using a needle, or through a pore formed in the patients' abdominal wall during laparoscopic surgery through a long syringe, or through a pore formed in the patients' chest wall during thoracoscopic surgery;

(vi) cancers or sarcoma of large intestine and rectum, and the pharmaceutical combination is injected into the tumor body through colonoscopy using a needle, or through a pore formed in the patients' abdominal wall during laparoscopic surgery using a syringe; or

(vii) cancers or sarcoma of lung and trachea, and the pharmaceutical combination is injected into the tumor body through a fiberbronchoscopy using a needle; or

(viii) cancer of lung, and the pharmaceutical combination is injected with a syringe using ultrasound, X-ray, CT scan, or MR scan, or through a pore formed in the patients' chest wall during thoracoscopic surgery; or

(ix) cancer or sarcoma of bladder, and the pharmaceutical combination is injected into the tumor body through cystoscopy using a needle, or through a pore formed in the patients' abdominal wall during laparoscopic surgery; or

(x) cancer or sarcoma of uterus, and the injectable formulation of the pharmaceutical combination is injected into the tumor body using a syringe or hysteroscopic needle; or injected through a pore formed in the patients' abdominal wall during laparoscopic surgery; or

(xi) cancer or sarcoma of nasopharynx and larynx, and the pharmaceutical combination is injected into the tumor body through laryngoscopy using a needle; or

(xii) cancer of brain, and the pharmaceutical combination is injected into the tumor body with a syringe or fibroscopic needle by use of X-ray, CT scan, or MR scan after drilling a pore in the corresponding skull; or

(xiii) malignant lymphoma or lymph nodes with metastasis, and the pharmaceutical combination is injected into the tumor body through the patients' skin using a needle, or through a pore formed in the patients' abdominal wall during laparoscopic surgery, or through a pore formed in the patients' chest wall during thoracoscopic surgery.

In some embodiments, the neoplasm includes warts, and the pharmaceutical combination is injected into the wart body through the patients' skin using a needle.
In some embodiments, where i) the PD-1 inhibitor or PD-L1 inhibitor, and ii) the STING pathway agonist in the pharmaceutical combination are administered by use of the same or different administration routes.
In some embodiments, it includes injection of the STING pathway agonist into the neoplasm.
In some embodiments, it also includes injection or systemic infusion (e.g., intravenous injection and intravenous instillation) of the PD-1 inhibitor or PD-L1 inhibitor into the neoplasm.
In some embodiments, it includes injection of i) the PD-1 inhibitor or PD-L1 inhibitor, and ii) the STING pathway agonist in the pharmaceutical combination into the neoplasm.
In some embodiments, where i) the PD-1 inhibitor or PD-L1 inhibitor, and ii) the STING pathway agonist in the pharmaceutical combination are administered simultaneously or at different times.
In some embodiments, where the PD-1 inhibitor or PD-L1 inhibitor is administered before and/or after the administration of the STING pathway agonist.
For example, the PD-1/PD-L1 inhibitor is administered at least 5 minutes, 10 minutes, 20 minutes, 40 minutes, 1 hour, 2 hours, 4 hours, 8 hours, 12 hours, 16 hours, 1 day, 2 days, 3 days, 4 days, 5 days, 6 days, 7 days or 2 weeks before and/or after the administration of the STING pathway agonist.

In some embodiments, i) the PD-1 inhibitor or PD-L1 inhibitor, and ii) the STING pathway agonist in the pharmaceutical combination are simultaneously administered by way of intraneoplasitc injection.

In some embodiments, i) the PD-1 inhibitor or PD-L1 inhibitor, and ii) the STING pathway agonist in the pharmaceutical combination are simultaneously administered by way of intraneoplasitc injection, and i) the PD-1 inhibitor or PD-L1 inhibitor and ii) the STING pathway agonist are present in a same dosage form.

In some embodiments, where the PD-1 inhibitor or PD-L1 inhibitor in the pharmaceutical combination is administered by way of intravenous injection, the STING pathway agonist is simultaneously administered by way of intraneoplasitc injection, and the PD-1 inhibitor or PD-L1 inhibitor and the STING pathway agonist are present in separate dosage forms.

In another aspect, the disclosure provides a pharmaceutical kit including the aforementioned pharmaceutical combination.

In some embodiments, each component of the pharmaceutical combination in the pharmaceutical kit can be provided in separated individual containers. Alternatively, the components of the pharmaceutical combination described herein can be provided in a single container. In such cases, the container can be a container prepared for disclosure to patients in need thereof, such as IV bags, ampoules, or syringes. In some embodiments, the STING pathway agonist in the pharmaceutical kit is formulated for administration through intratumoral or intraverrucous injection. The PD-1/PD-L1 inhibitor can be provided in the form of powder (e.g., lyophilized powder) or parenteral solution, for example. In some cases, the PD-1/PD-L1 inhibitor can be an anti PD-1/PD-L1 antibody as described herein, formulated for use, for example through intravenous administration or intratumoral or intraverrucous injection. In some embodiments, the STING pathway agonist and the PD-1/PD-L1 inhibitor are formulated as a compound formulation and used for administration through intratumoral or intraverrucous injection.

The content of the pharmaceutical kit described herein can be provided in sterile form. The pharmaceutical kit and content therein can be provided in the form of ready-to-use to a subject in need thereof. In such cases, the components in the pharmaceutical kit are provided as a formulation and optionally provided in an administration device, so that the administration requires almost no further action by the user. In the case where the pharmaceutical kit includes an administration device, such devices include devices known and understood by those skilled in the art for the administration routes described herein, such as but not limited to syringes, pumps, bags, cups, inhalators, droppers, patches, creams, or injectors.

The pharmaceutical kit described herein may also include an instruction, which contains information about, for example, usage, dosage, administration, contraindications, and/or warnings about the use of such drugs.

Without being limited by any theory, the examples hereinafter are only intended to illustrate the pharmaceutical combinations and uses of the disclosure, rather than limiting the scope of invention of the disclosure.

## Examples

### Animal species, strain, gender, weight, sources, certificate of conformity

C57/BL6JNifdc mouse, female, body weight of 17-22 g, aged 6-8 weeks, SPF grade, purchased from Beijing Vital River Laboratory Animal Technology Co., Ltd. [Experimental Animal Quality Certificate No.: SCXK (Beijing) 2016-0006].

### Feeding conditions

All mice were free to forage and drink water, and were raised at room temperature $(25 \pm 2)°C$. The feed and water were subjected to high-pressure sterilization treatment, and the entire experimental feeding process was SPF grade.

### Tumor cell strains

Mouse colorectal carcinoma cell strain MC38, mouse lung carcinoma Lewis tumor strain LLC1, mouse melanoma cell strain B16, mouse prostatic carcinoma cell strain RM-1, TRAMP-C1, mouse breast carcinoma cell strains 4T1, and NAFA, etc.

### Establishment of subcutaneous xenograft tumor model

Tumor cell strains were selected from the group consisting of : mouse colorectal carcinoma cell strains CT26 and MC38, mouse lung carcinoma Lewis tumor strain LLC1, mouse melanoma cell strain B16, mouse prostatic carcinoma cell strain RM-1, TRAMP-C1, mouse breast carcinoma cell strains 4T1, and NAFA, etc.

Tumor cells were cultured and passaged, cells were collected at the logarithmic phase, and formulated into a concentration of $(1.0\times10^7)$ per milliliter of cell suspension with 0.1 ml (with a cell number of $1.0\times10^6$) being injected into the right flank of the mice, the tumor grew to a diameter of about 5 mm within about 10 days, and the model was successfully established, they were randomly divided into 8 groups with 8 mice per group.

**Drug evaluation indicators**

Trend of changes in body weight and tumor volume of mice, survival rate of mice, and inhibitory rate of anatomical tumor weight.

Tumor weight inhibitory rate = [1- average tumor weight of experimental group (G2/G3/G4)/average tumor weight of negative control group G1)] × 100%.

Drug interaction coefficient: CDI(CI)=AB/A*B. AB is the ratio of the combination group of two drugs to the control group, as calculated based on the anatomical tumor weight. A or B is the ratio of single drug group to control group. If CDI is less than 1, it indicates that the two drugs have a synergistic effect; If CDI equals to 1, the two drugs have an additive effect; If CDI is greater than 1, the two drugs have an antagonistic effect.

**Statistical analysis**

The data were represented as $x \pm s$, and processed by SPSS10.0 software, using one-way ANOVA. Differences in data among groups (P value) were processed through statistical processing, and there was a significant difference among groups at $P<0.05$.

**Example 1**

The anti-tumor effect of a pharmaceutical combination composed of PD-L1 inhibitor RB0005 and the STING agonist 2',3'-cGAMP was tested using a xenograft tumor model, i.e., the inhibitory effect on the growth of subcutaneous xenograft tumor in mice.

**Main steps of the test**

**1.1 Xenograft tumor model in mice**

After successfully establishing a subcutaneous xenograft tumor model of colorectal carcinoma MC38 in mice, it was randomly grouped with 6-8 mice per group. The mice were administered once every two days, with a total of three times followed by observation until 15 days.

| Groups | Administration Method | Administration dosage | Administration frequency |
|---|---|---|---|
| G1 Negative Control | Intratumor injection | Solvent | Administered every other day with a total of 3 times |
| G2 single drug group | Intratumor injection | PD-L1 monoclonal antibody RB0005, 1 mg/kg | Administered every other day with a total of 3 times |
| G3 single drug group | Intratumoral injection (i.t.) | 2',3'-cGAMP monotherapy, 1 mg/kg | Administered every other day with a total of 3 times |

(continued)

| Groups | Administration Method | Administration dosage | Administration frequency |
|---|---|---|---|
| G4 trial group (Single dosage form) | Intratumor injection | PD-L1 monoclonal antibody RB0005, 1 mg/kg + 2',3'-cGAMP, 1 mg/kg; | Administered every other day with a total of 3 times |

**1.2 Results**

Figures 1-4 show that both the novel pharmaceutical combination and each single drug can significantly inhibit the growth of tumor, and the anatomical tumor weight is significantly lower than the negative control group (P<0.01, P<0.001). The novel pharmaceutical combinations have better efficacy than 2',3'-cGAMP or PD-L1 antibody RB0005 alone, indicating that the novel pharmaceutical combinations have greater advantages.

As shown in Figure 2, the mice in solvent control group began to experience "death" on day 9 (when the tumor volume exceeded 2000 $mm^3$ in a single mouse, the mouse could be considered as death during the statistical analysis of survival rate in mouse (which was not excluded in the analysis of tumor volume change curve). During day 11 to day 13, the survival rate of mice in the solvent control group decreased most significantly; all mice in single drug group began to experience "death" on day 15, there was no significant difference between the two groups; one mouse in the G4 trial group had tumor regression and one mouse had almost complete regression (with a tumor volume of <100 $mm^3$). At the endpoint of the experiment, the survival rates of mice in each group were 0%, 37.5%, 75%, and 87.5%, respectively.

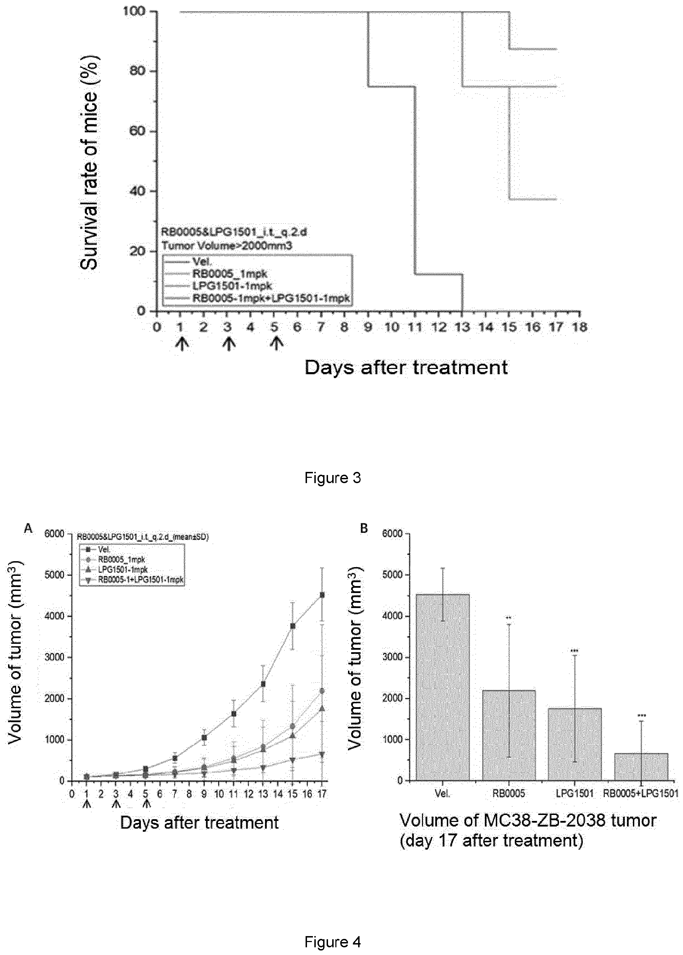

As shown in Figure 3A-3B, the tumor volume of mice in G1 solvent control group gradually increased over time; and the growth of tumor volume of mice in the G4 trial group (RB0005+2',3'-cGAMP) significantly slowed down and the overall volume decreased.

As shown in Figure 4, the tumor weight of mice in the G4 trial group was significantly reduced, with a tumor weight inhibitory rate of 73%, which showed a statistically significant difference (P<0.001).

**Example 2**

The anti-tumor effect of a pharmaceutical combination composed of PD-1 inhibitor RB0004 and the STING agonist 2',3'-cGAMP was tested using a xenograft tumor model, i.e., the inhibitory effect on the growth of subcutaneous xenograft tumor in mice.

**2.1 Xenograft tumor model in mice**

After successfully establishing a humanized PD-1 subcutaneous xenograft tumor model of colorectal carcinoma MC38 in mice, they were randomly grouped with 8 mice per group and administered once every two days.

| Groups | Administration Method | Administration dosage | Administration frequency |
|---|---|---|---|
| G1 Negative Control | Intratumor injection | Solvent | Administering every other day |
| G2 single drug group | Intratumor injection | 2',3'-cGAMP monotherapy, 3 mg/kg | Administering every other day |
| G3 single drug group | Intraperitoneal injection (i.p.) | PD-1 monoclonal antibody RB0004, 10 mg/kg | Administering every other day |
| G4 trial group (separate dosage form) | Intratumor injection | 2',3'-cGAMP monotherapy, 3 mg/kg | Administering every other day |
| | Intraperitoneal injection | PD-1 monoclonal antibody RB0004, 10 mg/kg | |

**2.2 Results**

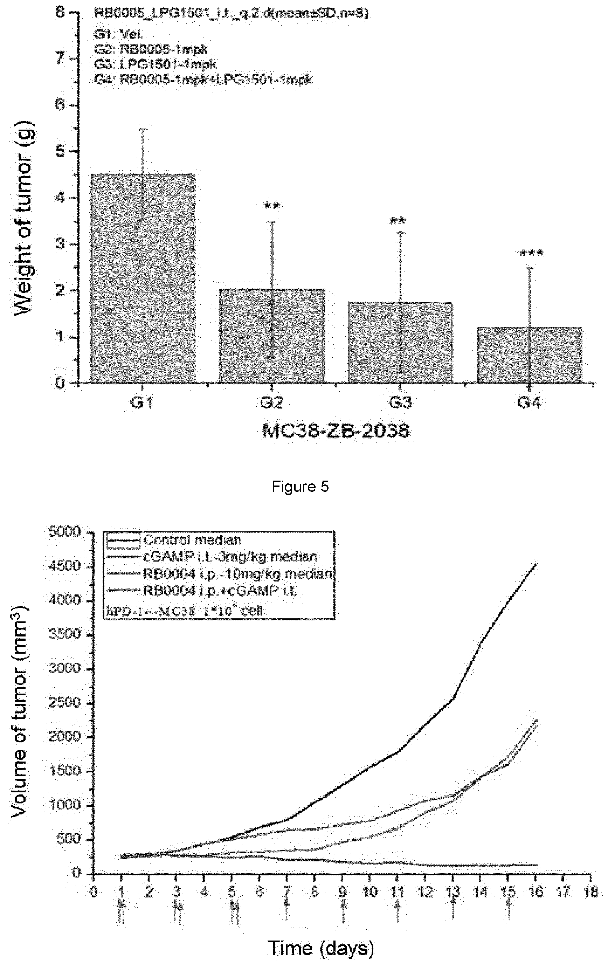

Figures 5-6 show that both the novel pharmaceutical combination (separate dosage form) and each single drug can inhibit the growth of tumor, and the anatomical tumor weight is significantly lower than the negative control group (P<0.05, P<0.001). The novel pharmaceutical combinations have better efficacy than 2',3'-cGAMP or PD-1 antibody RB0004 alone, indicating that the novel pharmaceutical combinations have greater advantages.

As shown in Figure 5, the tumor volume of mice in the solvent control group (G1) gradually increased over time; after treatment, the growth in tumor volume of mice in the single drug group (G2/G3) was slower than that in the solvent control group, and the tumor volume was smaller; the growth in tumor volume of mice in the trial group (G4) was significantly slowed down, and the overall volume showed a decrease.

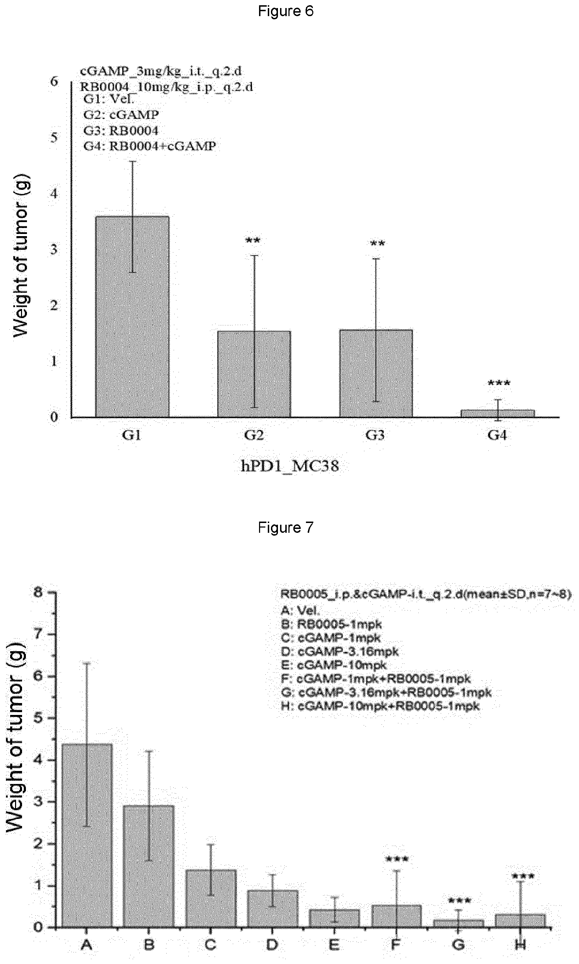

As shown in Figure 6, the tumor weight of mice in the trial group (G4) was significantly reduced, with a tumor weight inhibitory rate of 96%, which showed a statistically significant difference (P<0.001).

**Example 3**

The anti-tumor effect of a pharmaceutical combination composed of PD-L1 inhibitor RB0005 and different doses of the STING agonist 2',3'-cGAMP was tested using a xenograft tumor model of colorectal carcinoma MC38 in mice, i.e., the inhibitory effect on the growth of subcutaneous xenograft tumor in mice.

**3.1 Xenograft tumor model in mice**

After successfully establishing a subcutaneous xenograft tumor model in mice, they were randomly grouped with 8 mice per group and administered once every two days.

| Groups | Administration Method | Administration dosage | Administration frequency |
|---|---|---|---|
| A Negative Control | Intratumor injection | Solvent | Administering every other day |
| B single drug group | Intratumor injection | PD-L1 monoclonal antibody RB0005, 1 mg/kg | Administering every other day |
| C single drug group | Intratumor injection | 2',3'-cGAMP monotherapy, 1 mg/kg | Administering every other day |
| D single drug group | Intratumor injection | 2',3'-cGAMP monotherapy, 3.16 mg/kg | Administering every other day |
| E single drug group | Intratumor injection | 2',3'-cGAMP monotherapy, 10 mg/kg | Administering every other day |
| F trial group (separate dosage form) | Intratumor injection | 2',3'-cGAMP monotherapy, 1 mg/kg | Administering every other day |
| | Intraperitoneal injection | PD-L1 monoclonal antibody RB0005, 1 mg/kg | |
| G trial group (separate dosage form) | Intratumor injection | 2',3'-cGAMP monotherapy, 3.16 mg/kg | Administering every other day |
| | Intraperitoneal injection | PD-L1 monoclonal antibody RB0005, 1 mg/kg | |
| H trial group (separate dosage form) | Intratumor injection | 2',3'-cGAMP monotherapy, 10 mg/kg | Administering every other day |
| | Intraperitoneal injection | PD-L1 monoclonal antibody RB0005, 1 mg/kg | |

**3.2 Results**

Figure 7 shows that all three pharmaceutical combinations and single drug alone can inhibit the growth of tumor, and the pharmaceutical combination has a more significant inhibitory effect, with tumor weight inhibitory rates of over 90% (P<0.001). The novel pharmaceutical combinations have better efficacy than PD-L1 antibody RB0005 and 2',3'-cGAMP used alone, indicating that the novel pharmaceutical combinations have greater advantages.

**Example 4**

The anti-tumor effect of a pharmaceutical combination composed of PD-1 inhibitor RB0004 and different doses of the STING agonist 2',3'-cGAMP was tested using a xenograft tumor model, i.e., the inhibitory effect on the growth of subcutaneous xenograft tumor in mice.

**4.1 Xenograft tumor model in mice**

After successfully establishing a subcutaneous xenograft tumor model in mice, they were randomly grouped with 8 mice per group and administered once every two days.

| Groups | Administration Method | Administration dosage | Administration frequency |
|---|---|---|---|
| G1 Negative Control | Intratumor injection | Solvent | Administering every other day |
| G2 single drug group | Intraperitoneal injection | PD-1 monoclonal antibody RB0004, 10 mg/kg | Administering every other day |
| G3 trial group (separate dosage form) | Intratumor injection | 2',3'-cGAMP monotherapy, 0.03 mg/kg | Administering every other day |
| | Intraperitoneal injection | PD-1 monoclonal antibody RB0004, 10 mg/kg | |
| G4 trial group (separate dosage form) | Intratumor injection | 2',3'-cGAMP monotherapy, 0.1 mg/kg | Administering every other day |
| | Intraperitoneal injection | PD-1 monoclonal antibody RB0004, 10 mg/kg | |
| G5 trial group (separate dosage form) | Intratumor injection | 2',3'-cGAMP monotherapy, 0.3 mg/kg | Administering every other day |
| | Intraperitoneal injection | PD-1 monoclonal antibody RB0004, 10 mg/kg | |

**4.2 Results**

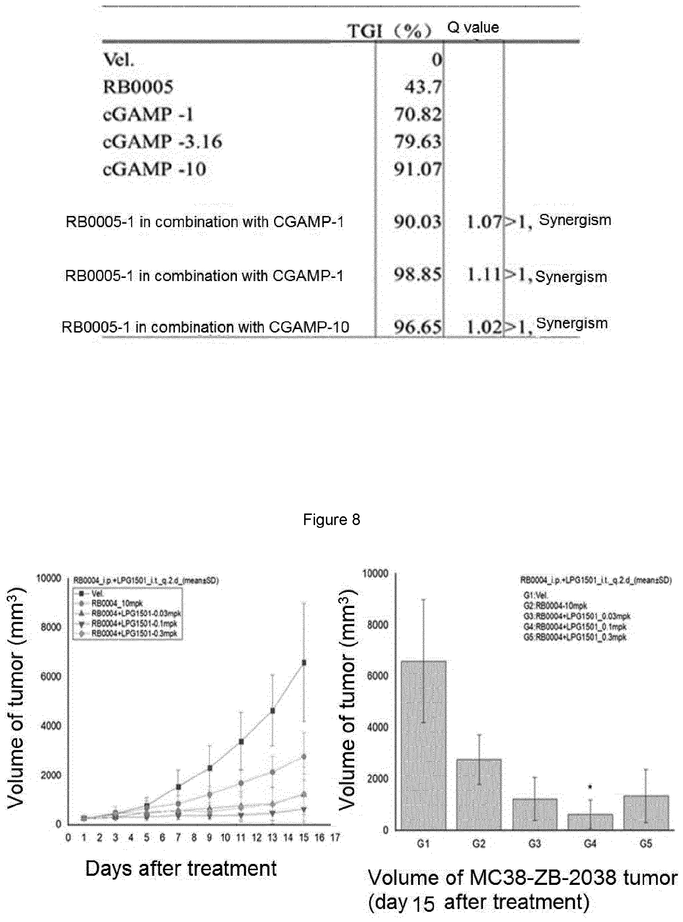

Figures 8-9 show that all three pharmaceutical combinations can inhibit the growth of tumor, and the tumor weight inhibitory rate is above 70%. Especially in the combined 2',3'-cGAMP 0.1 mpk dose group, the inhibitory effect on tumor is significant and the survival rate is high ($P < 0.05$). The efficacy of the novel pharmaceutical combinations is better than that of PD-1 antibody RB0004 alone, indicating that the novel pharmaceutical combinations have greater advantages.

**Example 5**

The anti-tumor effect of a pharmaceutical combination composed of PD-1/PD-L1 inhibitor and the STING agonist 2',3'-cGAMP was tested using a xenograft tumor model, i.e., the inhibitory effect on the growth of subcutaneous xenograft tumor in mice.

**5.1 Xenograft tumor model in mice**

After successfully establishing a humanized CD274 cell subcutaneous xenograft tumor model of colorectal carcinoma MC38 in mice, they were randomly grouped with 6-8 mice per group and administered once every two days, a total of 3 times.

| Groups | Administration Method | Administration dose | Administration frequency |
|---|---|---|---|
| G1 control group | Intratumor injection (i.t.) Intraperitoneal injection (i.p.) | Solvent | Administering every other day |
| G2 single drug group | Intraperitoneal injection (i.p.) | PD-1 monoclonal antibody RB0005, 1 mg/kg | Administering every other day |
| G3 single drug group | Intratumor injection (i.t.) | PD-1 monoclonal antibody RB0005, 1 mg/kg | Administering every other day |
| G4 single drug group | Intraperitoneal injection (i.p.) | 2',3'-cGAMP monotherapy, 1 mg/kg | Administering every other day |
| G5 single drug group | Intratumor injection (i.t.) | 2',3'-cGAMP monotherapy, 1 mg/kg | Administering every other day |
| G6 trial group (Single dosage form) | Intraperitoneal injection (i.p.) | LPG2008: PD-1 monoclonal antibody RB00051mg/kg + 2',3'-cGAMP, 1 mg/kg | Administering every other day |
| G7 trial group (Single dosage form) | Intratumor injection (i.t.) | LPG2008: PD-1 monoclonal antibody RB0005 1mg/kg + 2',3'-cGAMP, 1 mg/kg | Administering every other day |
| G8 trial group | Intraperitoneal injection (i.p.) | PD-1 monoclonal antibody RB0005, 1 mg/kg | Administering every other day |
| (separate dosage form) | Intratumor injection (i.t.) | 2',3'-cGAMP, 1 mg/kg | Administering every other day |

**5.2 Results**

Table 1 Average tumor mass and tumor inhibitory rate in each group

| Groups | Tumor Weight (g) | $IR_{TW}\%$ | CDI (Coefficient of drug interaction) |
|---|---|---|---|
| G1 | 4.43 | - | |
| G2 | 3.24 | 26.8 | |
| G3 | 3.55 | 19.9 | |
| G4 | 3.48 | 21.5 | |
| G5 | 1.84 | 58.5 | |
| G6 | 2.40 | 45.9 | 0.94 (<1) |
| G7 | 0.81 | 81.7 | 0.55 (<1) |
| G8 | 0.97 | 78.1 | 0.72 (<1) |

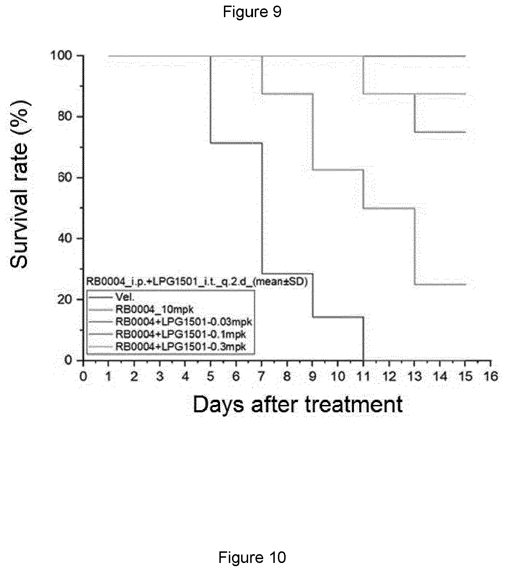

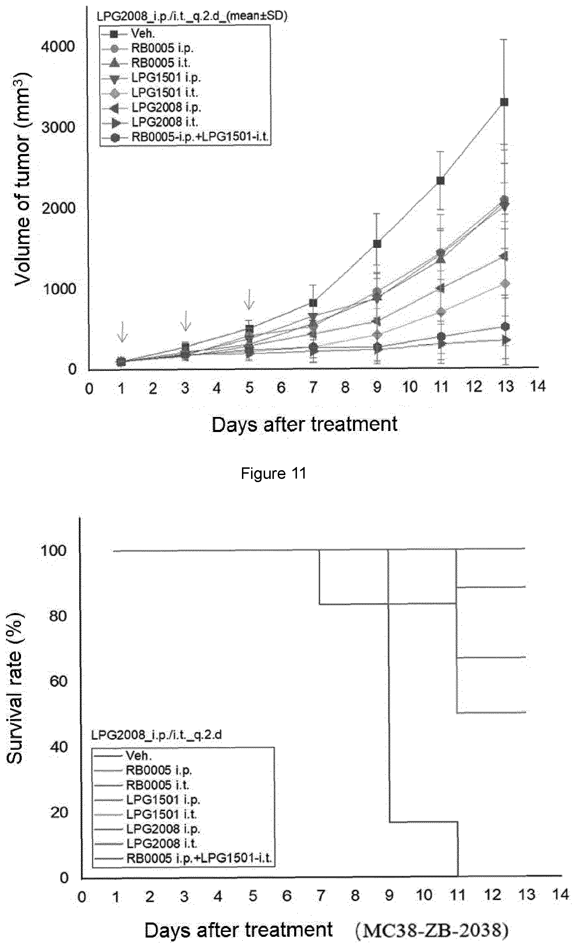

The results are shown in Table 1 and Figures 10 to 12:
The tumor weight inhibitory rates of single drug RB0005 via intraperitoneal and intratumoral (G2\G3) administration modes were 26.8% and 19.9%, respectively; compared with the intraperitoneal and intratumoral (G4\G5) administration modes of cGAMP, the intratumoral administration had a better effect, with a tumor weight inhibitory rate of 58.5%. The intraperitoneal administration had no significant tumor inhibitory effect, and there was statistically significant difference between the intratumoral administration and the control groups (P<0.001);

The novel composite formulation, single dosage form (G6\G7) composite formulation, was administered intraperitoneally and intratumorally. The tumor weight inhibitory rate of intratumoral administration was 81.7%, and the tumor inhibitory effect was superior to that of intraperitoneal administration. The survival rate of mice at the endpoint of the experiment was 100%.

The novel composite formulation in separate dosage form (G8) also exhibited tumor inhibitory effects, with a tumor weight inhibitory rate of 78.1% and a survival rate of mice of 100% at the endpoint of the experiment.

The novel pharmaceutical combinations in this disclosure exhibited good drug synergistic effects, whether in separate dosage forms or single dosage forms and regardless of administering intraperitoneally or intratumorally. Among them, the single dosage form of the composite formulation exhibited significant drug synergistic effects, with a CDI interaction value of 0.55.

**Example 6**

The anti-tumor effect of a pharmaceutical combination composed of PD-1 inhibitor RB0004 and the STING agonist 2',3'-cGAMP was tested using a xenograft tumor model, i.e., the inhibitory effect on the growth of subcutaneous xenograft tumor in mice.

**6.1 Xenograft tumor model in mice**

After successfully establishing a humanized PD-1 subcutaneous xenograft tumor model of colorectal carcinoma MC38 in mice, they were randomly grouped with 6-8 mice per group and administered once every two days, a total of 3 times.

**6.2 Grouping**

| Groups | Administration Method | Administration dose | Administration frequency |
|---|---|---|---|
| G1 control group | Intratumor injection (i.t.) | Solvent | Administering every other day |
| G2 single drug group | Intratumor injection (i.t.) | PD-1 monoclonal antibody RB0004, 10 mg/kg | Administering every other day |
| G3 single drug group | Intratumor injection (i.t.) | 2',3'-cGAMP monotherapy, 1 mg/kg | Administering every other day |
| G4 trial group | Intratumor injection (i.t.) | LPG2009: PD-1 monoclonal antibody RB0004 10 mg/kg + | Administering every other day |
| (Single dosage form) | | 2',3'-cGAMP, 1 mg/kg | |

**6.3 Results**

Table 2 Average tumor mass and tumor inhibitory rate in each group

| Groups | Tumor Weight (g) | $IR_{TW}$% | CDI (Coefficient of drug interaction) |
|---|---|---|---|
| G1 | 2.21 | - | |
| G2 | 0.87 | 60.8 | |
| G3 | 0.84 | 61.9 | |
| G4 | 0.15 | 93.2 | 0.45 |

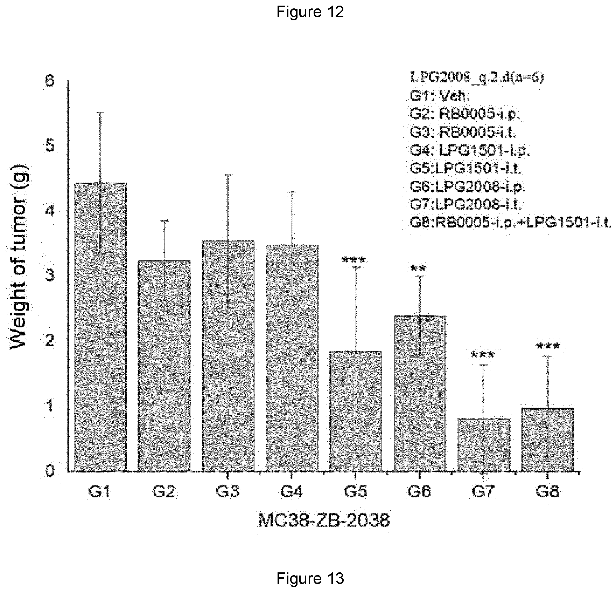

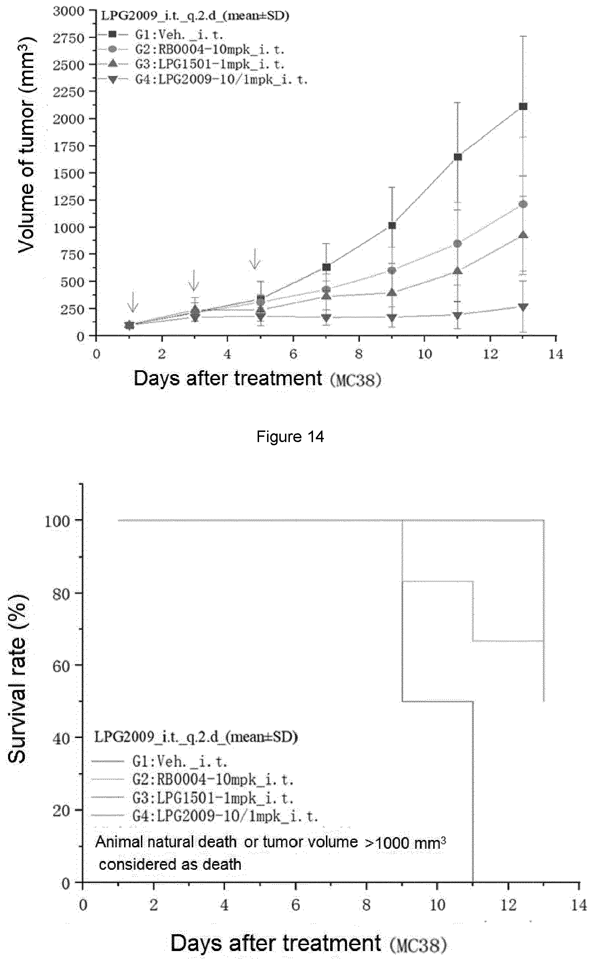

The results are shown in Table 2 and Figures 13 to 15:
The tumor weight inhibitory rate of RB0004 monotherapy was 60.8%; and the tumor weight inhibitory rate of cGAMP monotherapy was 61.9%;

The composite formulation (single dosage form) (G4) had a significant inhibitory effect on tumor proliferation. After administration, the tumor proliferation slowed down, and the tumor weight inhibitory rate was 93.2%, there was statistically significant difference compared with the control group (P<0.001). The survival rate of mice at the endpoint of the experiment was 100%, showing a significant drug synergistic effect (CDI value = 0.45).

In summary, the single dosage form of 2',3'-cGAMP and PD-1 monoclonal antibody has a significant tumor inhibitory effect, high survival rate (P<0.05), and significant drug synergistic effects. The efficacy of the novel pharmaceutical combinations is better than that of PD-1 antibody RB0004 alone, indicating that the novel pharmaceutical combinations have greater advantages.

## Example 7

The anti-tumor effect of a pharmaceutical combination composed of the marketed PD-1\PD-L1 inhibitor and the STING agonist 2',3'-cGAMP was tested using a xenograft tumor model, i.e., the inhibitory effect on the growth of subcutaneous xenograft tumor in mice.

### 7.1 Xenograft tumor model in mice

After successfully establishing a subcutaneous xenograft tumor model of colorectal carcinoma MC38 in mice, they were randomly grouped with 6-8 mice per group and administered once every two days, a total of 3 times.

### 7.2.1 Grouping 1 (PD-1 inhibitor)

| Groups | Administration Method | Administration dose | Administration frequency |
|---|---|---|---|
| G1 Control group | Intratumor injection (i.t.) | Solvent | Administering every other day |
| G2 single drug group | Intratumor injection (i.t.) | 2',3'-cGAMP monotherapy, 1 mg/kg | Administering every other day |
| G3 single drug group | Intratumor injection (i.t.) | PD-1 monoclonal antibody Tislelizumab, 10 mg/kg | Administering every other day |
| G4 trial group (Single dosage form) | Intratumor injection (i.t.) | PD-1 monoclonal antibody Tislelizumab, 10 mg/kg + 2',3'-cGAMP monotherapy, 1 mg/kg | Administering every other day |
| G5 single drug group | Intratumor injection (i.t.) | PD-1 monoclonal antibody Camrelizumab, 10 mg/kg | Administering every other day |
| G6 trial group (Single dosage form) | Intratumor injection (i.t.) | PD-1 monoclonal antibody Camrelizumab, 10 mg/kg + 2',3'-cGAMP monotherapy, 1 mg/kg | Administering every other day |
| G7 single drug group | Intratumor injection (i.t.) | PD-1 monoclonal antibody Sintilimab, 10 mg/kg | Administering every other day |
| G8 trial group (Single dosage form) | Intratumor injection (i.t.) | PD-1 monoclonal antibody Sintilimab, 10 mg/kg + 2',3'-cGAMP monotherapy, 1 mg/kg | Administering every other day |
| G9 single drug group | Intratumor injection (i.t.) | PD-1 monoclonal antibody Toripalimab, 10 mg/kg | Administering every other day |
| G10 trial group (Single dosage form) | Intratumor injection (i.t.) | PD-1 monoclonal antibody Toripalimab, 10 mg/kg + 2',3'-cGAMP monotherapy, 1mg/kg | Administering every other day |

### 7.3.1 Result 1 (PD-1 inhibitor)

Table 3 Average tumor mass and tumor inhibitory rate in each group

| Groups | Tumor Weight (g) | $IR_{TW}$% | CDI (Coefficient of drug interaction) |
|--------|------------------|-----------|---------------------------------------|
| G1 | 1.53 | - | |
| G2 | 0.98 | 36 | |
| G3 | 0.59 | 61.4 | |
| **G4** | **0.18** | **88.2** | |
| G5 | 1.23 | 19.6 | |
| **G6** | **0.32** | **79.1** | **0.41** |
| G7 | 1.47 | 3.9 | |
| **G8** | **0.1** | **93.5** | **0.11** |
| G9 | 0.92 | 49.7 | |
| G10 | 0.42 | 77.1 | 1 |

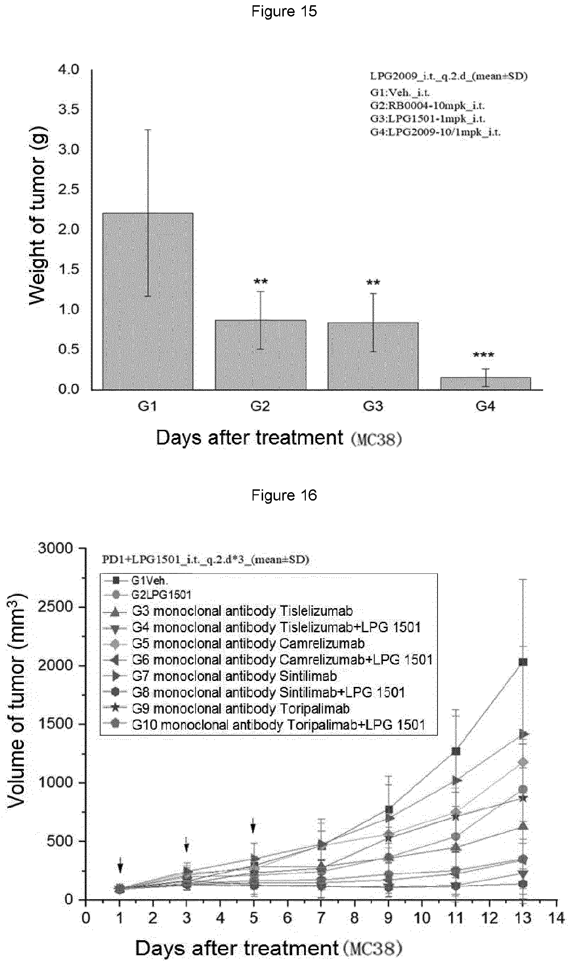

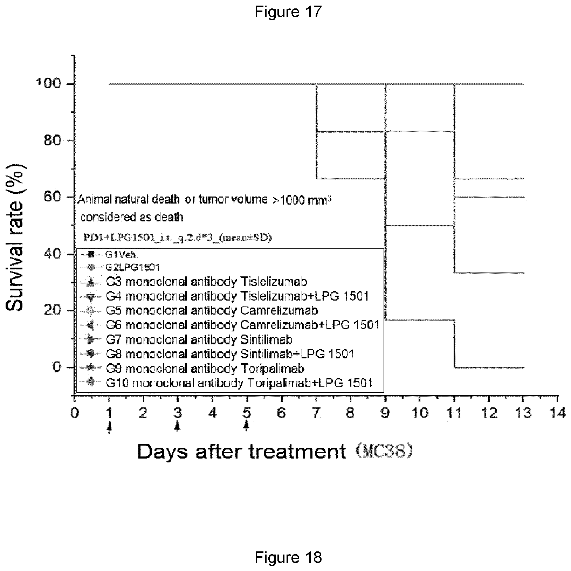

The results are shown in Table 3 and Figures 16 to 18:
Compared with the control group, the composite formulation of the cGAMP and the marketed PD1 inhibitor G4\6\8\10 showed statistically significant difference (P<0.001), with tumor weight inhibitory rates of 88.2%, 79.1%, 93.5%, and 77.1%, respectively. All four composite formulations showed drug synergistic effects, wherein the composite formulation in G8 group (Sintilimab) had the most significant drug synergistic effect.

At the endpoint of the experiment, the survival rate of G2 (LPG1501)\G3 (tislelizumab)\G9(TERRE) in the single drug group was above 60%; and the survival rates of the composite formulation G4\G6\G8\G10S were all 100%, the survival rates were significantly improved.

In summary, the composite formulations of 2',3'-cGAMP and four marketed PD-1 monoclonal antibodies (single dosage form) showed significant tumor inhibitory effect, high survival rate (P<0.05), and exhibited drug synergistic effects.

**7.2.2 Grouping 2 (PD-L1 inhibitor Durvalumab)**

| Groups | Administration Method | Administration dose | Administration frequency |
|--------|----------------------|---------------------|--------------------------|
| G1 control group | Intratumor injection (i.t.) | Solvent | Administering every other day |
| G2 single drug group | Intratumor injection (i.t.) | PD-1 monoclonal antibody Durvalumab, 1 mg/kg | Administering every other day |
| G3 single drug group | Intratumor injection (i.t.) | 2',3'-cGAMP monotherapy, 1 mg/kg | Administering every other day |
| G4 trial group (Single dosage form) | Intratumor injection (i.t.) | PD-1 monoclonal antibody Durvalumab, 1 mg/kg +2',3'-cGAMP, 1 mg/kg | Administering every other day |

**7.3.2 Results (PD-L1 inhibitor Durvalumab)**

Table 4 Average tumor mass and tumor inhibitory rate in each group

| Groups | Tumor Weight (g) | $IR_{TW}$% | CDI (Coefficient of drug interaction) |
|--------|------------------|-----------|---------------------------------------|
| G1 | 3.35 | - | |
| G2 | 2.42 | 27.7 | |

(continued)

| Groups | Tumor Weight (g) | IR$_{TW}$% | CDI (Coefficient of drug interaction) |
|---|---|---|---|
| G3 | 1.74 | 48.0 | |
| G4 | 1.22 | 63.6 | 0.97 |

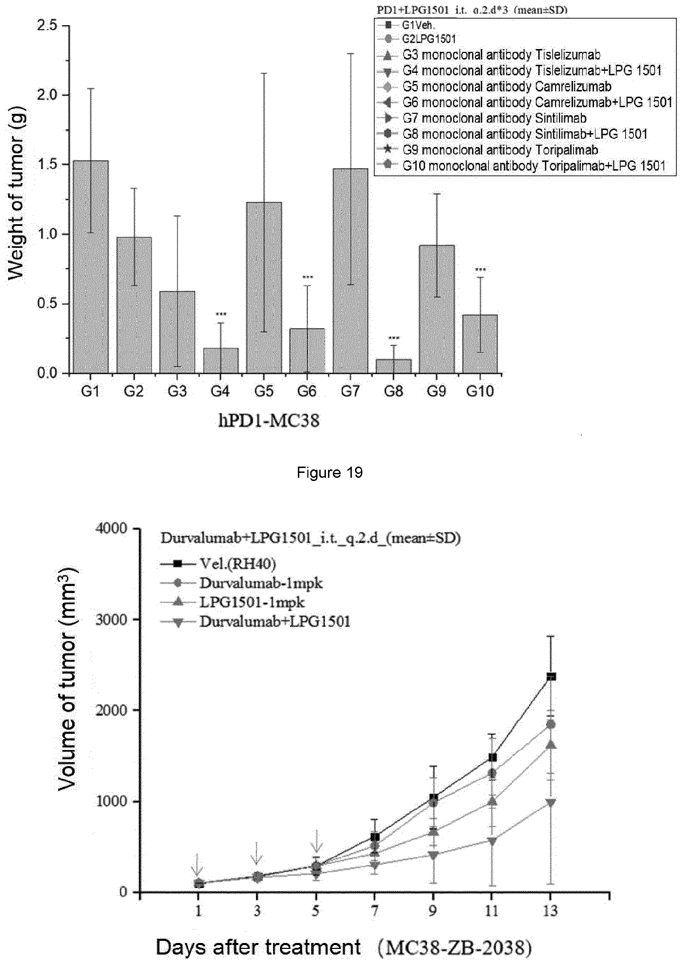

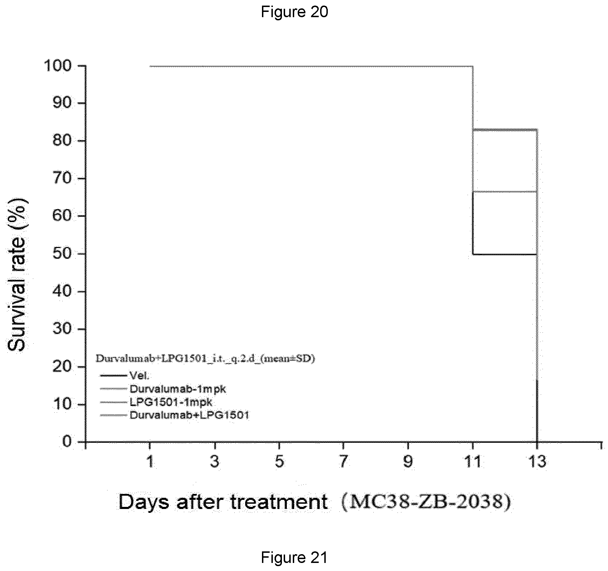

The results are shown in Table 4 and Figures 19 to 21:
The composite formulation (G4) of cGAMP and the marketed PD-L1 inhibitor (Durvalumab) had an inhibitory effect on tumor proliferation, and its efficacy was superior to that of cGAMP or PD-L1 antibody alone. There was a statistically significant difference (P<0.001), and the tumor weight inhibitory rate was 63.6%. At the endpoint of the experiment, the survival rate of mice in the composite formulation group was above 80%, the survival rate was significantly improved.

In summary, the composite formulation of 2',3'-cGAMP and the marketed PD-L1 monoclonal antibody (single dosage form) showed significant tumor inhibitory effect, high survival rate (P<0.05), and exhibited drug synergistic effect.

### 7.2.3 Grouping 3 (PD-L1 inhibitor Tecentriq)

| Groups | Administration Method | Administration dose | Administration frequency |
|---|---|---|---|
| G1 control group | Intratumor injection (i.t.) | Solvent | Administering every other day |
| G2 single drug group | Intratumor injection (i.t.) | PD-1 monoclonal antibody Tecentriq, 1 mg/kg | Administering every other day |
| G3 single drug group | Intratumor injection (i.t.) | 2',3'-cGAMP monotherapy, 1 mg/kg | Administering every other day |
| G4 trial group (Single dosage form) | Intratumor injection (i.t.) | PD-1 monoclonal antibody Tecentriq, 1 mg/kg +2',3'-cGAMP, 1 mg/kg | Administering every other day |
| G5 single drug group | Intratumor injection (i.t.) | PD-1 monoclonal antibody RB0005, 1 mg/kg | Administering every other day |
| G6 trial group (Single dosage form) | Intratumor injection (i.t.) | PD-1 monoclonal antibody RB0005, 1 mg/kg +2', 3'-cGAMP, 1 mg/kg | Administering every other day |

### 7.3.3 Results (PD-L1 inhibitor Tecentriq)

Table 5 Average tumor mass and tumor inhibitory rate in each group

| Groups | Tumor Weight (g) | IR$_{TW}$% |
|---|---|---|
| G1 | 1.86 | |
| G2 | 0.7 | 62.4 |
| G3 | 0.9 | 51.6 |
| G4 | 0.7 | 62.4 |
| G5 | 0.8 | 56.9 |
| G6 | 0.5 | 73.1 |

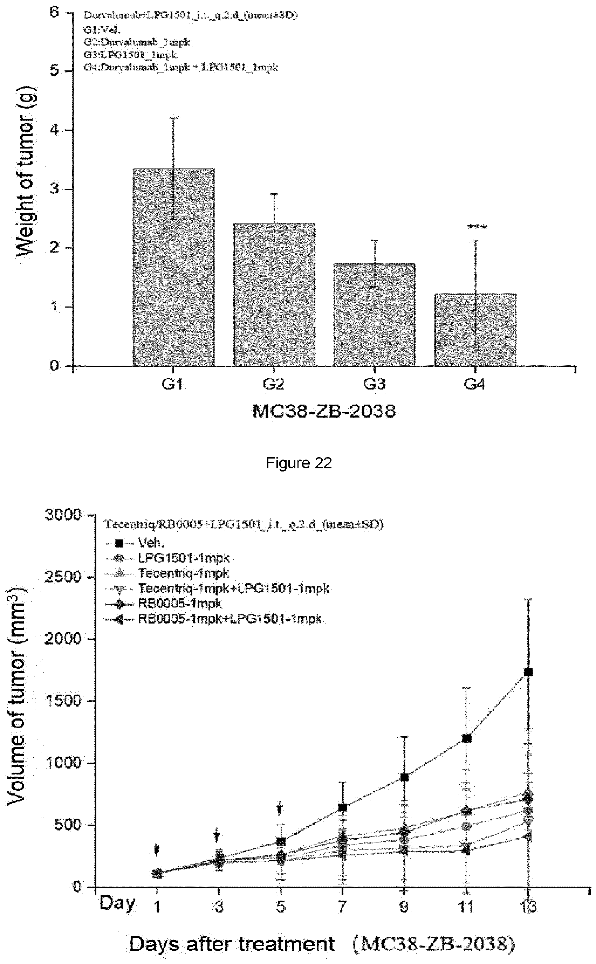

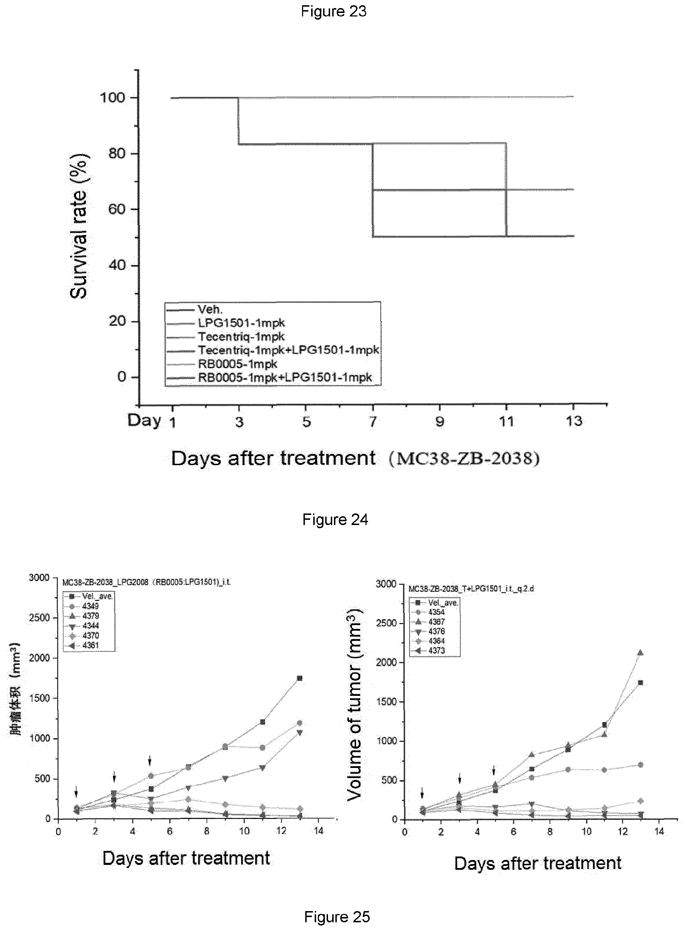

The results are shown in Table 5 and Figures 22 to 25:

The tumor weight inhibitory rate of the composite formulation of cGAMP and RB0005 (G6) was 73.1%, there was statistically significant difference compared with the control group (p<0.05); the tumor weight inhibitory rate of the composite formulation (G4) of cGAMP and the marketed PD-L1 inhibitor (Tecentriq) was 62.4%, and the efficacy of the composite formulation was superior to that of cGAMP or PD-L1 antibody alone.

During the administration, the tumor proliferation in the composite formulation group was slow or even disappeared, and at the endpoint of the experiment, the tumor of 50% mice in the composite formulation group disappeared (Tv<80 mm$^3$).

In summary, the composite formulation of 2',3'-cGAMP and RB0005 as well as the marketed PD-L1 monoclonal antibody (single dosage form) showed significant tumor inhibitory effect, and high survival rate (P<0.05).

**Example 8**

The anti-tumor effect of a pharmaceutical combination composed of PD-L1 inhibitor RB0005 and the STING agonist 2',3'-cGAMP during different tumor proliferation stages was tested using a xenograft tumor model, i.e., the inhibitory effect on the growth of subcutaneous xenograft tumor in mice.

**8.1 Xenograft tumor model in mice**

After successfully establishing a humanized CD274 subcutaneous xenograft tumor model of colorectal carcinoma MC38 in mice, they were randomly grouped with 6-8 mice per group and administered once every two days, a total of 3 times.

**8.2 Grouping**

| Groups | Administrati on Method | Initial tumor volume | Administration dose | Administrati on frequency |
|---|---|---|---|---|
| G1 control group | Intratumor injection (i.t.) | - | PD-1 monoclonal antibody RB0005, 1 mg/kg + 2',3'-cGAMP, 1 mg/kg | Administerin g every other day |
| G2 trial group (Single dosage form) | Intratumor injection (i.t.) | 250 mm$^3$ | | |
| G3 trial group (Single dosage form) | Intratumor injection (i.t.) | 500mm$^3$ | | |
| G4 trial group (Single dosage form) | Intratumor injection (i.t.) | 1000mm$^3$ | | |

**8.3 Results**

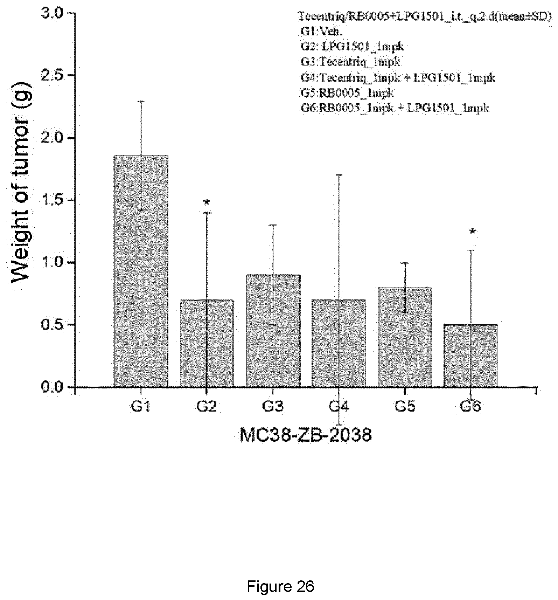

As shown in Figure 26,

The tumor volume inhibitory rate of the composite formulation after administration during the early stage of tumor proliferation (with an average volume of 250 mm$^3$) was 49.9%, which showed a significant statistical difference compared to the control group (P<0.01);

The tumor volume inhibitory rate of the composite formulation after administration during the rapid stage of tumor proliferation (with an average volume of 500 mm$^3$) was 55.5%, which showed a statistical difference compared to the control group (P<0.05);

The tumor volume inhibitory rate of the composite formulation after administration during the late stage of tumor proliferation (with an average volume of 1000 mm$^3$) was 26.4%, which showed no statistical difference compared to the control group (P>0.05);

In conclusion: the novel composite formulation (single dosage form) has tumor inhibitory effects during both the early stage of tumor proliferation and the rapid stage of tumor proliferation, especially during the early stage of tumor proliferation, where the inhibitory effect is more significant.

**Example 9**

The inhibitory effect on the growth of MC38 xenograft tumor model by a small molecule STING agonist 2',3'-cGAMP administered firstly, followed by PD-L1 inhibitor RB0005 administered after 48 hours, was tested with a xenograft tumor model.

**9.1 Xenograft tumor model in mice**

After successfully establishing a humanized PD-1 subcutaneous xenograft tumor model of colorectal carcinoma MC38 in mice, they were randomly grouped with 6-8 mice per group and administered once every two days, a total of 3 times.

**9.2 Grouping**

| Groups | Administrati on Method | Administration dose | Administration frequency |
|---|---|---|---|
| G1 control group | Intratumor injection (i.t.) | Solvent | Day 1, 3, 5 |
| | Intraperitoneal injection (i.p.) | | Day 3, 5, 7 |
| G2 single drug group | Intratumor injection (i.t.) | Solvent | Day 1, 3, 5 |
| | Intraperitoneal injection (i.p.) | PD-1 monoclonal antibody RB0005, 1 mg/kg | Day 3, 5, 7 |
| G3 single drug group | Intratumor injection (i.t.) | 2',3'-cGAMP monotherapy, 1 mg/kg | Day 1, 3, 5 |
| | Intraperitoneal injection (i.p.) | Solvent | Day 3, 5, 7 |
| G4 trial group ( | Intratumor injection (i.t.) | 2',3'-cGAMP, 1 mg/kg | Day 1, 3, 5 |
| | Intraperitoneal injection (i.p.) | PD-1 monoclonal antibody RB0005, 1 mg/kg | Day 3, 5, 7 |

**9.3 Results**

Table 6 Average tumor mass and tumor inhibitory rate in each group

| Groups | Tumor Weight (g) | $IR_{TW}\%$ | CDI (Coefficient of drug interaction) |
|---|---|---|---|
| G1 | 2.00 | - | |
| G2 | 1.98 | 8.4 | |
| G3 | 1.30 | 73.4 | |
| G4 | 0.63 | 95.5 | 0.17 |

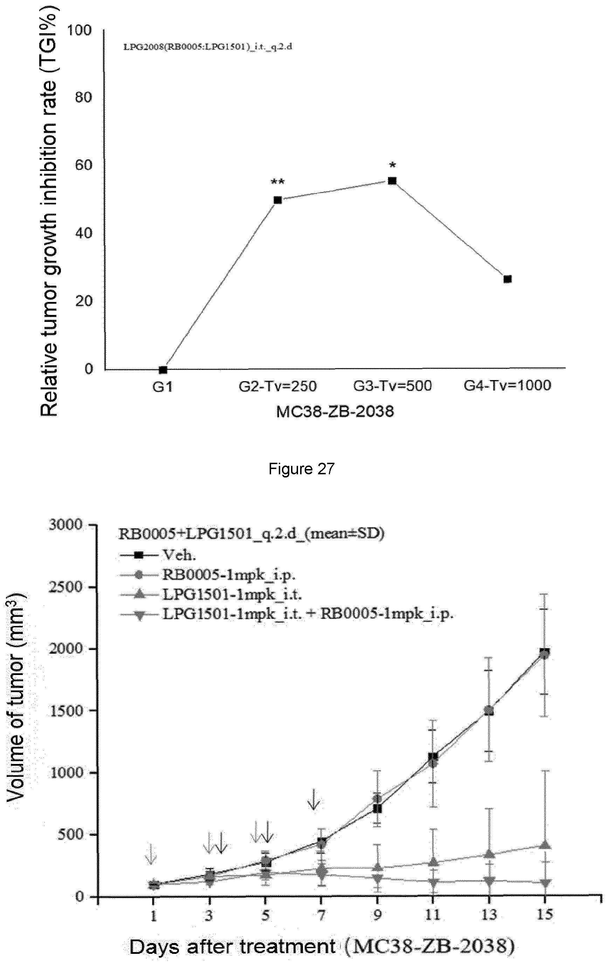

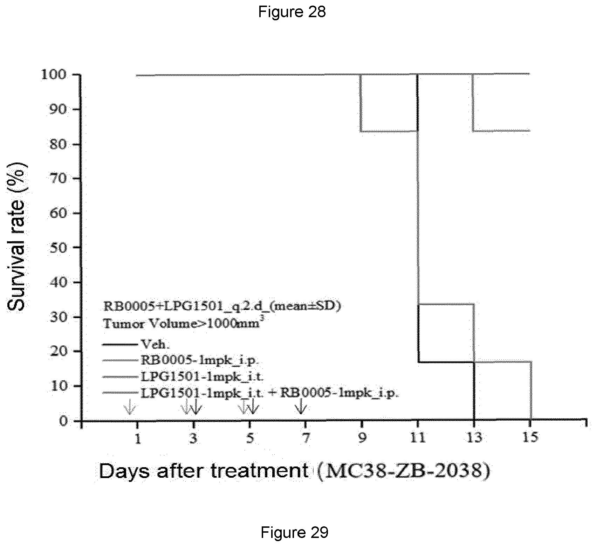

The results are shown in Table 6 and Figures 27 to 29:
The sequential administration group (G4) had a significant inhibitory effect on tumor proliferation, and during the observation period after administration, the tumor volume continued to decrease or even disappear. There was a statistically significant difference between the sequential administration group (G4) and the control group and RB0005 single drug group (ANOVA, P<0.001), with a tumor weight inhibitory rate of 95.5%.

Sequential combination (firstly administering small molecule 2',3'-cGAMP, followed by macromolecule, i.e., PD-L1 monoclonal antibody) showed significant tumor inhibitory effect and exhibited drug synergistic effect, with CDI=0.17.

At the endpoint of the experiment, the survival rate of mice in the sequential combination administration group was 100%, the survival rate was significantly improved compared to the control group and the RB0005 single drug group.

In summary, the administration method of the combination of 2',3'-cGAMP and PD-L1 monoclonal antibody, where the small molecule was firstly administered, followed by the PD-L1 inhibitor, showed significant tumor inhibitory effect, improved survival rate of mice, and exhibited drug synergistic effects.

## Example 10

The anti-tumor effect of a pharmaceutical combination composed of PD-L1 inhibitor and the STING agonist 2',3'-cGAMP was tested using a xenograft tumor model, i.e., the inhibitory effect on the growth of subcutaneous xenograft tumor in mice.

### 10.1 Xenograft tumor model in mice

After successfully establishing a humanized CD274 cell subcutaneous xenograft tumor model of breast carcinoma CT26 in BALBc mice, they were randomly grouped with 6-8 mice per group and administered once every two days, a total of 3 times.

### 10.2 Grouping

| Groups | Administrati on Method | Administration dose | Administration frequency |
|---|---|---|---|
| G1 control group | Intratumor injection (i.t.) | Solvent | Administering every other day |
| G2 single drug group | Intratumor injection (i.t.) | D-1 monoclonal antibody RB0005, 1 mg/kg | Administering every other day |
| G2 trial group | Intratumor injection (i.t.) | D-1 monoclonal antibody RB0005, 1 mg/kg + | Administering every other day |
| (Single dosage form) | | 2',3'-cGAMP monotherapy, 0.1 mg/kg | |
| G3 trial group (Single dosage form) | Intratumor injection (i.t.) | D-1 monoclonal antibody RB0005, 1 mg/kg + 2',3'-cGAMP monotherapy, 0.316mg/kg | Administering every other day |
| G4 trial group (Single dosage form) | Intratumor injection (i.t.) | PD-1 monoclonal antibody RB0005, 1 mg/kg + 2',3'-cGAMP monotherapy, 1 mg/kg | Administering every other day |

### 10.3 Results

Table 7 Average tumor volume and tumor inhibitory rate in each group

| Groups | Tv (mm$^3$) | TGI (%) |
|---|---|---|
| G1 | 1499.1 | - |
| G2 | 974.7 | 35.0 |
| G3 | 868.0 | 42.1 |
| G4 | 486.7 | 67.5 |
| G5 | 142.5 | 90.5 |

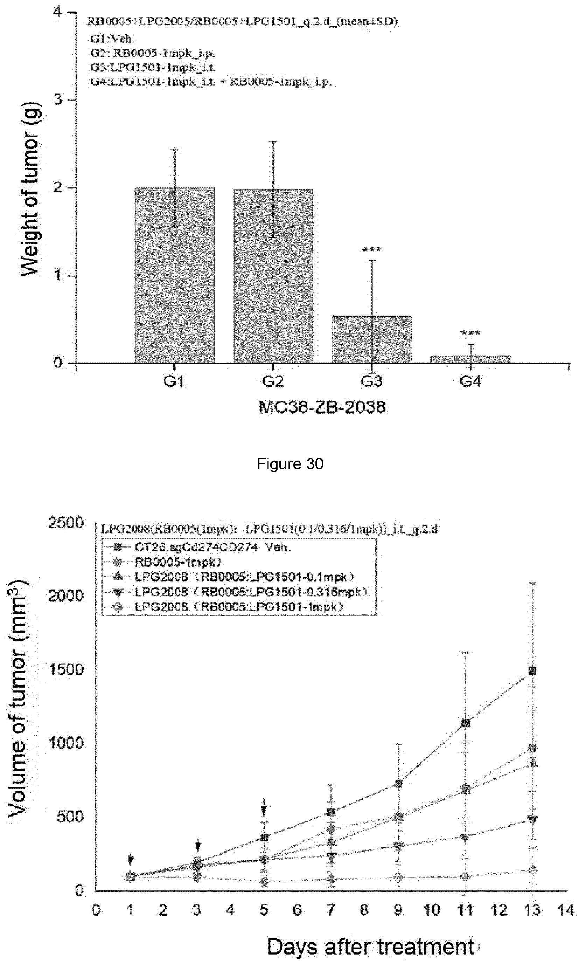

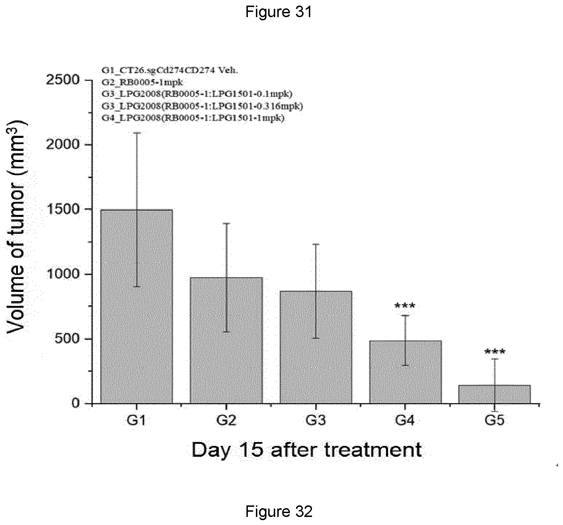

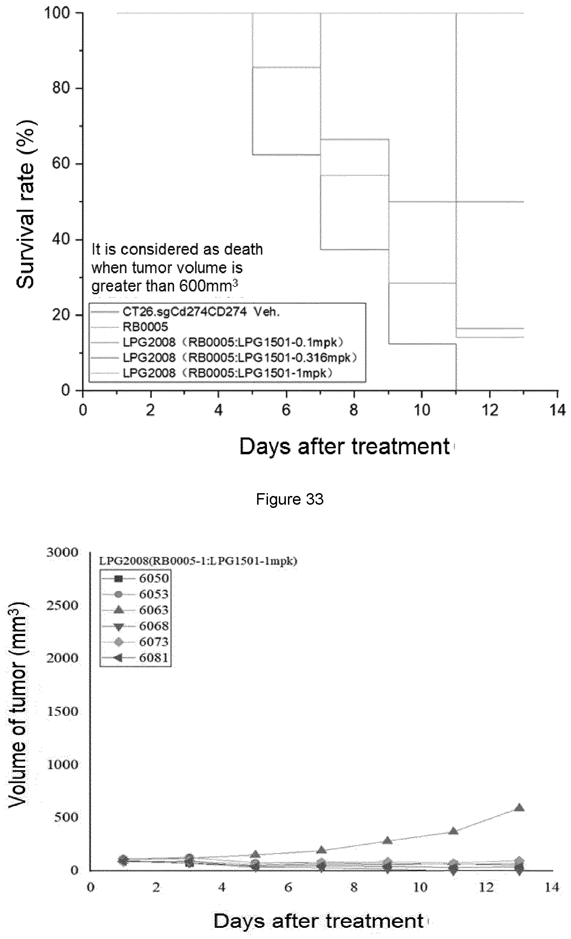

The results are shown in Table 7 and Figures 30 to 33:

During the administration and observation period of the composite formulation of different doses of cGAMP and RB0005, the tumor proliferation was slow, wherein the tumor in the composite formulation group (G4) of different doses of cGAMP and LPG1501 (1 mpk) was decreased or even disappeared, the tumor volume at the endpoint of the experiment showed statistically significant difference compared to the control group ($p<0.05$);

In the solvent control group, the survival rate of mice on day 11 was 0, while the survival rates of mice in the composite formulation G3 and G4 groups at the endpoint of the experiment were above 50%.

In summary, the composite formulations of different doses of 2',3'-cGAMP and RB0005 (single dosage form) showed significant tumor inhibitory effect and improved survival rate ($P<0.05$).

**Example 11**

The anti-tumor effect of a pharmaceutical combination composed of PD-L1 inhibitor RB0005 and the STING agonist 2',3'-cGAMP was tested using a xenograft tumor model, i.e., the inhibitory effect on the growth of subcutaneous xenograft tumor in mice.

**11.1 Xenograft tumor model in mice**

After successfully establishing a humanized CD274 cell subcutaneous xenograft tumor model of breast carcinoma 4T1 in BALBc mice, they were randomly grouped with 6-8 mice per group and administered once every two days, a total of 3 times.

**11.2 Grouping**

| Groups | Administration Method | Administration dose | Administration frequency |
|---|---|---|---|
| G1 control group | Intratumor injection (i.t.) | Solvent | Administering every other day |
| G2 single drug group | Intratumor injection (i.t.) | D-1 monoclonal antibody RB0005, 1 mg/kg | Administering every other day |
| G3 single drug group | Intratumor injection (i.t.) | 2',3'-cGAMP monotherapy, 1 mg/kg | Administering every other day |

(continued)

| Groups | Administration Method | Administration dose | Administration frequency |
|---|---|---|---|
| G4 trial group (Single dosage form) | Intratumor injection (i.t.) | D-1 monoclonal antibody RB0005, 1 mg/kg + 2',3'-cGAMP monotherapy, 1 mg/kg | Administering every other day |

**11.3 Results**

Table 8 Average tumor mass and tumor inhibitory rate in each group

| Groups | Tumor Weight (g) | IR$_{TW}$% | CDI (Coefficient of drug interaction) |
|---|---|---|---|
| G1 | 0.96 | | |
| G2 | 0.99 | - | |
| G3 | 0.63 | 34 | |
| G4 | 0.60 | 37.5 | 0.92 |

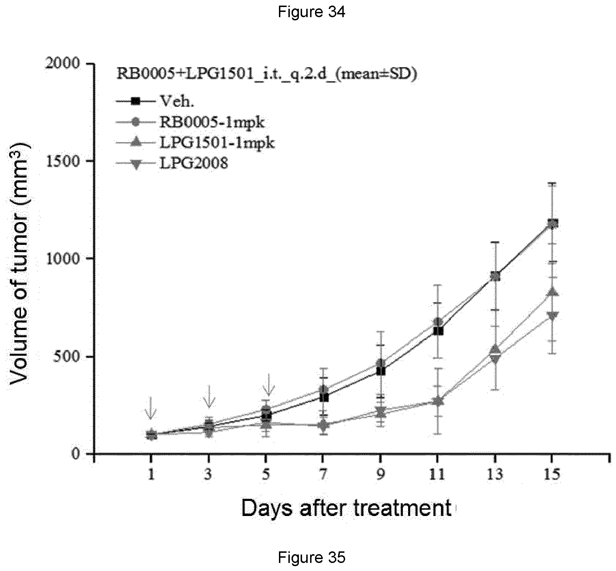

The results are shown in Table 8 and Figures 34 to 36:

4T1 model had poor responsiveness to RB0005 monotherapy, and thus RB0005 monotherapy had no significant tumor inhibitory effect;

cGAMP monotherapy had a certain inhibitory effect on tumor proliferation in 4T1 model, with a tumor weight inhibition ratio of 34%, but there was a great intra-group difference, and the survival rate at the endpoint of the experiment was 66.7%;

the tumor weight inhibitory rate of the composite formulation was 37.5%, which showed a statistically significant difference ($P<0.05$) compared to the control group and RB0005 single drug group, and exhibited drug synergistic effect as well, with a CDI value of less than 1 and a survival rate of 83.3% at the endpoint of the experiment;

In summary, the composite formulation of 2',3'-cGAMP and PD-L1 monoclonal antibody (single dosage form) had a certain tumor inhibitory effect, and improved survival rate in mice, and the responsiveness of RB0005 towards the 4T1 model had been improved by 2',3'-cGAMP.

**Example 12**

The anti-tumor effect of a pharmaceutical combination composed of PD-1 inhibitor RB0004 and the STING agonist 2',3'-cGAMP was tested using a xenograft tumor model, i.e., the inhibitory effect on the growth of subcutaneous xenograft tumor in mice.

**12.1 Xenograft tumor model in mice**

After successfully establishing a humanized PD-1 subcutaneous xenograft tumor model of melanoma B16 in mice, they were randomly grouped with 6-8 mice per group and administered once every two days, a total of 3 times.

**12.2 Grouping**

| Groups | Administration Method | Administration dose | Administration frequency |
|---|---|---|---|
| G1 control group | Intratumor injection (i.t.) | Solvent | Administering every other day |
| G2 single drug group | Intratumor injection (i.t.) | D-1 monoclonal antibody RB0004, 10mg/kg | Administering every other day |
| G3 single drug group | Intratumor injection (i.t.) | 2',3'-cGAMP monotherapy, 0.1mg/kg | Administering every other day |
| G4 trial group (Single dosage form) | Intratumor injection (i.t.) | LPG2009: D-1 monoclonal antibody RB0004, 10 mg/kg + 2', 3'-cGAMP monotherapy, 0.1 | Administering every other day |
| | | mg/kg | |

### 12.3 Results

Table 9 Average tumor volume and tumor inhibitory rate in each group

| Groups | Day 7-Tumor volume (mm$^3$) | TGI% | Q value (q≥1, indicating that the combination of two drugs has a coefficient of drug interaction (CDI) of synergistic effect) |
|---|---|---|---|
| G1 | 1412.1 | - | |
| G2 | 1849.9 | - | |
| G3 | 578.8 | 59.0 | |
| G4 | 213.4 | 84.9 | 1.8 |

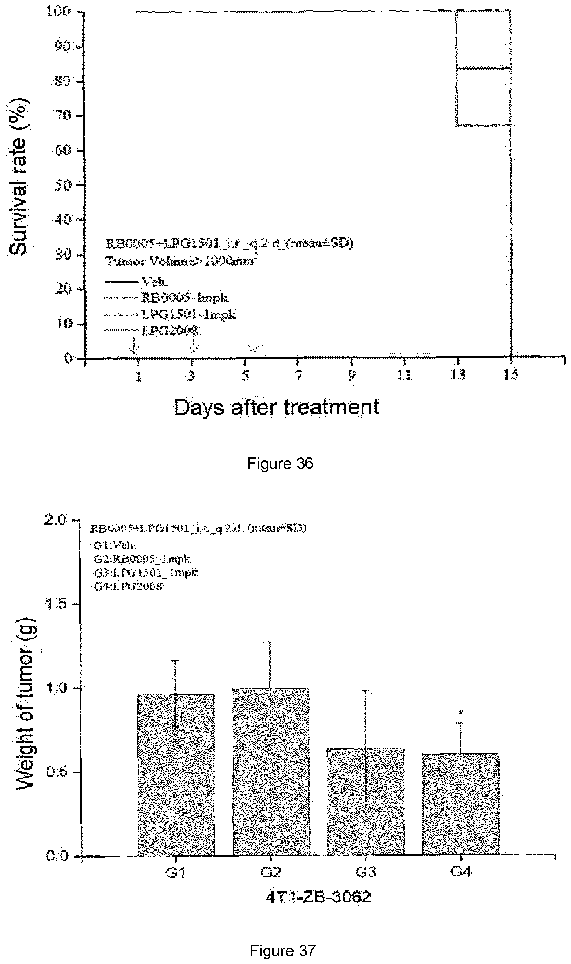

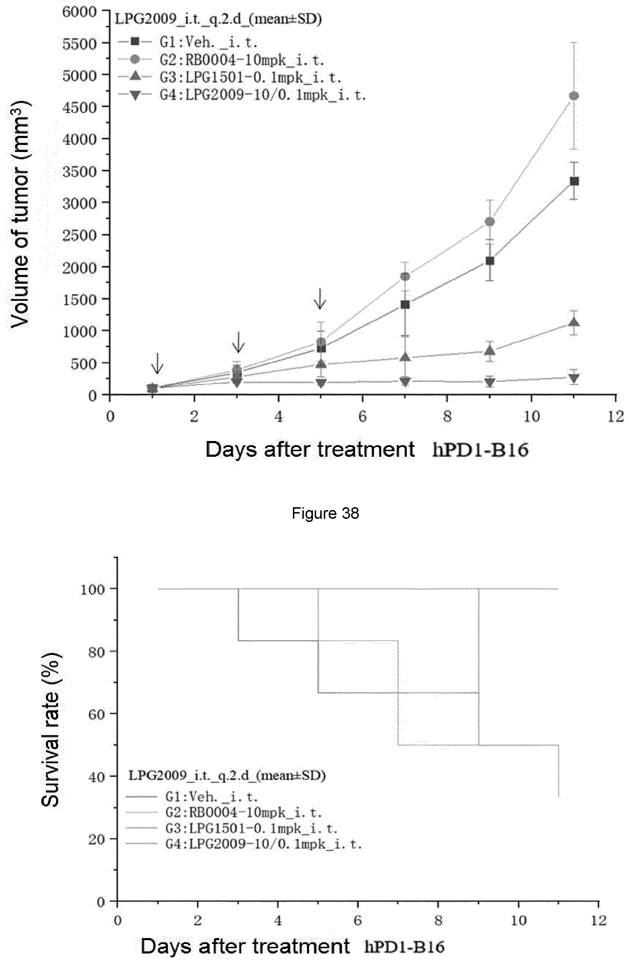

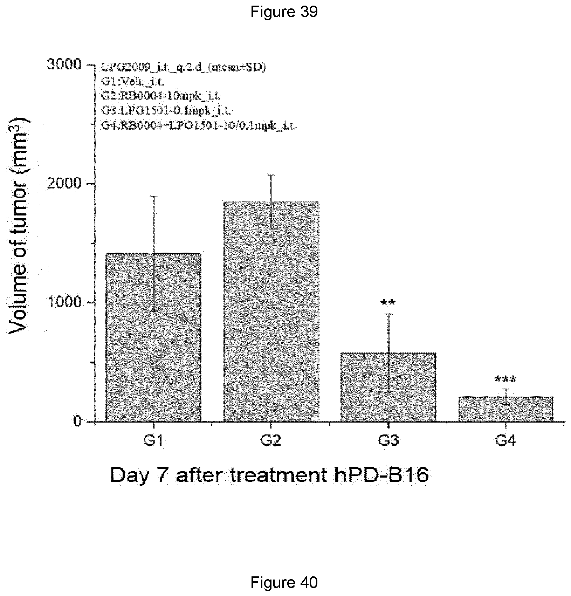

The results are shown in Table 9 and Figures 37 to 40:

On day 7 after the administration of single drug cGAMP, the tumor inhibitory rate was 59%;

the composite formulation (single dosage form) (G4) had a significant inhibitory effect on tumor proliferation. After administration, the tumor proliferation slowed down, and the tumor weight inhibitory rate on day 7 was 84.9%, there was statistically significant difference compared with the control group (P<0.001). The survival rate of mice at the endpoint of the experiment was 100%, showing a significant drug synergistic effect (King's Q value = 1.8).

In summary, the single dosage form of 2',3'-cGAMP and PD-1 monoclonal antibody has a significant tumor inhibitory effect, high survival rate (P<0.05), and significant drug synergistic effects. The efficacy of the novel pharmaceutical combinations is better than that of PD-1 antibody RB0004 alone, indicating that the novel pharmaceutical combinations have greater advantages.

### Example 13

The anti-tumor effect of a pharmaceutical combination composed of PD-1 inhibitor RB0004 and the STING agonist 2',3'-cGAMP was tested using a xenograft tumor model, i.e., the inhibitory effect on the growth of subcutaneous xenograft tumor in mice.

### 13.1 Xenograft tumor model in mice

After successfully establishing a humanized PD-1 subcutaneous xenograft tumor model of lung carcinoma LLC-1 in mice, they were randomly grouped with 6-8 mice per group and administered once every two days, a total of 3 times.

**13.2 Grouping**

| Groups | Administration Method | Administration dose | Administration frequency |
|---|---|---|---|
| G1 control group | Intratumor injection (i.t.) | Solvent | Administering every other day |
| G2 single drug group | Intratumor injection (i.t.) | D-1 monoclonal antibody RB0004, 10 mg/kg | Administering every other day |
| G3 single drug group | Intratumor injection (i.t.) | 2',3'-cGAMP monotherapy, 0.1 mg/kg | Administering every other day |
| G4 trial group (Single dosage form) | Intratumor injection (i.t.) | LPG2009: D-1 monoclonal antibody RB0004, 10 mg/kg + 2',3'-cGAMP monotherapy, 0.1 mg/kg | Administering every other day |

**13.3 Results**

Table 10 Average tumor inhibitory rate in each group

| Groups | TGI% | CDI (Coefficient of drug interaction) |
|---|---|---|
| G1 | - | |
| G2 | - | |
| G3 | 47 | |
| G4 | 72 | 0.55 (<1) |

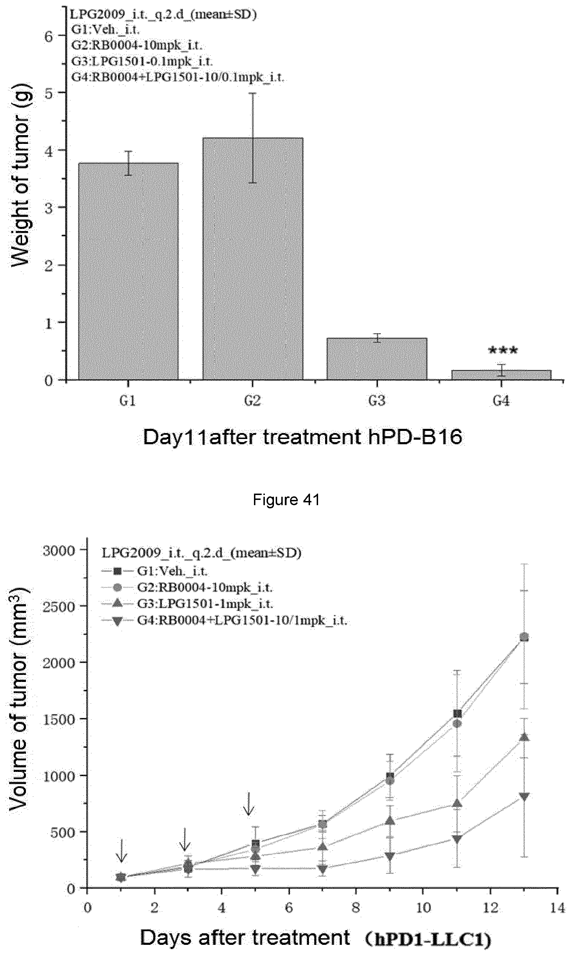

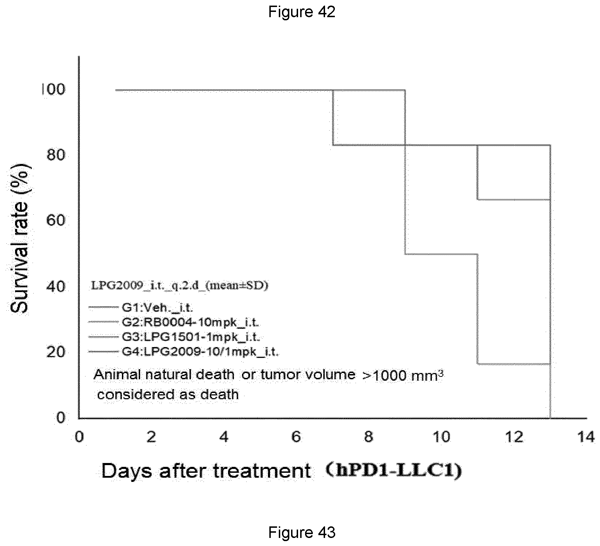

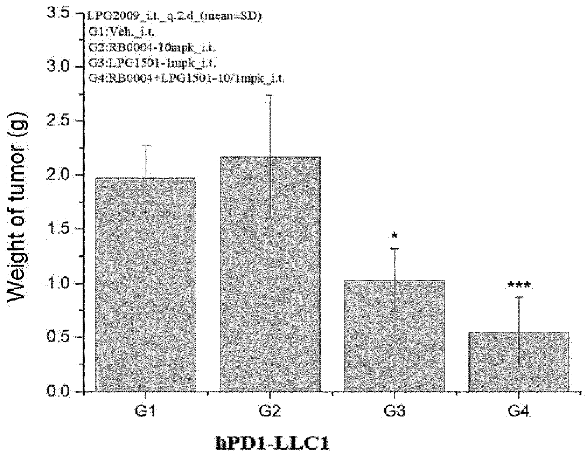

The results are shown in Table 10 and Figures 41 to 43:

RB0004 monotherapy had no significant tumor inhibitory effect on the model; cGAMP monotherapy had tumor inhibitory effect, with a tumor weight inhibitory rate of 47%;

The composite formulation (single dosage form) (G4) had an inhibitory effect on tumor proliferation. After administration, the tumor proliferation slowed down, and the tumor weight inhibitory rate was 72%, there was statistically significant difference compared with the control group (P<0.001), and showed a significant drug synergistic effect (CDI = 0.55).

In summary, the single dosage form of 2',3'-cGAMP and PD-1 monoclonal antibody has a significant tumor inhibitory effect, high survival rate (P<0.05), and drug synergistic effects. The efficacy of the novel pharmaceutical combinations is better than that of PD-1 antibody RB0004 alone, indicating that the novel pharmaceutical combinations have greater advantages.

**Claims**

1. A pharmaceutical combination comprising a programmed cell death protein 1 (PD-1) inhibitor and/or a programmed death ligand 1 (PD-L1) inhibitor, and a STING pathway agonist.

2. The pharmaceutical combination according to claim 1, wherein the STING pathway agonist comprises a cyclic dinucleotide.

3. The pharmaceutical combination according to claim 2, wherein the cyclic dinucleotide is selected from the group consisting of c-di-AMP, c-di-GMP, c-di-GMP-F, 3',3'-cGAMP, 3',3'-cGAMP-F, 2',3'-cGAMP, Rp/Sp(CL656), ADU-S100, ADU-S100 disodium, and derivatives or combinations thereof.

4. The pharmaceutical combination according to claim 3, wherein the STING pathway agonist comprises 2',3'-cGAMP, or derivatives thereof.

5. The pharmaceutical combination according to claim 1, wherein the STING pathway agonist comprises flavonoids.

6. The pharmaceutical combination according to claim 5, wherein the flavonoid comprises CMA, DMXAA, methoxyflavone, 6,4'- dimethoxyflavone, 4'-methoxyflavone, 3',6'-dihydroxyflavone, 7,2'-dihydroxyflavone, daidzein, Formononetin, retinene 7-methyl ether, xanthone, and/or any combination thereof.

7. The pharmaceutical combination according to claim 1, wherein the STING pathway agonist comprises DNA.

8. The pharmaceutical combination according to claim 1, wherein the STING pathway agonist comprises type I interferons (IFNs).

9. The pharmaceutical combination according to claim 8, wherein the type I interferon comprises IFN-$\alpha$ or IFN-$\beta$.

10. The pharmaceutical combination according to any one of claims 1 to 9, wherein the PD-1 inhibitor has one or more of the following characteristics: a. inhibition or reduction of PD-L1 expression, such as transcription or translation of PD-L1; b. inhibition or reduction of PD-1 activity, such as inhibition or reduction of PD-1 binding to its homologous ligands, such as PD-L1 or PD-L2; and c. binding PD-1 or one or more of its ligands, such as PD-L1 or PD-L2.

11. The pharmaceutical combination according to any one of claims 1 to 9, wherein the PD-1 inhibitor comprises an anti PD-1 antibody or antigen-binding fragments thereof.

12. The pharmaceutical combination according to claim 11, wherein the anti PD-1 antibody is selected from the group consisting of Pembrolizumab, Nivolumab, Pidilizumab, SHR-1210, MEDI0680, BGB-A317, TSR-042, REGN2810, PF-06801591, RB0004, Tislelizumab, Camrelizumab, Toripalimab, Sintilimab, bio-analogues, bio-enhancers, bio-equivalents, or combinations thereof.

13. The pharmaceutical combination according to any one of claims 11 to 12, wherein the anti PD-1 antibody comprises at least one CDR in the antibody heavy chain variable region (VH), and the VH comprises an amino acid sequence shown in SEQ ID NO: 8.

14. The pharmaceutical combination according to any one of claims 11 to 13, wherein the anti PD-1 antibody comprises VH comprising HCDR3, and the HCDR3 comprises an amino acid sequence shown in SEQ ID NO: 3.

15. The pharmaceutical combination according to claim 14, wherein the VH further comprises HCDR2, wherein the HCDR2 comprises an amino acid sequence shown in SEQ ID NO: 2.

16. The pharmaceutical combination according to any one of claims 14 to 15, wherein the VH further comprises HCDR1, wherein the HCDR1 comprises an amino acid sequence shown in SEQ ID NO: 1.

17. The pharmaceutical combination according to any one of claims 14 to 16, wherein the VH comprises HCDR1, HCDR2, and HCDR3, wherein the HCDR3 comprises an amino acid sequence shown in SEQ ID NO: 3, the HCDR2 comprises an amino acid sequence shown in SEQ ID NO: 2, and the HCDR1 comprises an amino acid sequence shown in SEQ ID NO: 1.

18. The pharmaceutical combination according to any one of claims 14 to 17, wherein the VH comprises a framework region HFR1, the C-terminal of HFR1 is directly or indirectly connected to the N-terminal of HCDR1, and the HFR1 comprises an amino acid sequence shown in SEQ ID NO: 4 or an amino acid sequence having at least about 70% sequence identity to the amino acid sequence shown in SEQ ID NO: 4.

19. The pharmaceutical combination according to any one of claims 14 to 18, wherein the VH comprises a framework region HFR2, the N-terminal of HFR2 is directly or indirectly connected to the C-terminal of HCDR1, and the C-terminal of HFR2 is directly or indirectly connected to the N-terminal of HCDR2; and the HFR2 comprises an amino acid sequence shown in SEQ ID NO: 5 or an amino acid sequence having at least about 70% sequence identity to the amino acid sequence shown in SEQ ID NO: 5.

20. The pharmaceutical combination according to any one of claims 14 to 19, wherein the VH comprises a framework region HFR3, the N-terminal of HFR3 is directly or indirectly connected to the C-terminal of HCDR2, and the C-terminal of HFR3 is directly or indirectly connected to the N-terminal of HCDR3; and the HFR3 comprises an amino acid sequence shown in SEQ ID NO: 6 or an amino acid sequence having at least about 70% sequence identity to the amino acid sequence shown in SEQ ID NO: 6.

21. The pharmaceutical combination according to any one of claims 14 to 20, wherein the VH comprises a framework region HFR4, the N-terminal of HFR4 is directly or indirectly connected to the C-terminal of HCDR3, and the HFR4 comprises an amino acid sequence shown in SEQ ID NO: 7 or an amino acid sequence having at least about 70% sequence identity to the amino acid sequence shown in SEQ ID NO: 7.

22. The pharmaceutical combination according to any one of claims 14 to 21, wherein the VH comprises framework regions HFR1, HFR2, HFR3, and HFR4, the C-terminal of HFR1 is directly or indirectly connected to the N-terminal of HCDR1, the N-terminal of HFR2 is directly or indirectly connected to the C-terminal of HCDR1, and the C-terminal of HFR2 is directly or indirectly connected to the N-terminal of HCDR2, the N-terminal of HFR3 is directly or indirectly connected to the C-terminal of HCDR2, and the C-terminal of HFR3 is directly or indirectly connected to the N-terminal of HCDR3, the N-terminal of HFR4 is directly or indirectly connected to the C-terminal of HCDR3; among them, the HFR1 comprises an amino acid sequence shown in SEQ ID NO: 4 or an amino acid sequence having at least about 70% sequence identity to the amino acid sequence shown in SEQ ID NO: 4, the HFR2 comprises an amino acid sequence shown in SEQ ID NO: 5 or an amino acid sequence having at least about 70% sequence identity to the amino acid sequence shown in SEQ ID NO: 5, the HFR3 comprises an amino acid sequence shown in SEQ ID NO: 6 or an amino acid sequence having at least about 70% sequence identity to the amino acid sequence shown in SEQ ID NO: 6, and the HFR4 comprises an amino acid sequence shown in SEQ ID NO: 7 or an amino acid sequence having at least about 70% sequence identity to the amino acid sequence shown in SEQ ID NO: 7.

23. The pharmaceutical combination according to any one of claims 11 to 22, wherein the anti PD-1 antibody comprises VH, and the VH comprises an amino acid sequence shown in SEQ ID NO: 8.

24. The pharmaceutical combination according to any one of claims 11 to 23, wherein the anti PD-1 antibody comprises antibody heavy chain (HC), and the HC comprises an amino acid sequence shown in SEQ ID NO: 9.

25. The pharmaceutical combination according to any one of claims 11 to 24, wherein the anti PD-1 antibody comprises at least one CDR in the antibody light chain variable region (VL), and the VL comprises an amino acid sequence shown in SEQ ID NO: 17.

26. The pharmaceutical combination according to any one of claims 11 to 25, wherein the anti PD-1 antibody comprises at least one CDR in VH, the VH comprises an amino acid sequence shown in SEQ ID NO: 8, and the anti PD-1 antibody comprises at least one CDR in VL, and the VL comprises an amino acid sequence shown in SEQ ID NO: 17.

27. The pharmaceutical combination according to any one of claims 11 to 26, wherein the anti PD-1 antibody comprises VL comprising LCDR1, and the LCDR1 comprises an amino acid sequence shown in SEQ ID NO: 10.

28. The pharmaceutical combination according to claim 27, wherein the VL further comprises LCDR2, wherein the LCDR2 comprises an amino acid sequence shown in SEQ ID NO: 11.

29. The pharmaceutical combination according to any one of claims 27 to 28, wherein the VL further comprises LCDR3, wherein the LCDR3 comprises an amino acid sequence shown in SEQ ID NO: 12.

30. The pharmaceutical combination according to any one of claims 27 to 29, wherein the VL comprises LCDR1, LCDR2 and LCDR3, wherein the LCDR1 comprises an amino acid sequence shown in SEQ ID NO: 10, the LCDR2 comprises an amino acid sequence shown in SEQ ID NO: 11, and the LCDR3 comprises an amino acid sequence shown in SEQ ID NO: 12.

31. The pharmaceutical combination according to any one of claims 11 to 30, wherein the anti PD-1 antibody comprises VH and antibody VL, the VH comprises HCDR1, HCDR2, and HCDR3, wherein the HCDR3 comprises an amino acid sequence shown in SEQ ID NO: 3, the HCDR2 comprises an amino acid sequence shown in SEQ ID NO: 2, and the HCDR1 comprises an amino acid sequence shown in SEQ ID NO: 1; and the VL comprises LCDR1, LCDR2, and LCDR3, wherein the LCDR1 comprises an amino acid sequence shown in SEQ ID NO: 10, the LCDR2 comprises an amino acid sequence shown in SEQ ID NO: 11, and the LCDR3 comprises an amino acid sequence shown in SEQ ID NO: 12.

32. The pharmaceutical combination according to any one of claims 27 to 31, wherein the VL comprises a framework region LFR1, the C-terminal of LFR1 is directly or indirectly connected to the N-terminal of LCDR1, and the LFR1 comprises an amino acid sequence shown in SEQ ID NO: 13 or an amino acid sequence having at least about 70% sequence identity to the amino acid sequence shown in SEQ ID NO: 13.

33. The pharmaceutical combination according to any one of claims 27 to 32, wherein the VL comprises a framework region LFR2, the N-terminal of LFR2 is directly or indirectly connected to the C-terminal of LCDR1, and the C-terminal of LFR2 is directly or indirectly connected to the N-terminal of LCDR2; and the LFR2 comprises an amino acid sequence shown in SEQ ID NO: 14 or an amino acid sequence having at least about 70% sequence identity to the amino acid sequence shown in SEQ ID NO: 14.

34. The pharmaceutical combination according to any one of claims 27 to 33, wherein the VL comprises a framework region LFR3, the N-terminal of LFR3 is directly or indirectly connected to the C-terminal of LCDR2, and the C-terminal of LFR3 is directly or indirectly connected to the N-terminal of LCDR3; and the LFR3 comprises an amino acid sequence shown in SEQ ID NO: 15 or an amino acid sequence having at least about 70% sequence identity to the amino acid sequence shown in SEQ ID NO: 15.

35. The pharmaceutical combination according to any one of claims 27 to 34, wherein the VL comprises a framework region LFR4, the N-terminal of LFR4 is directly or indirectly connected to the C-terminal of LCDR3, and the LFR4 comprises an amino acid sequence shown in SEQ ID NO: 16 or an amino acid sequence having at least about 70% sequence identity to the amino acid sequence shown in SEQ ID NO: 16.

36. The pharmaceutical combination according to any one of claims 27 to 35, wherein the VL comprises framework regions LFR1, LFR2, LFR3 and LFR4, the C-terminal of LFR1 is directly or indirectly connected to the N-terminal of LCDR1, the N-terminal of LFR2 is directly or indirectly connected to the C-terminal of LCDR1, and the C-terminal of LFR2 is directly or indirectly connected to the N-terminal of LCDR2, the N-terminal of LFR3 is directly or indirectly connected to the C-terminal of LCDR2, and the C-terminal of LFR3 is directly or indirectly connected to the N-terminal of LCDR3, the N-terminal of LFR4 is directly or indirectly connected to the C-terminal of LCDR3; among them, the LFR1 comprises an amino acid sequence shown in SEQ ID NO: 13 or an amino acid sequence having at least about 70% sequence identity to the amino acid sequence shown in SEQ ID NO: 13, the LFR2 comprises an amino acid sequence shown in SEQ ID NO: 14 or an amino acid sequence having at least about 70% sequence identity to the amino acid sequence shown in SEQ ID NO: 14, the LFR3 comprises an amino acid sequence shown in SEQ ID NO: 15 or an amino acid sequence having at least about 70% sequence identity to the amino acid sequence shown in SEQ ID NO: 15, and the LFR4 comprises an amino acid sequence shown in SEQ ID NO: 16 or an amino acid sequence having at least about 70% sequence identity to the amino acid sequence shown in SEQ ID NO:16.

37. The pharmaceutical combination according to any one of claims 11 to 36, wherein the anti PD-1 antibody comprises VL, and the VL comprises an amino acid sequence shown in SEQ ID NO: 17.

38. The pharmaceutical combination according to any one of claims 11 to 37, wherein the anti PD-1 antibody comprises VH and VL, the VH comprises an amino acid sequence shown in SEQ ID NO: 8 and the VL comprises an amino acid sequence shown in SEQ ID NO: 17.

39. The pharmaceutical combination according to any one of claims 11 to 38, wherein the anti PD-1 antibody comprises antibody light chain (LC), and the LC comprises an amino acid sequence shown in SEQ ID NO: 18.

40. The pharmaceutical combination according to any one of claims 11 to 39, wherein the anti PD-1 antibody comprises HC and LC, the HC comprises an amino acid sequence shown in SEQ ID NO: 9 and the LC comprises an amino acid sequence shown in SEQ ID NO: 18.

41. The pharmaceutical combination according to claim 1, wherein the PD-L1 inhibitor has one or more of the following characteristics: a. inhibition or reduction of PD-L1 expression, such as transcription or translation of PD-L1; b. inhibition or reduction of PD-L1 activity, such as inhibition or reduction of PD-L1 binding to its associated receptors such as PD-1; and c. binding of PD-L1 or its receptors such as PD-1.

42. The pharmaceutical combination according to any one of claims 1 to 41, wherein the PD-L1 inhibitor comprises an anti PD-L1 antibody or antigen-binding fragments thereof.

43. The pharmaceutical combination according to claim 42, wherein the anti PD-L1 antibody is selected from the group consisting of Durvalumab, Atezolizumab, Envafolimab, Avelumab, MDX-1105, YW243.55.S70, MDPL3280A, AMP-224, LY3300054, RB0005, bio-analogues, bio-enhancers, bio-equivalents, or combinations thereof.

44. The pharmaceutical combination according to any one of claims 42 to 43, wherein the anti PD-L1 antibody comprises at least one CDR in VH, and the VH comprises an amino acid sequence shown in SEQ ID NO: 25.

45. The pharmaceutical combination according to any one of claims 42 to 44, wherein the anti PD-L1 antibody comprises VH comprising HCDR3, and the HCDR3 comprises an amino acid sequence shown in SEQ ID NO: 21.

46. The pharmaceutical combination according to claim 45, wherein the VH further comprises HCDR2, wherein the HCDR2 comprises an amino acid sequence shown in SEQ ID NO: 20.

47. The pharmaceutical combination according to any one of claims 45 to 46, wherein the VH further comprises HCDR1, wherein the HCDR1 comprises an amino acid sequence shown in SEQ ID NO: 19.

48. The pharmaceutical combination according to any one of claims 45 to 47, wherein the VH comprises HCDR1, HCDR2, and HCDR3, wherein the HCDR3 comprises an amino acid sequence shown in SEQ ID NO: 21, the HCDR2 comprises an amino acid sequence shown in SEQ ID NO: 20, and the HCDR1 comprises an amino acid sequence shown in SEQ ID NO: 19.

49. The pharmaceutical combination according to any one of claims 45 to 48, wherein the VH comprises a framework region HFR1, the C-terminal of HFR1 is directly or indirectly connected to the N-terminal of HCDR1, and the HFR1 comprises an amino acid sequence shown in SEQ ID NO: 22 or an amino acid sequence having at least about 70% sequence identity to the amino acid sequence shown in SEQ ID NO: 22.

50. The pharmaceutical combination according to any one of claims 45 to 49, wherein the VH comprises a framework region HFR2, the N-terminal of HFR2 is directly or indirectly connected to the C-terminal of HCDR1, and the C-terminal of HFR2 is directly or indirectly connected to the N-terminal of HCDR2; and the HFR2 comprises an amino acid sequence shown in SEQ ID NO: 23 or an amino acid sequence having at least about 70% sequence identity to the amino acid sequence shown in SEQ ID NO: 23.

51. The pharmaceutical combination according to any one of claims 45 to 50, wherein the VH comprises a framework region HFR3, the N-terminal of HFR3 is directly or indirectly connected to the C-terminal of HCDR2, and the C-terminal of HFR3 is directly or indirectly connected to the N-terminal of HCDR3; and the HFR3 comprises an amino acid sequence shown in SEQ ID NO: 24 or an amino acid sequence having at least about 70% sequence identity to the amino acid sequence shown in SEQ ID NO: 24.

52. The pharmaceutical combination according to any one of claims 45 to 51, wherein the VH comprises a framework region HFR4, the N-terminal of HFR4 is directly or indirectly connected to the C-terminal of HCDR3, and the HFR4 comprises an amino acid sequence shown in SEQ ID NO: 7 or an amino acid sequence having at least about 70% sequence identity to the amino acid sequence shown in SEQ ID NO: 7.

53. The pharmaceutical combination according to any one of claims 45 to 52, wherein the VH comprises framework regions HFR1, HFR2, HFR3 and HFR4, the C-terminal of HFR1 is directly or indirectly connected to the N-terminal of HCDR1, the N-terminal of HFR2 is directly or indirectly connected to the C-terminal of HCDR1, and the C-terminal of HFR2 is directly or indirectly connected to the N-terminal of HCDR2, the N-terminal of HFR3 is directly or indirectly connected to the C-terminal of HCDR2, and the C-terminal of HFR3 is directly or indirectly connected to the N-terminal of HCDR3, the N-terminal of HFR4 is directly or indirectly connected to the C-terminal of HCDR3; among them, the HFR1 comprises an amino acid sequence shown in SEQ ID NO: 22 or an amino acid sequence having at least about 70% sequence identity to the amino acid sequence shown in SEQ ID NO: 22, the HFR2 comprises an amino acid sequence shown in SEQ ID NO: 23 or an amino acid sequence having at least about 70% sequence identity to the amino acid sequence shown in SEQ ID NO: 23, the HFR3 comprises an amino acid sequence shown in SEQ ID NO: 24 or an amino acid sequence having at least about 70% sequence identity to the amino acid sequence shown in SEQ ID NO: 24, and the HFR4 comprises an amino acid sequence shown in SEQ ID NO: 7 or an amino acid sequence having at least about 70% sequence identity to the amino acid sequence shown in SEQ ID NO: 7.

54. The pharmaceutical combination according to any one of claims 42 to 53, wherein the anti PD-L1 antibody comprises VH, and the VH comprises an amino acid sequence shown in SEQ ID NO: 25.

55. The pharmaceutical combination according to any one of claims 42 to 54, wherein the anti PD-L1 antibody comprises HC, and the HC comprises an amino acid sequence shown in SEQ ID NO: 26.

56. The pharmaceutical combination according to any one of claims 42 to 55, wherein the anti PD-L1 antibody comprises at least one CDR in VL, and the VL comprises an amino acid sequence shown in SEQ ID NO: 37.

57. The pharmaceutical combination according to any one of claims 42 to 56, wherein the anti PD-L1 antibody comprises at least one CDR in VH, the VH comprises an amino acid sequence shown in SEQ ID NO: 25, and the anti PD-L1 antibody comprises at least one CDR in VL, and the VL comprises an amino acid sequence shown in SEQ ID NO: 37.

58. The pharmaceutical combination according to any one of claims 42 to 57, wherein the anti PD-L1 antibody comprises at least one CDR in VH, the VH comprises an amino acid sequence shown in SEQ ID NO: 25, and the anti PD-L1 antibody comprises at least one CDR in VL, and the VL comprises an amino acid sequence shown in SEQ ID NO:38, SEQ ID NO:39 or SEQ ID NO:40.

59. The pharmaceutical combination according to any one of claims 42 to 58, wherein the anti PD-L1 antibody comprises VL, the VL comprises LCDR1, and the LCDR1 comprises an amino acid sequence shown in SEQ ID NO: 27.

60. The pharmaceutical combination according to claim 59, wherein the anti PD-L1 antibody comprises VL, the VL comprises LCDR1, and the LCDR1 comprises an amino acid sequence shown in SEQ ID NO:28, SEQ ID NO:29 or SEQ ID NO:30.

61. The pharmaceutical combination according to any one of claims 59 to 60, wherein the VL further comprises LCDR2, wherein the LCDR2 comprises an amino acid sequence shown in SEQ ID NO: 31.

62. The pharmaceutical combination according to any one of claims 59 to 61, wherein the VL further comprises LCDR3, wherein the LCDR3 comprises an amino acid sequence shown in SEQ ID NO: 32.

63. The pharmaceutical combination according to any one of claims 59 to 62, wherein the VL comprises LCDR1, LCDR2 and LCDR3, wherein the LCDR1 comprises an amino acid sequence shown in SEQ ID NO: 27, the LCDR2 comprises an amino acid sequence shown in SEQ ID NO: 31, and the LCDR3 comprises an amino acid sequence shown in SEQ ID NO: 32.

64. The pharmaceutical combination according to claim 63, wherein the VL comprises LCDR1, LCDR2 and LCDR3, wherein the LCDR1 comprises an amino acid sequence shown in SEQ ID NO: 28, the LCDR2 comprises an amino acid sequence shown in SEQ ID NO: 31, and the LCDR3 comprises an amino acid sequence shown in SEQ ID NO: 32; the LCDR1 comprises an amino acid sequence shown in SEQ ID NO: 29, the LCDR2 comprises an amino acid sequence shown in SEQ ID NO: 31, the LCDR3 comprises an amino acid sequence shown in SEQ ID NO: 32; or the LCDR1 comprises an amino acid sequence shown in SEQ ID NO: 30, the LCDR2 comprises an amino acid sequence shown in SEQ ID NO: 31, and the LCDR3 comprises an amino acid sequence shown in SEQ ID NO: 32.

65. The pharmaceutical combination according to any one of claims 42 to 64, wherein the anti PD-L1 antibody comprises VH and antibody VL, the VH comprises HCDR1, HCDR2, and HCDR3, wherein the HCDR3 comprises an amino acid sequence shown in SEQ ID NO: 21, the HCDR2 comprises an amino acid sequence shown in SEQ ID NO: 20, and the HCDR1 comprises an amino acid sequence shown in SEQ ID NO: 19; and the VL comprises LCDR1, LCDR2, and LCDR3, wherein the LCDR1 comprises an amino acid sequence shown in SEQ ID NO: 27, the LCDR2 comprises an amino acid sequence shown in SEQ ID NO: 31, and the LCDR3 comprises an amino acid sequence shown in SEQ ID NO: 32.

66. The pharmaceutical combination according to claim 65, wherein the anti PD-L1 antibody comprises VH and antibody VL, the VH comprises HCDR1, HCDR2, and HCDR3, wherein the HCDR3 comprises an amino acid sequence shown in SEQ ID NO: 21, the HCDR2 comprises an amino acid sequence shown in SEQ ID NO: 20, and the HCDR1 comprises an amino acid sequence shown in SEQ ID NO: 19; and the VL comprises LCDR1, LCDR2, and LCDR3, wherein the LCDR1 comprises an amino acid sequence shown in SEQ ID NO:28, SEQ ID NO:29 or SEQ ID NO:30, the LCDR2 comprises an amino acid sequence shown in SEQ ID NO: 31, and the LCDR3 comprises an amino acid sequence shown in SEQ ID NO: 32.

67. The pharmaceutical combination according to any one of claims 59 to 66, wherein the VL comprises a framework region LFR1, the C-terminal of LFR1 is directly or indirectly connected to the N-terminal of LCDR1, and the LFR1 comprises an amino acid sequence shown in SEQ ID NO: 33 or an amino acid sequence having at least about 70% sequence identity to the amino acid sequence shown in SEQ ID NO: 33.

68. The pharmaceutical combination according to any one of claims 59 to 67, wherein the VL comprises a framework region LFR2, the N-terminal of LFR2 is directly or indirectly connected to the C-terminal of LCDR1, and the C-terminal of LFR2 is directly or indirectly connected to the N-terminal of LCDR2; and the LFR2 comprises an amino acid sequence shown in SEQ ID NO: 34 or an amino acid sequence having at least about 70% sequence identity to the amino acid sequence shown in SEQ ID NO: 34.

69. The pharmaceutical combination according to any one of claims 59 to 68, wherein the VL comprises a framework region LFR3, the N-terminal of LFR3 is directly or indirectly connected to the C-terminal of LCDR2, and the C-terminal of LFR3 is directly or indirectly connected to the N-terminal of LCDR3; and the LFR3 comprises an amino acid sequence shown in SEQ ID NO: 35 or an amino acid sequence having at least about 70% sequence identity to the amino acid sequence shown in SEQ ID NO: 35.

70. The pharmaceutical combination according to any one of claims 59 to 69, wherein the VL comprises a framework region LFR4, the N-terminal of LFR4 is directly or indirectly connected to the C-terminal of LCDR3, and the LFR4 comprises an amino acid sequence shown in SEQ ID NO: 36 or an amino acid sequence having at least about 70% sequence identity to the amino acid sequence shown in SEQ ID NO: 36.

71. The pharmaceutical combination according to any one of claims 59 to 70, wherein the VL comprises framework regions LFR1, LFR2, LFR3 and LFR4, the C-terminal of LFR1 is directly or indirectly connected to the N-terminal of LCDR1, the N-terminal of LFR2 is directly or indirectly connected to the C-terminal of LCDR1, and the C-terminal of LFR2 is directly or indirectly connected to the N-terminal of LCDR2, the N-terminal of LFR3 is directly or indirectly connected to the C-terminal of LCDR2, and the C-terminal of LFR3 is directly or indirectly connected to the N-terminal of LCDR3, the N-terminal of LFR4 is directly or indirectly connected to the C-terminal of LCDR3; among them, the LFR1 comprises an amino acid sequence shown in SEQ ID NO: 33 or an amino acid sequence having at least about 70% sequence identity to the amino acid sequence shown in SEQ ID NO: 33, the LFR2 comprises an amino acid sequence shown in SEQ ID NO: 34 or an amino acid sequence having at least about 70% sequence identity to the amino acid sequence shown in SEQ ID NO: 34, the LFR3 comprises an amino acid sequence shown in SEQ ID NO: 35 or an amino acid sequence having at least about 70% sequence identity to the amino acid sequence shown in SEQ ID NO: 35, and the LFR4 comprises an amino acid sequence shown in SEQ ID NO: 36 or an amino acid sequence having at least about 70% sequence identity to the amino acid sequence shown in SEQ ID NO:36.

72. The pharmaceutical combination according to any one of claims 42 to 71, wherein the anti PD-L1 antibody comprises VL, and the VL comprises an amino acid sequence shown in SEQ ID NO: 37.

73. The pharmaceutical combination according to claim 72, wherein the anti PD-L1 antibody comprises VL, and the VL comprises an amino acid sequence shown in SEQ ID NO:38, SEQ ID NO:39 or SEQ ID NO:40.

74. The pharmaceutical combination according to any one of claims 42 to 73, wherein the anti PD-L1 antibody comprises VH and VL, the VH comprises an amino acid sequence shown in SEQ ID NO: 25, and the VL comprises an amino acid sequence shown in SEQ ID NO: 37.

75. The pharmaceutical combination according to claim 74, wherein the anti PD-L1 antibody comprises VH and VL, the VH comprises an amino acid sequence shown in SEQ ID NO: 25, and the VL comprises an amino acid sequence shown in SEQ ID NO:38, SEQ ID NO:39 or SEQ ID NO:40.

76. The pharmaceutical combination according to any one of claims 42 to 75, wherein the anti PD-L1 antibody comprises LC, and the LC comprises an amino acid sequence shown in SEQ ID NO: 41.

77. The pharmaceutical combination according to claim 76, wherein the anti PD-L1 antibody comprises LC, and the LC comprises an amino acid sequence shown in SEQ ID NO:42, SEQ ID NO:43 or SEQ ID NO:44.

78. The pharmaceutical combination according to any one of claims 42 to 77, wherein the anti PD-L1 antibody comprises HC and LC, the HC comprises an amino acid sequence shown in SEQ ID NO: 26, and the LC comprises an amino acid sequence shown in SEQ ID NO: 41.

79. The pharmaceutical combination according to claim 78, wherein the anti PD-L1 antibody comprises HC and LC, the HC comprises an amino acid sequence shown in SEQ ID NO: 26, and the LC comprises an amino acid sequence shown in SEQ ID NO:42, SEQ ID NO:43 or SEQ ID NO:44.

80. The pharmaceutical combination according to any one of claims 1 to 79, wherein i) the PD-1 inhibitor and/or PD-L1 inhibitor in the pharmaceutical combination are not mixed with ii) the STING pathway agonist to each other in the pharmaceutical combination.

81. The pharmaceutical combination according to any one of claims 1 to 79, wherein i) the PD-1 inhibitor and/or PD-L1 inhibitor, and ii) the STING pathway agonist are present in the pharmaceutical combination in a single dosage form.

82. The pharmaceutical combination according to claim 81, wherein the pharmaceutical combination is formulated into a pharmaceutical composition.

83. The pharmaceutical combination according to claim 82, wherein the pharmaceutical composition comprises a PD-1 inhibitor or a PD-L1 inhibitor, and a STING pathway agonist.

84. The pharmaceutical combination according to any one of claims 82 to 83, wherein the STING pathway agonist is present in an amount of about 0.0001 mg/kg to about 200 mg/kg.

85. The pharmaceutical combination according to any one of claims 82 to 84, wherein the PD-1 inhibitor or PD-L1 inhibitor is present in an amount of about 0.0001 mg/kg to about 200 mg/kg.

86. The pharmaceutical combination according to any one of claims 82 to 85, wherein the pharmaceutical composition further comprises one or more pharmaceutically acceptable carriers.

87. Use of the pharmaceutical combination according to any one of claims 1 to 86 in the preparation of drugs for the treatment of neoplastic diseases.

88. Use according to claim 87, wherein the neoplastic diseases comprise tumors and/or wart diseases.

89. The pharmaceutical combination according to any one of claims 1 to 86 for use in the treatment of neoplastic diseases.

90. A drug for the treatment of neoplastic diseases, comprising the pharmaceutical combination according to any one of claims 1 to 86.

91. A method for treating neoplastic diseases, comprising administering an effective amount of the pharmaceutical combination according to any one of claims 1 to 86 to a subject in need thereof.

92. The method according to claim 91, wherein the subject suffers from neoplasm.

93. The method according to claim 92, wherein the neoplasm comprises tumors and/or warts.

94. The method according to any one of claims 91 to 93, wherein the administration comprises local, intraneoplasitc or systemic administrations.

95. The method according to any one of claims 91 to 94, wherein the administration comprises intravenous injection, intravenous instillation, intramuscular injection, subcutaneous injection, and/or intraneoplasitc injection.

96. The method according to any one of claims 91 to 95, wherein i) the PD-1 inhibitor or PD-L1 inhibitor, and ii) the STING pathway agonist in the pharmaceutical combination are administered by use of the same or different administration routes.

97. The method according to any one of claims 91 to 96, comprising injection of the STING pathway agonist into the neoplasm.

98. The method according to any one of claims 91 to 97, further comprising injection or systemic infusion of the PD-1 inhibitor or PD-L1 inhibitor into the neoplasm.

99. The method according to claim 98, comprising injection of the STING pathway agonist and systemic infusion of the PD-1 inhibitor or PD-L1 inhibitor into the neoplasm.

100. The method according to any one of claims 91 to 98, comprising injecting the i) the PD-1 inhibitor or PD-L1 inhibitor, and ii) the STING pathway agonist in the pharmaceutical combination into the neoplasm.

101. The method according to any one of claims 91 to 100, wherein i) the PD-1 inhibitor or PD-L1 inhibitor, and ii) the STING pathway agonist in the pharmaceutical combination are administered simultaneously or at different times.

102. The method according to claim 101, wherein the PD-1 inhibitor or PD-L1 inhibitor is administered before and/or after the administration of the STING pathway agonist.

103. The method according to claim 102, wherein the PD-1 inhibitor or PD-L1 inhibitor is administered after the administration of the STING pathway agonist.

104. The method according to claim 103, wherein the method comprises: i) injection of the STING pathway agonist into the neoplasm; ii) injection or systemic infusion of the PD-1 inhibitor or PD-L1 inhibitor into the neoplasm after administering the STING pathway agonist.

105. The method according to claim 104, wherein the method comprises: i) injection of the STING pathway agonist into the neoplasm; and ii) systemic infusion of the PD-1 inhibitor or PD-L1 inhibitor after administering the STING pathway agonist.

106. The method according to claim 101, wherein i) the PD-1 inhibitor or PD-L1 inhibitor, and ii) the STING pathway agonist in the pharmaceutical combination are administered simultaneously.

107. The method according to claim 106, wherein i) the PD-1 inhibitor or PD-L1 inhibitor, and ii) the STING pathway agonist in the pharmaceutical combination are simultaneously administered by way of intraneoplasitc injection, and the PD-1 inhibitor or PD-L1 inhibitor and the STING pathway agonist are present in a same dosage form.

108. A pharmaceutical kit, comprising the pharmaceutical combination according to any one of claims 1 to 86.

Figure 3

Figure 4

Figure 5

Figure 6

hPD1_MC38

Figure 7

| | TGI (%) | Q value | |
|---|---|---|---|
| Vel. | 0 | | |
| RB0005 | 43.7 | | |
| cGAMP -1 | 70.82 | | |
| cGAMP -3.16 | 79.63 | | |
| cGAMP -10 | 91.07 | | |
| RB0005-1 in combination with CGAMP-1 | 90.03 | 1.07>1, | Synergism |
| RB0005-1 in combination with CGAMP-1 | 98.85 | 1.11>1, | Synergism |
| RB0005-1 in combination with CGAMP-10 | 96.65 | 1.02>1, | Synergism |

Figure 8

Days after treatment

Volume of MC38-ZB-2038 tumor
(day 15 after treatment)

Figure 9

RB0004_i.p.+LPG1501_i.t._q.2.d_(mean±SD)
- Vel.
- RB0004_10mpk
- RB0004+LPG1501-0.03mpk
- RB0004+LPG1501-0.1mpk
- RB0004+LPG1501-0.3mpk

Figure 10

Figure 11

Figure 12

MC38-ZB-2038

Figure 13

Figure 14

Figure 15

Days after treatment (MC38)

Figure 16

Days after treatment (MC38)

Figure 17

Figure 18

Figure 19

Figure 20

Figure 21

Figure 22

Figure 23

Days after treatment （MC38-ZB-2038）

Figure 24

Days after treatment

Days after treatment

Figure 25

Figure 26

Figure 27

Figure 28

Days after treatment (MC38-ZB-2038)

Figure 29

RB0005+LPG2005/RB0005+LPG1501_q.2.d_(mean±SD)

G1:Veh.
G2: RB0005-1mpk_i.p.
G3:LPG1501-1mpk_i.t.
G4:LPG1501-1mpk_i.t. + RB0005-1mpk_i.p.

MC38-ZB-2038

Figure 30

LPG2008(RB0005(1mpk): LPG1501(0.1/0.316/1mpk))_i.t._q.2.d

- CT26.sgCd274CD274 Veh.
- RB0005-1mpk)
- LPG2008 (RB0005:LPG1501-0.1mpk)
- LPG2008 (RB0005:LPG1501-0.316mpk)
- LPG2008 (RB0005:LPG1501-1mpk)

Figure 31

Figure 32

Figure 33

Figure 34

RB0005+LPG1501_i.t._q.2.d_(mean±SD)

- Veh.
- RB0005-1mpk
- LPG1501-1mpk
- LPG2008

Volume of tumor (mm$^3$)

Days after treatment

Figure 35

Figure 36

Figure 37

Figure 38

Figure 39

Figure 40

Figure 41

Figure 42

Figure 43

Days after treatment (hPD1-LLC1)

<div align="center">

**INTERNATIONAL SEARCH REPORT**

</div>

| International application No. |
|---|
| **PCT/CN2022/094047** |

| A. | CLASSIFICATION OF SUBJECT MATTER |
|---|---|

A61K 39/395(2006.01)i;  A61K 31/7084(2006.01)i;  A61P 35/00(2006.01)i

According to International Patent Classification (IPC) or to both national classification and IPC

| B. | FIELDS SEARCHED |
|---|---|

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNTXT; ENTXT; WPABS; CJFD; DWPI; CNKI; PubMed; 中国专利生物序列检索系统, Chinese Patent Biological Sequence Retrieval System; STN: 抗体, 天津立博美华, 免疫检查点, 程序性细胞死亡蛋白1, 程序性死亡配体1, PD-L1, PD-1, 单抗, RB0004, RB0005, SEQ ID NOs: 1-44, 激动剂, STING, 干扰素基因刺激蛋白, TMEM173, 环状二核苷酸, 环二核苷酸, CDP, agonist, cGAMP, AMP, GMP

| C. | DOCUMENTS CONSIDERED TO BE RELEVANT |
|---|---|

| Category* | Citation of document, with indication, where appropriate, of the relevant passages | Relevant to claim No. |
|---|---|---|
| X | CN 108310378 A (HANGZHOU XINGAO BIOTECHNOLOGY CO., LTD.) 24 July 2018 (2018-07-24)<br> claims 1-5 | 1-11, 41-42, 80-108 |
| Y | CN 108310378 A (HANGZHOU XINGAO BIOTECHNOLOGY CO., LTD.) 24 July 2018 (2018-07-24)<br> claims 1-5 | 12-108 |
| Y | CN 106008714 A (REYOUNG (SUZHOU) BIOPHARMACEUTICALS., LTD.) 12 October 2016 (2016-10-12)<br> description, paragraphs 9-17 | 12-108 |
| Y | CN 105968200 A (REYOUNG (SUZHOU) BIOPHARMACEUTICALS., LTD.) 28 September 2016 (2016-09-28)<br> claims 1-5 | 43-108 |
| X | CN 108025051 A (NOVARTIS AG et al.) 11 May 2018 (2018-05-11)<br> claims 1-16 | 1-11, 41-42, 80-108 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **05 August 2022** | **16 August 2022** |

| Name and mailing address of the ISA/CN | Authorized officer |
|---|---|
| **China National Intellectual Property Administration (ISA/ CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2022/094047** |

**C.** **DOCUMENTS CONSIDERED TO BE RELEVANT**

| Category* | Citation of document, with indication, where appropriate, of the relevant passages | Relevant to claim No. |
|---|---|---|
| Y | CN 108025051 A (NOVARTIS AG et al.) 11 May 2018 (2018-05-11)<br>claims 1-16 | 12-108 |
| X | CN 106727331 A (LIAOCHENG CITY ORIENT BIOMEDICAL TECHNOLOGY CO., LTD.) 31 May 2017 (2017-05-31)<br>claims 1-6 | 1-11, 41-42, 80-108 |
| Y | CN 106727331 A (LIAOCHENG CITY ORIENT BIOMEDICAL TECHNOLOGY CO., LTD.) 31 May 2017 (2017-05-31)<br>claims 1-6 | 12-108 |
| X | DORTA-ESTREMERA, S. et al. "Targeting Interferon Signaling and CTLA-4 Enhance the Therapeutic Efficacy of Anti-PD-1 Immunotherapy in Preclinical Model of HPV + Oral Cancer"<br>*Journal for ImmunoTherapy of Cancer,* Vol. 7, No. 252, 18 September 2019 (2019-09-18),<br>abstract, p. 6, right column, paragraph 2 to p. 8, right column, paragraph 1 | 1-11, 41-42, 80-108 |
| Y | DORTA-ESTREMERA, S. et al. "Targeting Interferon Signaling and CTLA-4 Enhance the Therapeutic Efficacy of Anti-PD-1 Immunotherapy in Preclinical Model of HPV + Oral Cancer"<br>*Journal for ImmunoTherapy of Cancer,* Vol. 7, No. 252, 18 September 2019 (2019-09-18),<br>abstract, p. 6, right column, paragraph 2 to p. 8, right column, paragraph 1 | 12-108 |

Form PCT/ISA/210 (second sheet) (January 2015)

**INTERNATIONAL SEARCH REPORT**

International application No.

**PCT/CN2022/094047**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

| International application No. |
| --- |
| **PCT/CN2022/094047** |

Box No. II    Observations where certain claims were found unsearchable (Continuation of item 2 of first sheet)

This international search report has not been established in respect of certain claims under Article 17(2)(a) for the following reasons:

1. ☑ Claims Nos.: **89、91-107**
because they relate to subject matter not required to be searched by this Authority, namely:

   [1]   Claims 89 and 91-107 relate to a method for treating neoplastic diseases, belonging to a treatment method. Therefore, no international search is performed according to PCT Rule 39.1(iv). A search was performed on the basis of a pharmaceutical use of the pharmaceutical composition.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
*Information on patent family members*

International application No.

**PCT/CN2022/094047**

| Patent document cited in search report | | | Publication date (day/month/year) | Patent family member(s) | | | Publication date (day/month/year) |
|---|---|---|---|---|---|---|---|
| CN | 108310378 | A | 24 July 2018 | None | | | |
| CN | 106008714 | A | 12 October 2016 | JP | 2019514355 | A | 06 June 2019 |
| | | | | EP | 3466974 | A1 | 10 April 2019 |
| | | | | WO | 2017201766 | A1 | 30 November 2017 |
| | | | | US | 2019071501 | A1 | 07 March 2019 |
| CN | 105968200 | A | 28 September 2016 | WO | 2017197667 | A1 | 23 November 2017 |
| | | | | JP | 2019514853 | A | 06 June 2019 |
| | | | | US | 2019077867 | A1 | 14 March 2019 |
| | | | | EP | 3459973 | A1 | 27 March 2019 |
| CN | 108025051 | A | 11 May 2018 | JP | 2021119167 | A | 12 August 2021 |
| | | | | WO | 2017019896 | A1 | 02 February 2017 |
| | | | | US | 2018222982 | A1 | 09 August 2018 |
| | | | | JP | 2018522027 | A | 09 August 2018 |
| | | | | CN | 114272371 | A | 05 April 2022 |
| | | | | EP | 3328418 | A1 | 06 June 2018 |
| CN | 106727331 | A | 31 May 2017 | None | | | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 201610345750
- WO 2017201766 A1
- WO 2009114335 A
- US 8354509 B
- US 8008449 B
- WO 2006121168 A
- WO 2009101611 A
- US 8609089 B
- US 2010028330 A
- US 20120114649 A
- US 20130034559 A1
- US 8217149 B
- US 20140341917 A1
- CN 201610340678
- WO 2017197667 A1
- WO 2007005874 A
- WO 2010077634 A
- US 7943743 B
- US 20120039906 A

### Non-patent literature cited in the description

- **ISHIDA et al.** *EMBO J.,* 1992, vol. 11, 3887-3895
- **AGATA et al.** *Int. Immunology,* 1996, vol. 8, 765-772
- **CHINAI et al.** *Trends in Pharmacological Sciences,* 2015, vol. 36, 587-595
- **MILLER et al.** *Jour. of Immunology,* 2003, vol. 170, 4854-4861
- **WARD et al.** Binding Activities of a Repertoire of Single Immunoglobulin Variable Domains Secreted From Escherichia coli. *Nature,* 1989, vol. 341, 544-546
- **HUSTON et al.** Protein Engineering of Antibody Binding Sites: Recovery of Specific Activity in an Anti-Digoxin Single-Chain Fv Analogue Produced in Escherichia coli. *Proc.Natl.Acad.Sci.USA,* 1988, vol. 85, 5879-5883
- **NGUYEN V.K. et al.** *The EMBO Journal,* 2000, vol. 19, 921-930
- **MUYLDERMANS S.** *J Biotechnol.,* 2001, vol. 74, 277-302
- **VANLANDSCHOOT P. et al.** *Antiviral Research,* 2011, vol. 92, 389-407
- **KABAT et al.** Sequences of Immunological Interest. National Institute of Health,Bethesda, 1991
- **KABAT et al.** Sequences of Proteins of Immunological Interest. National Institutes of Health, Bethesda, 1987
- **CHOTHIA ; LESK.** *J.Mol.Biol.,* 1987, vol. 196, 901-917
- **CHOTHIA et al.** *Nature,* 1989, vol. 342, 877-883
- *FASEB J.,* 1995, vol. 9, 133-139
- **MACCALLUM.** *JMol Biol,* 1996, vol. 262 (5), 732-45
- **ALTSCHULSF et al.** *Nuclear Acids Res.,* 1997, vol. 25, 3389-3402
- Acute Lymphoblastic Leukemia. *Clin Cancer Res,* 2017, vol. 23, 1012-1024
- **HAMID et al.** *New England Journal of Medicine,* 2013, vol. 369 (2), 134-44
- *CHEMICAL ABSTRACTS,* 946414-94-4